# EUROPEAN PATENT APPLICATION

(11) **EP 4 233 882 A1**
(43) Date of publication of application: **30.08.2023**
(21) Application number: 21883324.2
(22) Date of filing: 22.10.2021
(51) Int. Cl.: A61K 35/12, A61K 47/69, A61K 47/46, A61K 31/662, A61K 39/395, A61K 31/704, A61P 35/00, A61K 39/00

(54) **MITOCHONDRIA COMPRISING ANTICANCER DRUG AND USE THEREOF**

(30) Priority: 22.10.2020 KR 20200137779
(71) Applicant: Paean Biotechnology Inc., Daejeon 34013 (KR)
(72) Inventor: KIM, Chun-Hyung, Sejong 30130 (KR); HAN, Kyuboem, Daejeon 34119 (KR); YU, Shin-Hye, Seoul 04736 (KR); KIM, Yu Jin, Seoul 07007 (KR); LEE, Seo-Eun, Suwon-si Gyeonggi-do 16558 (KR); PARK, Jong Hyeok, Seoul 04597 (KR); CHO, Gayoung, Seoul 01180 (KR); KANG, Young-Cheol, Wonju-si Gangwon-do 26463 (KR)
(74) Representative: HGF
(86) International application number: PCT/KR2021/014886
(87) International publication number: WO 2022/086257

(57) **Abstract**

The present invention relates to a mitochondrion containing a compound having the anticancer efficacy and uses thereof. It was confirmed that the mitochondrion efficiently deliver the compound to the tumor, and the anticancer effect of the mitochondrion containing the compound was confirmed. In particular, when an antibody or a fragment thereof that specifically binds to a tumor is bound to the surface of mitochondrion, the compound may be delivered specifically to the tumor. Therefore, the mitochondrion containing the compound according to the present invention may be effectively used for the treatment of cancer because the side effects of the compound are few.

## Description

### Technical Field

The present invention provides a compound capable of modifying mitochondrion, mitochondrion containing the compound, and a pharmaceutical composition comprising the same as an active ingredient.

### Background Art

Mitochondria are cellular organelles of eukaryotic cells involved in the synthesis and regulation of adenosine triphosphate (ATP), an intracellular energy source. Mitochondria are associated with various metabolic pathways *in vivo,* for example, cell signaling, cell differentiation, cell death, as well as control of cell cycle and cell growth. Mitochondria have their own genomes and are organelles that play a central role in the energy metabolism of cells. Mitochondria produce energy through the electron transport and oxidative phosphorylation process, and play an important role in being involved in apoptosis signaling pathways.

It has been reported that a reduction in energy production due to a decrease in mitochondrial function causes various diseases. When the function of the electron transport chain reaction decreases according to the variation of the mitochondria genome and proteome, a reduction in ATP production, an excessive production of the reactive oxygen species, a decrease in calcium regulation function and the like occur. In this case, a change in the membrane permeability of the mitochondria occurs, and the function of apoptosis may occur abnormally and lead to cancer and incurable diseases.

As such, human diseases that have been reported to result from mitochondrial dysfunction include mitochondria related genetic disease, diabetes mellitus, heart disease, senile dementia such as Parkinson's disease or Alzheimer's disease, and the occurrence of various cancers and cancer metastasis and the like. In addition, features commonly found in more than 200 types of various cancers consisted of impaired apoptosis function, increased inflammatory response, and increased abnormal metabolism. All of these processes are closely related to mitochondrial function, and the correlation between cancer and mitochondria is drawing attention.

On the other hand, it is known that normal cells produce 36 ATP per molecule of glucose through an electron transport system process, but cancer cells, unlike normal cells, produce 2 ATP per molecule of glucose through glycolysis under a sufficient oxygen condition (aerobic glycolysis). As such, it is known that cancer cells, unlike normal cells, use the inefficient glycolysis process in terms of energy in order to produce amino acids, lipids, nucleic acids and the like necessary for rapid cell proliferation. For this reason, it is known that cancer cells require less oxygen and produce a larger amount of lactic acid than normal cells.

Therefore, a change in the composition of the cancer microenvironment due to abnormal metabolism occurring in cancer cells, an inhibition of apoptosis caused by dysfunctional mitochondria, and an increase in inflammatory response, and abnormal metabolic reaction in cancer cells play a very important role in cancer proliferation. Thus, developing metabolism-related anticancer agents using these features may be a good way capable of solving the side effects and economic problems of conventional anticancer agents. On the other hand, recent attempts to directly administer or develop mitochondria as drugs have been made (Korean Patent Registration No. 10-2111321).

### Detailed Description of Invention

### Technical Problem

Accordingly, the present inventors completed the present invention by confirming that mitochondria have an excellent anticancer effect when used in combination with a conventionally used anticancer agent in the process of studying the anticancer activity of mitochondria.

### Solution to Problem

In one aspect of the present invention, there is provided mitochondrion containing an anticancer agent and a pharmaceutical composition for the prevention or treatment of cancer comprising the same.

In another aspect of the present invention, there is provided a method for preparing mitochondrion containing an anticancer agent, comprising mixing the anticancer agent and the isolated mitochondrion.

In another aspect of the present invention, there is provided a modified anticancer agent to which TPP is chemically bound.

In another aspect of the present invention, there is provided a method for preventing or treating cancer, comprising administering a pharmaceutical composition comprising mitochondrion containing an anticancer agent to a subject.

### Effects of the Invention

It was confirmed that the mitochondrion containing the compound having the anticancer efficacy according to the present invention efficiently deliver the compound to the tumor, and the anticancer effect of the mitochondrion containing the compound was confirmed. In particular, when an antibody or a fragment thereof that specifically binds to a tumor is bound to the surface of mitochondria, the compound may be delivered specifically to the tumor. Therefore, the mitochondrion containing the compound according to the present invention may be effectively used for the treatment of cancer because the side effects of the compound are few.

### Brief Description of Drawings

FIG. 1 is a view schematically showing an expression vector for expressing a single chain variable region fragment of an anti-HER2 antibody in a form fused to the mitochondria.
FIG. 2 is a view showing the results of confirming a single chain variable region fragment of an anti-HER2 antibody expressed in the human fetal kidney cell line (HEK293) by Western blot.
FIG. 3 is a view showing the results of confirming the intracellular location of a single chain variable region fragment of an anti-HER2 antibody expressed in the human fetal kidney cell line (HEK293) by a confocal microscope. The scale bar represents 10 µm.
FIG. 4 is a view showing the results of confirming by Western blot that a single chain variable region fragment of an anti-HER2 antibody expressed in the human fetal kidney cell line (HEK293) exists at the same location as the mitochondria in the cell.
FIG. 5 is a graph showing the results of measuring ATP in the mitochondria (MT^{α-HER2scFV}) isolated from the human fetal kidney cell line (HEK293) that expresses a single chain variable region fragment of an anti-HER2 antibody. In this case, the control group is the mitochondria isolated from the HEK293 cells that do not express a single chain variable region fragment of an anti-HER2 antibody.
FIG. 6 is a graph showing the results of measuring the membrane potential in the mitochondria (MT^{α-HER2scFv}) isolated from the human fetal kidney cell line (HEK293) that expresses a single chain variable region fragment of an anti-HER2 antibody. In this case, the control group is the mitochondria isolated from the HEK293 cells that do not express a single chain variable region fragment of an anti-HER2 antibody.
FIG. 7 is a graph showing the results of measuring the reactive oxygen species (ROS) in the mitochondria (MT^{α-HER2scFv}) isolated from the human fetal kidney cell line (HEK293) that expresses a single chain variable region fragment of an anti-HER2 antibody. In this case, the control group is the mitochondria isolated from the HEK293 cells that do not express a single chain variable region fragment of an anti-HER2 antibody.
FIG. 8 is a view showing the results of confirming the expression of HER2 in the human breast cancer cell line (SK-BR-3, BT-474 and MDA-MB-231), the human lung cancer cell line (NCI-H1975), the lung fibroblast cell line (WI-38) and the human gastric cancer cell line (NCI-N87 and MKN-74) by Western blot.
FIG. 9 is a view showing the results of confirming the expression of HER2 in the human breast cancer cell line (SK-BR-3, BT-474 and MDA-MB-231), the human lung cancer cell line (NCI-H1975), the human lung fibroblast cell line (WI-38) and the human gastric cancer cell line (NCI-N87 and MKN-74) by immunocytochemistry staining.
FIG. 10 is a view showing the results of confirming the expression of HER2 in the human breast cancer cell line (SK-BR-3, BT-474 and MDA-MB-231), the human lung cancer cell line (NCI-H1975) and the human gastric cancer cell line (NCI-N87 and MKN-74) by flow cytometry.
FIG. 11 is a view showing an experimental method for verifying the HER2 targeting ability of the mitochondria (MT^{α-HER2scFv}) isolated from the human fetal kidney cell line (HEK293) that expresses a single chain variable region fragment of an anti-HER2 antibody.
FIG. 12 is a view showing the results of confirming the intracellular expression of HER2 and a single chain variable region fragment of an anti-HER2 antibody by immunocytochemistry staining after co-culture of HER2⁺ cells, the human breast cancer cell line SK-BR-3, and HER2⁻ cells, the human lung cancer cell line NCI-H1975 cells, and then treatment of the culture solution with MT^{α-HER2scFv}. In this case, MT is the mitochondria, to which a single chain variable region fragment of an anti-HER2 antibody is not fused, isolated from the HEK293 cells.
FIG. 13 is a view showing the results of confirming the intracellular expression of HER2 and a single chain variable region fragment of an anti-HER2 antibody by immunocytochemistry staining after co-culture of HER2⁺ cells, the human breast cancer cell line BT-474, and HER2⁻ cells, the human lung cancer cell line NCI-H1975 cells, and then treatment of the cells with MT^{α-HER2scFv}. In this case, MT is the mitochondria, to which a single chain variable region fragment of an anti-HER2 antibody is not fused, isolated from the HEK293 cells.
FIG. 14 is a view showing the results of confirming the intracellular expression of HER2 and a single chain variable region fragment of an anti-HER2 antibody by immunocytochemistry staining after co-culture of HER2⁺ cells, the human gastric cancer cell line NCI-N87, and HER2⁻ cells, the human gastric cancer cell line MKN-74 cells, and then treatment of the cells with MT^{α-HER2scFv}. In this case, MT is the mitochondria, to which a single chain variable region fragment of an anti-HER2 antibody is not fused, isolated from the HEK293 cells.
FIG. 15 is a view showing the results of confirming the intracellular expression of HER2 and a single chain variable region fragment of an anti-HER2 antibody by immunocytochemistry staining after co-culture of HER2⁺ cells, the human breast cancer cell line SK-BR-3, and HER2⁻ cells, the human breast cancer cell line MDA-MB-231 cells, and then treatment of the cells with MT^{α-HER2scFv}. In this case, MT is the mitochondria, to which a single chain variable region fragment of an anti-HER2 antibody is not fused, isolated from the HEK293 cells.
FIG. 16 is a view showing the results of confirming the intracellular expression of HER2 and a single chain variable region fragment of an anti-HER2 antibody by immunocytochemistry staining after co-culture of HER2⁺ cells, the human breast cancer cell line BT-474, and HER2⁻ cells, the human breast cancer cell line MDA-MB-231 cells, and then treatment of the cells with MT^{α-HER2scFv}. In this case, MT is the mitochondria, to which a single chain variable region fragment of an anti-HER2 antibody is not fused, isolated from the HEK293 cells.
FIG. 17 is a view showing a method for preparing DOX_MT^{α-HER2scFv} in which MT^{α-HER2scFv} stained with MitoTracker green and doxorubicin, a compound payload, are bound.
FIG. 18 is a view showing the results of observing the human breast cancer cell line (SK-BR-3, BT-474 and MDA-MB-231) and the human lung cancer cell line (NCI-H1975) after treatment with DOX_MT^{α-HER2scFv} by a confocal microscope.
FIG. 19 is a view showing the results of observing the morphological change and death process of cancer cells over time by a confocal microscope after treatment of the human breast cancer cell line (SK-BR-3 and BT-474) and the human gastric cancer cell line (NCI-N87) with DOX_MT^{α-HER2scFv}.
FIG. 20 is a graph showing a standard curve obtained by measuring the absorbance for each concentration of doxorubicin.
FIG. 21 is a graph showing the results of measuring the cytotoxicity by treatment of the human breast cancer cell line (SK-BR-3, BT-474 and MDA-MB-231), the human lung cancer cell line (NCI-H1975) and the human gastric cancer cell line (NCI-N87 and MKN-74) with DOX or DOX_MT^{α-HER2scFv}. In this case, MT is MT^{α-HER2scFv} in which DOX is not loaded.
FIG. 22 is a graph showing the results of measuring the cytotoxicity by treatment of the human lung fibroblast cell line (WI-38 and CCD-8Lu) with DOX or DOX_MT^{α-HER2scFv}. In this case, MT is MT^{α-HER2scFv} in which DOX is not loaded.
FIG. 23 is a view showing the results of confirming the IC₅₀ value of DOX or DOX_MT^{α-HER2scFv} in the human breast cancer cell line (SK-BR-3, BT-474 and MDA-MB-231), the human lung cancer cell line (NCI-H1975), the human gastric cancer cell line (NCI-N87 and MKN-74) and the human lung fibroblast cell line (WI-38 and CCD-8Lu).
FIG. 24 is a graph showing the results of measuring the cytotoxicity by treatment of the human breast cancer cell line (SK-BR-3, BT-474 and MDA-MB-231), the human lung cancer cell line (NCI-H1975), the human gastric cancer cell line (NCI-N87 and MKN-74), the human lung fibroblast cell line (WI-38 and CCD-8Lu) with 0.5 µM DOX or DOX_MT^{α-HER2scFv}. In this case, MT is MT^{α-HER2scFv} in which DOX is not loaded.
FIG. 25 is a view showing the results of confirming the apoptosis by treatment of the human breast cancer cell line (SK-BR-3 and BT-474) and the human gastric cancer cell line (NCI-N87) with MT^{α-HER2scFv} or DOX_MT^{α-HER2scFv} by TUNEL assay.
FIG. 26 is a view showing the results of confirming the apoptosis caused by treatment of the human breast cancer cell line (SK-BR-3 and BT-474) with MT^{α-HER2scFv} or DOX_MT^{α-HER2scFv} by Western blot.
FIG. 27 is a graph showing the results of confirming the apoptosis by treatment of the human breast cancer cell line (SK-BR-3 and BT-474) and the human gastric cancer cell line (NCI-N87) with MT^{α-HER2scFv} or DOX_MT^{α-HER2scFv}by flow cytometry. In this case, MT is MT^{α-HER2scFv} in which DOX is not loaded.
FIG. 28 is a view showing an experimental method for confirming the selective killing effect of HER2 expressing cancer cells by DOX_MT^{α-HER2scFv}.
FIG. 29 is a view showing the results of confirming the apoptosis by co-culture of the human breast cancer cell line BT-474 (HER2⁺, white arrow) and the human lung fibroblast cell line WI-38 (HER2⁻), and then treatment of the cells with DOX_MT^{α-HER2scFv} by TUNEL assay.
FIG. 30 is a view showing the synthesis process of triphenylphosphonium (TPP)-doxorubicin (TPP-DOX).
FIG. 31 is a view showing the results of MRI analysis of TPP-DOX.
FIG. 32 is a view showing a method for preparing TPP-DOX MT^{α-HER2scFv} in which MT^{α-HER2scFv} stained with MitoTracker green and TPP-DOX, a compound payload, are bound.
FIG. 33 is a view showing the results of observing the human breast cancer cell line (SK-BR-3, BT-474 and MDA-MB-231) and the lung cancer cell line (NCI-H1975) after treatment with TPP-DOX_MT^{α-HER2scFv} by a confocal microscope.
FIG. 34 is a view showing the results of observing the morphological change and death process of cancer cells over time by a confocal microscope after treatment of the human breast cancer cell line (SK-BR-3 and BT-474) and the human gastric cancer cell line (NCI-N87) with TPP-DOX_MT^{α-HER2scFv}.
FIG. 35 is a view showing the results of measuring the cytotoxicity by treatment of the human breast cancer cell line (SK-BR-3, BT-474 and MDA-MB-231), the human lung cancer cell line (NCI-H1975) and the human gastric cancer cell line (NCI-N87 and MKN-74) with TPP-DOX and TPP-DOX_MT^{α-HER2scFv}. In this case, MT is MT^{α-HER2scFv} in which DOX is not loaded.
FIG. 36 is a view showing the results of measuring the cytotoxicity by treatment of the human lung fibroblast cell line (WI-38 and CCD-8Lu) with TPP-DOX and TPP-DOX_MT^{α-HER2scFv}. In this case, MT is MT^{α-HER2scFv} in which DOX is not loaded.
FIG. 37 is a view showing the results of confirming the IC₅₀ value of TPP-DOX and TPP-DOX_MT^{α-HER2scFv} in the human breast cancer cell line (SK-BR-3, BT-474 and MDA-MB-231), the human lung cancer cell line (NCI-H1975), the human gastric cancer cell line (NCI-N87 and MKN-74) and the human lung fibroblast cell line (WI-38 and CCD-8Lu).
FIG. 38 is a view showing the results of measuring the cytotoxicity by treatment of the human breast cancer cell line (SK-BR-3, BT-474 and MDA-MB-231), the human lung cancer cell line (NCI-H1975), the human gastric cancer cell line (NCI-N87 and MKN-74) and the human lung fibroblast cell line (WI-38 and CCD-8Lu) with 0.5 µM TPP-DOX or TPP-DOX_MT^{α-HER2scFv}.
FIG. 39 is a view showing the results of confirming the apoptosis by treatment of the human breast cancer cell line (SK-BR-3 and BT-474 and MDA-MB-231) and the gastric cancer cell line (NCI-N87) with MT^{α-HER2scFv} or TPP-DOX MT^{α-HER2scFv} by TUNEL assay.
FIG. 40 is a view showing the results of confirming the apoptosis caused by treatment of the human breast cancer cell line (SK-BR-3 and BT-474) and the human gastric cancer cell line (NCI-N87) with MT^{α-HER2scFv} or TPP-DOX_MT^{α-HER2scFv} by Western blot.
FIG. 41 is a graph showing the results of confirming the apoptosis caused by treatment of the human breast cancer cell line (SK-BR-3 and BT-474) and the gastric cancer cell line (NCI-N87) with MT^{α-HER2scFv} or TPP-DOX MT^{α-HER2scFv} by flow cytometry.
FIG. 42 is a view showing an experimental method for confirming the selective killing effect of HER2 expressing cells by TPP-DOX_MT^{α-HER2scFv}.
FIG. 43 is a view showing the results of confirming the apoptosis by co-culture of the human breast cancer cell line SK-BR-3 (HER2⁺, white arrow) and the human lung fibroblast cell line CCD-8Lu (HER2⁻), and then treatment of the cells with TTP-DOX_MT^{α-HER2scFv} by TUNEL assay.
FIG. 44 is a view schematically showing an expression vector for expressing a single chain variable region fragment of an anti-PDL1 antibody in a form fused to the mitochondria.
FIG. 45 is a view showing the results of confirming a single chain variable region fragment of an anti-PDL1 antibody expressed in the human fetal kidney cell line (HEK293) by Western blot.
FIG. 46 is a view showing the results of confirming the expression of PD-L1 in the human pancreatic cancer cell line (BXPC-3 and CFPAC-1) and the human gastric cancer cell line (SNU-216 and SNU-484) by Western blot.
FIG. 47 is a view showing an experimental method for verifying the PDL1 targeting ability of MT^{α-PDL1scFv} after co-culture of PDL1⁺ cells and PDL1⁻ cells, and then treatment of the cells with the mitochondria (MT^{α-PDL1scFv}) isolated from the human fetal kidney cell line (HEK293) that expresses a single chain variable region fragment of an anti-PDL1 antibody.

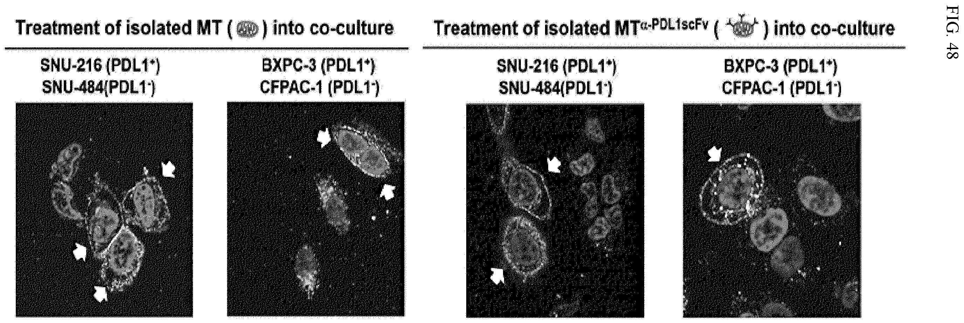

FIG. 48 is a view showing the results of confirming the intracellular expression of a single chain variable region fragment of an anti-PDL1 antibody by immunocytochemistry staining after co-culture of the human gastric cancer cell line SNU-216 (PDL1⁺, white arrow) and SNU-484 (PDL1⁻) or co-culture of the human pancreatic cancer cell line BXPC-3 (PDL1⁺, white arrow) and the human pancreatic cancer cell line CFPAC-1 (PDL1⁻), and then treatment of the cells with MT^{α-PDL1scFv}. In this case, MT is the mitochondriua to which a single chain variable region fragment of an anti-PDL1 antibody is not fused, isolated from the HEK293 cells.
FIG. 49 is a view showing a method for preparing DOX_MT^{α-PDL1scFv} in which MT^{α-PDL1scFv} stained with MitoTracker green and doxorubicin (DOX), a compound payload, are bound.
FIG. 50 is a graph showing the results of measuring the cytotoxicity by treatment of the human pancreatic cancer cell line (BXPC-3 and CFPAC-1) and the human gastric cancer cell line (SNU-216 and SNU-484) with MT^{α-PDL1scFv} or DOX_MT^{α-PDL1scFv}.
FIG. 51 is a view showing the results of confirming the apoptosis by treatment of the human pancreatic cancer cell line (BXPC-3 and CFPAC-1) and the human gastric cancer cell line (SNU-216 and SNU-484) with MT^{α-PDL1scFv} or DOX_MT^{α-PDL1scFv} by Western blot.
FIG. 52 is a view schematically showing an expression vector for expressing a single chain variable region fragment of an anti-Mesothelin (MSLN) antibody in a form fused to the mitochondria.
FIG. 53 is a view showing the results of confirming a single chain variable region fragment of an anti-MSLN antibody expressed in the human fetal kidney cell line (HEK293) by Western blot.
FIG. 54 is a view showing a method for preparing PBA_MT^{α-MSLNscFv} in which MT^{α-MSLNscFv} stained with MitoTracker green and pheophorbide A (PBA), a compound payload, are bound.
FIG. 55 is a view and graph showing the results of measuring the cytotoxicity by treatment of the human pancreatic cancer cell line (AsPC-1) with MT^{α-MSLNscFv} or PBA_MT^{α-MSLNscFv}. MT^{α-MSLNscFv} is the negative control for PBA_MT^{α-MSLNscFv}.
FIG. 56 is a view and graph showing the results of measuring the cytotoxicity by treatment of the human pancreatic cancer cell line (Capan-1) with MT^{α-MSLNscFv} or PBA_MT^{α-MSLNscFv}. MT^{α-MSLNscFv} is the negative control for PBA_MT^{α-MSLNscFv}.
FIG. 57 is a view and graph showing the results of measuring the cytotoxicity by treatment of the human pancreatic cancer cell line (Capan-2) with MT^{α-MSLNscFv} or PBA_MT^{α-MSLNscFv}. MT^{α-MSLNscFv} is the negative control for PBA_MT^{α-MSLNscFv}.
FIG. 58 is a view and graph showing the results of measuring the cytotoxicity by treatment of the human pancreatic cancer cell line (Mia PaCa-2) with MT^{α-MSLNscFv} or PBA_MT^{α-MSLNscFv}. MT^{α-MSLNscFv} is the negative control for PBA_MT^{α-MSLNscFv}.
FIG. 59 is a view showing a method for preparing Gem_MT^{α-MSLNscFv} in which MT^{α-MSLNscFv} stained with MitoTracker green and gemcitabine, a compound payload, are bound.
FIG. 60 is a view and graph showing the results of measuring the cytotoxicity by treatment of the human pancreatic cancer cell line (AsPC-1, Capan-1, Capan-2 and Mia PaCa-2) with MT^{α-MSLNscFv} or Gem_MT^{α-MSLNscFv}. MT^{α-MSLNscFv} is the negative control for Gem_MT^{α-MSLNscFv}.
FIG. 61 is a view showing a schematic diagram of a method for preparing VIBE_MT^{α-MSLNscFv} in which MT^{α-MSLNscFv} stained with MitoTracker green and vinorelbine, a compound payload, are bound.
FIG. 62 is a graph showing the results of measuring the cytotoxicity by treatment of the human pancreatic cancer cell line (AsPC-1, Capan-1, Capan-2 and Mia PaCa-2) with MT^{α-MSLNscFv} or VIBE_MT^{α-MSLNscFv}. MT^{α-MSLNscFv} is the negative control for VIBE_MT^{α-MSLNscFv}.
FIG. 63 is a view showing an experimental method for verifying the HER2 targeting ability of MT^{α-HER2scFv} after administration of MT^{α-HER2scFv} to a xenograft mouse model formed by transplanting the human gastric cancer cell line NCI-N87 (HER2⁺).
FIG. 64 is a view showing the results of observing that MT^{α-HER2scFv} is located in the tumor after administration of MT^{α-HER2scFv} to a xenograft mouse model formed by transplanting the human gastric cancer cell line NCI-N87 (HER2⁺).
FIG. 65 is a view showing an experimental method for confirming the anticancer efficacy of TPP-DOX or TPP-DOX MT^{α-HER2scFv} in a xenograft mouse model formed by transplanting the human gastric cancer cell line NCI-N87 (HER2⁺).
FIG. 66 is a graph showing the results of measuring the tumor size (tumor volume) by date after administration of TPP-DOX or TPP-DOX MT^{α-HER2scFv} to a xenograft mouse model formed by transplanting the human gastric cancer cell line NCI-N87 (HER2⁺).
FIG. 67 is a view confirming the size of the tumor extracted after 25 days from the start of administration of TPP-DOX or TPP-DOX_MT^{α-HER2scFv} in a xenograft mouse model formed by transplanting the human gastric cancer cell line NCI-N87 (HER2⁺).
FIG. 68 is a graph showing the results of measuring the weight of the tumor extracted after 25 days from the start of administration of TPP-DOX or TPP-DOX MT^{α-HER2scFv} in a xenograft mouse model formed by transplanting the human gastric cancer cell line NCI-N87 (HER2⁺).
FIG. 69 is a view showing an experimental method for confirming the anticancer efficacy of MT^{α-HER2scFv}, TPP-DOX or TPP-DOX MT^{α-HER2scFv} in an allograft mouse model formed by transplanting the mouse melanoma cell line B16F10 that overexpresses human HER2.
FIG. 70 is a graph showing the results of measuring the tumor size (tumor volume) by date after administration of MT^{α-HER2scFv}, TPP-DOX or TPP-DOX MT^{α-HER2scFv} to an allograft mouse model formed by transplanting the mouse melanoma cell line B 16F 10 that overexpresses human HER2.
FIG. 71 is a view confirming the size of the tumor extracted after 21 days from the start of administration of MT^{α-HER2scFv}, TPP-DOX or TPP-DOX MT^{α-HER2scFv} to an allograft mouse model formed by transplanting the mouse melanoma cell line B16F10 that overexpresses human HER2.
FIG. 72 is a graph showing the results of measuring the weight of the tumor extracted after 21 days from the start of administration of MT^{α-HER2scFv}, TPP-DOX or TPP-DOX MT^{α-HER2scFv} to an allograft mouse model formed by transplanting the mouse melanoma cell line B 16F 10 that overexpresses human HER2.
FIG. 73 is a view showing an experimental method for confirming the anticancer efficacy of MT^{α-HER2scFv}, DOX or DOX_MT^{α-HER2scFv} in an allograft mouse model formed by transplanting the mouse melanoma cell line B 16F 10 that overexpresses human HER2.
FIG. 74 is a graph showing the results of measuring the tumor size by date after administration of MT^{α-HER2scFv}, DOX or DOX MT^{α-HER2scFv} to an allograft mouse model formed by transplanting the mouse melanoma cell line B 16F 10 that overexpresses human HER2.
FIG. 75 is a view confirming the size of the tumor extracted after 21 days from the start of administration of MT^{α-HER2scFv}, TPP-DOX or TPP-DOX MT^{α-HER2scFv} to an allograft mouse model formed by transplanting the mouse melanoma cell line B16F10 that overexpresses human HER2.
FIG. 76 is a graph showing the results of measuring the weight of the tumor extracted after 21 days from the start of administration of MT^{α-HER2scFv}, DOX or DOX_MT^{α-HER2scFv} to an allograft mouse model formed by transplanting the mouse melanoma cell line B 16F 10 that overexpresses human HER2.

### Best Mode for Carrying out the Invention

### Mitochondrion containing anticancer agent

In one aspect of the present invention, there is provided mitochondrion containing an anticancer agent. In addition, in another aspect of the present invention, there is provided a pharmaceutical composition for the prevention or treatment of cancer comprising the mitochondrion containing an anticancer agent as an active ingredient.

As used herein, the term "mitochondrion" is a cellular organelle of eukaryotic cells involved in the synthesis and regulation of adenosine triphosphate (ATP), an intracellular energy source. Mitochondria are associated with various metabolic pathways in vivo, for example, cell signaling, cell differentiation, cell death, as well as control of cell cycle and cell growth. Therefore, it has been reported that mitochondrial hypofunction or dysfunction due to genetic, environmental, or unknown causes is related to the occurrence of mitochondria related genetic disease, inflammatory diseases such as rheumatoid arthritis, ischemic diseases, infectious diseases, heart diseases, muscle disease, degenerative diseases such as Parkinson's disease or Alzheimer's disease, etc. and the development of various diseases such as carcinogenesis and cancer metastasis.

The mitochondrion may be obtained from eukaryotes, and may be obtained from mammals or humans. Specifically, the mitochondrion may be isolated from cells or tissues. For example, the mitochondrion may be obtained from somatic cells, germ cells, or stem cells, and may be isolated from blood cells or platelets. In addition, the mitochondrion may be isolated after disruption by concentrating the tissues or cells, or may be isolated after disruption from a tissue or cell sample thawed after freezing and storage.

Specifically, the somatic cells may be muscle cells, hepatocytes, nerve cells, fibroblasts, epithelial cells, adipocytes, osteocytes, leukocytes, lymphocytes, platelets, or mucosal cells.

In addition, the stem cells are undifferentiated cells having the ability to differentiate into various types of tissue cells, and may be any one selected from the group consisting of mesenchymal stem cells, adult stem cells, induced pluripotent stem cells, embryonic stem cells, bone marrow stem cells, neural stem cells, limbal stem cells, and tissue-derived stem cell, but are not limited thereto. In this case, the mesenchymal stem cells may be obtained from any one selected from the group consisting of umbilical cord, umbilical cord blood, bone marrow, fat, muscle, nerve, skin, synovial fluid, testis, amniotic membrane and placenta.

In addition, the mitochondrion may be isolated by culturing cells or tissues *in vitro,* or may be isolated from a sample thawed after freezing and storage.

In addition, the mitochondrion may be obtained from an autologous, allogenic, or xenogenic subject. Specifically, the autologous mitochondrion refers to mitochondrion obtained from tissues or cells of the same subject. In addition, the allogenic mitochondrion refers to mitochondrion obtained from a subject that belongs to the same species as the subject and has different genotypes for alleles. In addition, the xenogenic mitochondrion refer to mitochondrion obtained from a subject that belongs to the different species from the subject.

In addition, the mitochondrion may be mitochondrion isolated from cells. In addition, the mitochondrion may be intact and have mitochondrial activity.

On the other hand, when the mitochondria are isolated from specific cells, the mitochondria may be isolated through a variety of modified methods, including various known methods, for example, using a specific buffer solution or using a potential difference and a magnetic field and the like.

The mitochondrial isolation may be obtained by disrupting cells and centrifuging in terms of maintaining mitochondrial activity.

In this case, an anticancer agent may be present in the outer membrane of the mitochondrion. Specifically, the anticancer agent may be located between the outer membrane and the inner membrane. In addition, the anticancer agent may be located in cristae. It may also be present in a matrix inside the inner membrane.

In addition, the mitochondrion may be modified mitochondrion. In this case, the modified mitochondrion may be one in which an antibody or a fragment thereof specifically binding to a tumor-associated antigen (TAA) is present on the surface of a cell.

In this case, the tumor-associated antigen may be any one selected from the group consisting of CD19, CD20, melanoma antigen E (MAGE), NY-ESO-1, carcinoembryonic antigen (CEA), mucin 1 cell surface associated (MUC-1), prostatic acid phosphatase (PAP), prostate specific antigen (PSA), survivin, tyrosine related protein 1 (tyrp1), tyrosine related protein 2 (tyrp2), Brachyury, Mesothelin, Epidermal growth factor receptor (EGFR), human epidermal growth factor receptor 2 (HER-2), ERBB2, Wilms tumor protein (WT1), FAP, EpCAM, PD-L1, ACPP, CPT1A, IFNG, CD274, FOLR1, EPCAM, ICAM2, NCAM1, LRRC4, UNCSH2 LILRB2, CEACAM, MSLN, Nectin-3 and a combination thereof, but is not limited to the above types.

As used herein, the term "cancer" is classified as a disease in which normal tissue cells proliferate unrestrictedly for some reason and continue to grow rapidly regardless of the living phenomenon of the living body or the surrounding tissue condition. In the present invention, cancer may be any one cancer selected from the group consisting of various cancers of the human body, such as breast cancer, lung cancer, pancreatic cancer, glioblastoma, gastric cancer, liver cancer, colorectal cancer, prostate cancer, ovarian cancer, cervical cancer, thyroid cancer, laryngeal cancer, acute myeloid leukemia, brain tumor, neuroblastoma, retinoblastoma, head and neck cancer, salivary gland cancer and lymphoma, but is not limited to the above types.

As used herein, the term "anticancer agent" is a drug used to inhibit the proliferation of cancer cells. In the present invention, the anticancer agent may be selected from the group consisting of a chemical anticancer agent, a target anticancer agent and an anthelmintic agent, but is not limited thereto.

Specifically, the "chemical anticancer agent" may be any one selected from the group consisting of an alkylating agent, a microtuble inhibitor, an antimetabolite and a topoisomerase inhibitor, but is not limited thereto.

The alkylating agent may be any one selected from the group consisting of mechlorethamine, cyclophosphamide, ifosfamide, melphalan, chlorambucil, thiotepa, altretamine, procarbazine, busulfan, streptozocin, carmustine, iomustine, dacarbazine, doxorubicin, cisplatin, carboplatin and oxaliplatin, but is not limited thereto. The microtuble inhibitor may be any one selected from the group consisting of docetaxel, vinblastine, oncovin and vinorelbine, but is not limited thereto. The antimetabolite may be any one selected from the group consisting of fluorouracil, capecitabine, cytarabine, gemcitabine, fludarabine, methotrexate, pemetrexed and mercaptopurine, but is not limited thereto. The topoisomerase inhibitor may be any one selected from the group consisting of hycamtin, camptosar, vepesid, taxol, bleomycin, adriamycin and cerubidine, but is not limited thereto.

In addition, the "target anticancer agent" may be a compound that regulates a specific target biomarker. For example, the target anticancer agent may be any one selected from the group consisting of compounds targeting BTK, Bcr-abl, EGFR, PDGFR/VEGFR/FGFR family, MEK/RAF, HER2/Neu, CDK, ubiquitin, JAK, MAP2K, ALK, PARP, TGFβRI, Proteasome, Bcl-2, Braf, C-Met, VR1, VR2, VR3, c-kit, AXL and RET, but is not limited thereto.

One embodiment of the compound among the target anticancer agents may be ibrutinib, which targets BTK. In addition, it may be dasatinib, nilotinib, imatinib or bosutinib, which targets Bcr-abl. In addition, it may be osimertinib, erlotinib or gefitinib, which targets EGFR, but is not limited thereto. In addition, it may be nintedanib, sunitinib, sorafenib, cabozantinib, lenvatinib, regorafenib, masitinib, semaxanib, tivozanib, vandetanib, axitinib, or pazopanib, which targets PDGFR/VEGFR/FGFR family, but is not limited thereto. In addition, it may be trametinib/dabrafenib, which targets MEK/RAF, but is not limited thereto.

In addition, it may be afatinib, lapatinib or neratinib, which targets HER2/Neu, but is not limited thereto. In addition, it may be abemaciclib or palbociclib, which targets CDK, but is not limited thereto. In addition, it may be lenalidomide, which targets ubiquitin, but is not limited thereto. In addition, it may be ruxolitinib, lestaurtinib or pacritinib, which targets JAK, but is not limited thereto. In addition, it may be cobimethinib, selumetinib, trametinib or binimetinib, which targets MAP2K, but is not limited thereto. In addition, it may be alectinib or crizotinib, which targets ALK, but is not limited thereto. In addition, it may be olaparib, which targets PARP, but is not limited thereto. In addition, it may be galunisertib, which targets TGFβRI, but is not limited thereto. In addition, it may be ixazomib, which targets Proteasome, but is not limited thereto. In addition, it may be venetoclax, which targets Bcl-2, but is not limited thereto. In addition, it may be vemurafenib, which targets Braf, but is not limited thereto.

In addition, the "anthelmintic agent" may be any one selected from the group consisting of an OXPHOS (oxidative phosphorylation) inhibitor, a glycolysis inhibitor, a glycolysis related indirect inhibitor, a PPP (pentose phosphate pathway) inhibitor and an autophagy inhibitor, but is not limited thereto. Specifically, the anthelmintic agent may be any one selected from the group consisting of pyrvinium, niclosamide, nitazoxanide, ivermectin, fenbendazole, nitazoxanide and albendazole, but is not limited thereto.

### Modified mitochondrion

The modified mitochondrion refers to mitochondrion in which a foreign protein is bound to the outer membrane of the mitochondrion. In this case, the modified mitochondrion may be disclosed in Korean Patent Application Publication No. 10-2019-0048279.

As used herein, the term "foreign protein" refers to a protein that includes a target protein capable of functioning inside and outside the cell. In this case, the foreign protein is a protein that does not exist in the mitochondrion and may be a recombinant protein. Specifically, the foreign protein may comprise a mitochondrial anchoring peptide and a target protein. In addition, the foreign protein may be a recombinant fusion protein comprising a mitochondrial anchoring peptide and a target protein. In this case, the foreign protein may comprise a mitochondrial anchoring peptide. Preferably, the mitochondrial anchoring peptide may be a peptide that may be located on the mitochondrial outer membrane. Therefore, the foreign protein may be bound to the outer membrane of the mitochondrion by a mitochondrial anchoring peptide. The mitochondrial anchoring peptide may be a peptide comprising an N terminal region or a C terminal region of a protein present in a mitochondrial membrane protein, and the N terminal region or the C terminal region of a protein present in the outer membrane of the mitochondrial protein may be located on the outer membrane of the mitochondria. In this case, the anchoring peptide may further comprise a mitochondrial signal sequence.

One embodiment of the protein present in a mitochondrial membrane protein may be any one selected from the group consisting of TOM20, TOM70, OM45, TOM5, TOM6, TOM7, TOM22, Fis1, Bcl-2, Bcl-x and VAMP1B. In particular, when the mitochondrial anchoring peptide is derived from any one selected from the group consisting of TOM20, TOM70 and OM45, it may comprise the N terminal region of TOM20, TOM70 or OM45. One embodiment of the mitochondrial anchoring peptide may be TOM70 derived from yeast represented by SEQ ID NO: 75, or TOM70 derived from human represented by SEQ ID NO: 76. Another embodiment may be TOM20 derived from yeast represented by SEQ ID NO: 77, or TOM20 derived from human represented by SEQ ID NO: 78. Another embodiment may be OM45 derived from yeast represented by SEQ ID NO: 79.

In addition, when the mitochondrial anchoring peptide is derived from any one selected from the group consisting of TOM5, TOM6, TOM7, TOM22, Fis1, Bcl-2, Bcl-x and VAMP1B, it may comprise the C terminal region of any one selected from the group consisting of TOM5, TOM6, TOM7, TOM22, Fis1, Bcl-2, Bcl-x and VAMP1B. One embodiment of the mitochondrial anchoring peptide may be TOM5 derived from yeast represented by SEQ ID NO: 80 or TOM5 derived from human represented by SEQ ID NO: 81. Another embodiment may be TOM7 derived from yeast represented by SEQ ID NO: 82, or TOM7 derived from human represented by SEQ ID NO: 83. Another embodiment may be TOM22 derived from yeast represented by SEQ ID NO: 84, or TOM22 derived from human represented by SEQ ID NO: 85. Another embodiment may be Fis1 derived from yeast represented by SEQ ID NO: 86, or Fis1 derived from human represented by SEQ ID NO: 87. Another embodiment may be Bcl-2 alpha derived from human represented by SEQ ID NO: 88. Another embodiment may be VAMP1 derived from yeast represented by SEQ ID NO: 89, or VAMP1 derived from human represented by SEQ ID NO: 90.

In this case, a target protein capable of functioning inside and outside the cell included in the foreign protein may be any one selected from the group consisting of an active protein exhibiting an activity in a cell, a protein present in a cell, and a protein having the ability to bind to a ligand or receptor present in a cell membrane.

An embodiment of the active protein or the protein present in a cell may be any one selected from the group consisting of p53, Granzyme B, Bax, Bak, PDCD5, E2F, AP-1 (Jun/Fos), EGR-1, Retinoblastoma (RB), phosphatase and tensin homolog (PTEN), E-cadherin, Neurofibromin-2 (NF-2), poly[ADP-ribose] synthase 1 (PARP-1), BRCA-1, BRCA-2, Adenomatous polyposis coli (APC), Tumor necrosis factor receptor-associated factor (TRAF), RAF kinase inhibitory protein (RKIP), p16, KLF-10, LKB1, LHX6, C-RASSF, DKK-3PD1, Oct3/4, Sox2, Klf4, and c-Myc. When the target protein is selected from the above group, the target protein may be bound to an anchoring peptide comprising the N terminal region of TOM20, TOM70 or OM45.

Such fusion protein may be bound in the following order:
N terminus-anchoring peptide comprising the N terminal region of TOM20, TOM70 or OM45-target protein-C terminus.

In addition, the foreign protein may further comprise an amino acid sequence recognized by a proteolytic enzyme in eukaryotic cells, or ubiquitin or a fragment thereof between a mitochondrial anchoring peptide and a target protein. The proteolytic enzyme in eukaryotic cells refers to an enzyme that degrades a protein present in eukaryotic cells. In this case, because a foreign protein comprises an amino acid sequence recognized by the enzyme that degrades the protein, the foreign protein bound to the mitochondrial outer membrane may be isolated into an anchoring peptide and a target protein in a cell.

In this case, the ubiquitin fragment may comprise the C terminal Gly-Gly of an amino acid sequence of SEQ ID NO: 71, and may comprise 3 to 75 amino acids consecutive from the C terminus. In addition, the foreign protein may further comprise a linker between a target protein and ubiquitin or a fragment thereof. In this case, the linker may be composed of 1 to 150 amino acids, or be composed of 10 to 100 amino acids, or be composed of 20 to 50 amino acids, but is not limited thereto. The linker may be composed of amino acids that are appropriately selected from 20 amino acids, preferably be composed of glycine and/or serine. One embodiment of the linker may be composed of 5 to 50 amino acids consisting of glycine and serine. One embodiment of the linker may be (G4S)n, in which n is an integer of 1 to 10, and n may be 1, 2, 3, 4, 5, 6, 7, 8, 9, or 10.

In addition, the protein having the ability to bind to a ligand or receptor present in a cell membrane may be a ligand or receptor present on the surface of a tumor cell. In this case, the ligand or receptor present on the surface of a tumor cell may be, but is not limited to, any one selected from the group consisting of CD19, CD20, melanoma antigen E (MAGE), NY-ESO-1, carcinoembryonic antigen (CEA), mucin 1 cell surface associated (MUC-1), prostatic acid phosphatase (PAP), prostate specific antigen (PSA), survivin, tyrosine related protein 1 (tyrp1), tyrosine related protein 2 (tyrp2), Brachyury, Mesothelin, Epidermal growth factor receptor (EGFR), human epidermal growth factor receptor 2 (HER-2), ERBB2, Wilms tumor protein (WT1), FAP, EpCAM, PD-L1, ACPP, CPT1A, IFNG, CD274, FOLR1, EPCAM, ICAM2, NCAM1, LRRC4, UNCSH2 LILRB2, CEACAM, Nectin-3 and a combination thereof.

In addition, the protein having the ability to bind to a ligand or receptor present in a cell membrane may be an antibody or a fragment thereof that binds to any one selected from the above group. In particular, a fragment of an antibody refers to a fragment having the same complementarity determining region (CDR) as that of the antibody. Specifically, it may be Fab, scFv, F(ab')₂ or a combination thereof.

In this case, the target protein may be bound to an anchoring peptide comprising an C terminal region of any one selected from the group consisting of TOM5, TOM6, TOM7, TOM22, Fis1, Bcl-2, Bcl-x and VAMP1B, and the foreign protein may be bound in the following order:
N terminus-target protein-anchoring peptide comprising a C terminal region of any one selected from the group consisting of TOM5, TOM6, TOM7, TOM22, Fis1, Bcl-2, Bcl-x and VAMP1B-C terminus.

In addition, the foreign protein may further comprise a linker between a target protein and a C terminal region of any one selected from the group consisting of TOM5, TOM6, TOM7, TOM22, Fis1, Bcl-2, Bcl-x and VAMP1B. In this case, a linker is as described above. In this case, a target protein, an active protein, a protein present in a cell, and a protein having the ability to bind to a ligand or receptor present in a cell membrane and the like are as described above.

In one embodiment of the target protein, an antibody or a fragment thereof targeting a specific cell may be in a form bound to the anchoring peptide comprising the C terminal region of any one selected from the group consisting of TOM5, TOM6, TOM7, TOM22, Fis1, Bcl-2, Bcl-x and VAMP1B. The modified mitochondrion to which the target protein is bound may be easily introduced into a specific target, so that the mitochondrion may be efficiently entered into a specific cell.

One embodiment of the modified mitochondrion may be in a form to which one or more target proteins are bound. Specifically, it may be in a form to which a target protein comprising p53 and a target protein comprising anti-HER-2 antibody or a fragment thereof are bound. Such modified mitochondrion may effectively deliver the mitochondrion into cancer cells expressing HER-2. In addition, cancer cells may be effectively killed by p53 bound to the modified mitochondrion.

Depending on the purpose of the modified mitochondrion, a target protein comprising one or more active proteins may be constructed and be allowed to be bound to the mitochondrion. In addition, a target protein targeting a cell may be constructed in various ways depending on the targeted cell.

The fusion protein comprising the mitochondrial outer membrane targeting protein and the target protein may be referred to as a fusion protein that modifies the mitochondrial activity. Such fusion protein may have any one of the following structures:
**<Structural Formula 1>**
   N terminus-mitochondrial outer membrane anchoring peptide-target protein-C terminus
**<Structural Formula 2>**
   N terminus-mitochondrial outer membrane anchoring peptide-ubiquitin or fragment thereof-target protein-C terminus
**<Structural Formula 3>**
   N terminus-mitochondrial outer membrane targeting peptide-linker 1-ubiquitin or fragment thereof-target protein-C terminus
**<Structural Formula 4>**
   N terminus-mitochondrial outer membrane anchoring peptide-ubiquitin or fragment thereof-linker 2-target protein-C terminus
**<Structural Formula 5>**
   N terminus-mitochondrial outer membrane anchoring peptide-linker 1-ubiquitin or fragment thereof-linker 2-target protein-C terminus

In the above Structural Formulae 1 to 5, the outer membrane anchoring peptide may be a terminal sequence of a protein selected from the group consisting of TOM20, TOM70 and OM45, and the target protein may be any one selected from the group consisting of p53, Granzyme B, Bax, Bak, PDCD5, E2F, AP-1 (Jun/Fos), EGR-1, Retinoblastoma (RB), phosphatase and tensin homolog (PTEN), E-cadherin, Neurofibromin-2 (NF-2), poly[ADP-ribose] synthase 1 (PARP-1), BRCA-1, BRCA-2, Adenomatous polyposis coli (APC), Tumor necrosis factor receptor-associated factor (TRAF), RAF kinase inhibitory protein (RKIP), p16, KLF-10, LKB1, LHX6, C-RASSF and DKK-3PD1.

In this case, the linker 1 or 2 may be a polypeptide composed of 1 to 100, 1 to 80, 1 to 50, or 1 to 30 amino acids, respectively, and may be preferably a polypeptide composed of 1 to 30 amino acids that consist of serine, glycine or threonine alone or in combination. In addition, the linker 1 or 2 may be a polypeptide composed of 5 to 15 amino acids, respectively, and may be preferably a polypeptide composed of 5 to 15 amino acids that consist of serine, glycine or threonine alone or in combination. One embodiment of the linker may be (GGGGS)3 (SEQ ID NO: 70).
**<Structural Formula 6>**
   N terminus-target protein-mitochondrial outer membrane anchoring peptide-C terminus
**<Structural Formula** 7>
   N terminus-target protein-ubiquitin or fragment thereof-mitochondrial outer membrane anchoring peptide-C terminus
**<Structural Formula 8>**
   N terminus-target protein-linker 1-ubiquitin or fragment thereof-mitochondrial outer membrane anchoring peptide-C terminus
**<Structural Formula 9>**
   N terminus-target protein-ubiquitin or fragment thereof-linker 2-mitochondrial outer membrane anchoring peptide-C terminus
**<Structural Formula 10>**
   N terminus-target protein-linker 1-ubiquitin or fragment thereof-linker 2-mitochondrial outer membrane targeting peptide-C terminus

In the above Structural Formulae 6 to 10, the outer membrane anchoring peptide may be a terminal sequence of a protein selected from the group consisting of TOM5, TOM6, TOM7, TOM22, Fis1, Bcl-2, Bcl-X, and VAMP1B, and the target protein may be any one selected from the group consisting of p53, Granzyme B, Bax, Bak, PDCD5, E2F, AP-1 (Jun/Fos), EGR-1, Retinoblastoma (RB), phosphatase and tensin homolog (PTEN), E-cadherin, Neurofibromin-2 (NF-2), poly[ADP-ribose] synthase 1 (PARP-1), BRCA-1, BRCA-2, Adenomatous polyposis coli (APC), Tumor necrosis factor receptor-associated factor (TRAF), RAF kinase inhibitory protein (RKIP), p16, KLF-10, LKB1, LHX6, C-RASSF, DKK-3PD1, Oct3/4, Sox2, Klf4, and c-Myc. In this case, the linker 1 or 2 is as described above.

For the pharmaceutical composition, the mitochondrion may be included at a concentration of about 0.1 µg/mL to about 500 µg/mL, about 0.2 µg/mL to about 450 µg/mL, or about 0.5 µg/mL to about 400 µg/mL, but is not limited thereto. The inclusion of the mitochondria in the above range may facilitate the dose adjustment of mitochondria upon administration and may enhance the degree of improvement of the symptoms of a disease of a patient. In this case, the dose of mitochondria may be determined through the quantification of mitochondria by quantifying the membrane protein of the isolated mitochondria. Specifically, the isolated mitochondria may be quantified through the Bradford protein assay.

In addition, for the pharmaceutical composition, an anticancer agent binding to mitochondrion may be included at a concentration of 0.1 µg/mL to 500 µg/mL, 0.2 µg/mL to 450 µg/mL, or 0.5 µg/mL to 400 µg/mL, but is not limited thereto. The inclusion of the anticancer agent in the above range may facilitate the dose adjustment of the anticancer agent upon administration and may enhance the degree of improvement of the symptoms of a disease of a patient.

The anticancer agent binding to mitochondrion may be included in an amount of about 0.01 µg to 1 µg, about 0.05 µg to 0.5 µg, or about 0.1 µg to 0.3 µg per 1 µg of mitochondria. Preferably, it may be included in an amount of about 0.2 µg.

The anticancer agent and the mitochondrion may be mixed in an appropriate ratio so the anticancer agent may be contained in the mitochondrion. For example, the mixing ratio of the anticancer agent and the mitochondrion based on a weight ratio may be a weight ratio of about 1:10 to about 10:1, about 1:9 to about 9:1, about 1:8 to about 8:1, about 1:7 to about 7:1, about 1:6 to about 6:1, about 1:5 to about 5:1, about 1:4 to about 4:1, about 1:3 to about 3:1, about 1:2 to about 2:1, or about 1:1. Preferably, it may be about 1:5.

In particular, the anticancer agent may be one to which triphenylphosphonium (TPP) is bound.

As used herein, the term "triphenylphosphonium (TPP)" is a lipophilic cationic compound, which may easily penetrate cell membrane and mitochondrial membrane, and is absorbed directly into the mitochondrion in response to a negative voltage (-150 to -170 mV) present inside the mitochondrial membrane. Due to these properties, TPP is used as a material for targeting cancer cells, cancer stem cells and fibroblasts because it accumulates more in the mitochondria of cancer cells or cardiomyocytes having a large difference in the membrane potential of mitochondria compared to normal cells.

In this case, the anticancer agent in which TPP is bound to mitochondrion may be included in an amount of about 0.01 µg to 1 µg, about 0.05 µg to 0.5 µg, or about 0.1 µg to 0.3 µg per 1 µg of mitochondria. Preferably, it may be included in an amount of about 0.25 µg.

In addition, the anticancer agent and the mitochondrion to which TPP is bound may be mixed in an appropriate ratio so the anticancer agent may be contained in the mitochondrion. For example, the mixing ratio of the anticancer agent and the mitochondrion to which TPP is bound based on a weight ratio may be a weight ratio of about 1:10 to about 10:1, about 1:9 to about 9:1, about 1:8 to about 8:1, about 1:7 to about 7:1, about 1:6 to about 6:1, about 1:5 to about 5:1, about 1:4 to about 4:1, about 1:3 to about 3:1, about 1:2 to about 2:1, or about 1:1. Preferably, it may be about 1:4.

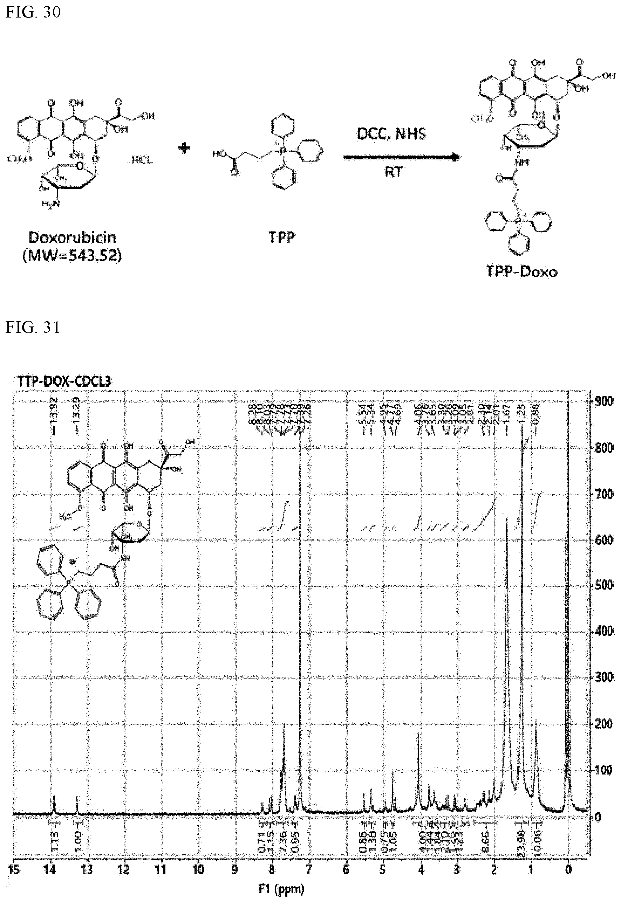

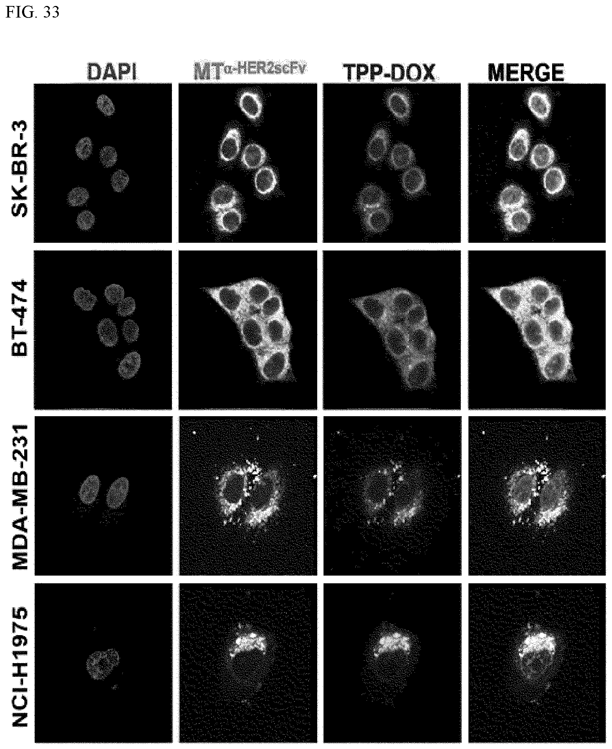

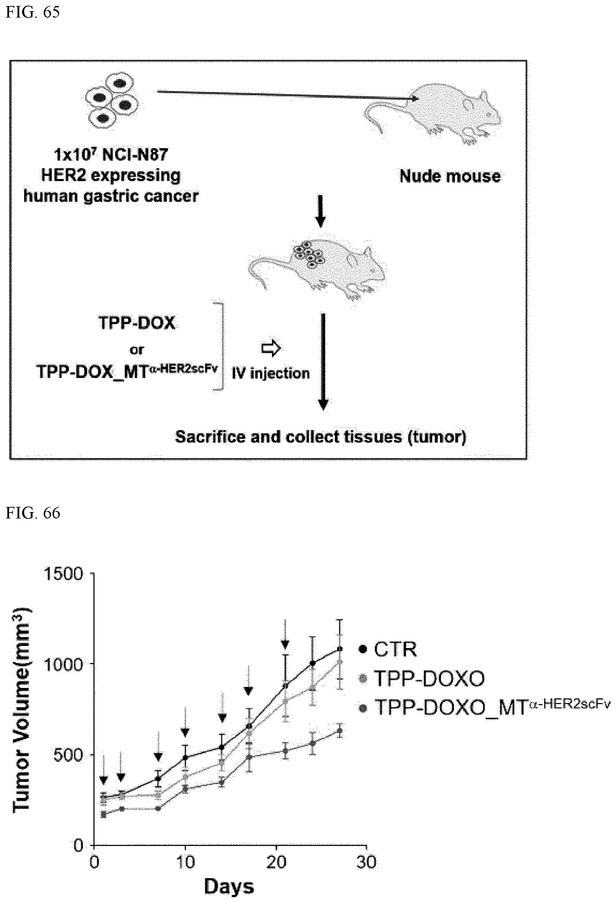

In one embodiment of the present specification, a mitochondrion (TPP-DOX_MT^{α-HER2scFv}) fused with an anti-HER2 antibody fragment comprising TPP-DOX in which TPP is bound to doxorubicin (DOX) was constructed (FIGS. 30 to 32), and the excellent anticancer activity was confirmed in a cancer cell (FIGS. 33 to 43) and a tumor mouse model (FIGS. 65 to 68) using the same.

In addition, the TPP may include a TPP derivative. The TPP derivative may be 2-butene-1,4-bis-TPP, 2-chlorobenzyl-TPP, 3-methylbenzyl-TPP, 2,4-dichlorobenzyl-TPP, 1-naphthylmethyl-TPP and p-xylylenebis-TPP and the like, but is not limited thereto. In addition, the TPP derivative may further include a derivative thereof. For example, it may be a derivative of 2-butene-1,4-bis-TPP, a derivative of 2-chlorobenzyl-TPP, a derivative of 3-methylbenzyl-TPP, a derivative of 2,4-dichlorobenzyl-TPP, a derivative of 1-naphthylmethyl-TPP and a derivative of p-xylylenebis-TPP and the like, but is not limited thereto.

The pharmaceutical composition comprising the mitochondrion containing the anticancer agent of the present invention may be for the prevention or treatment of cancer, and/or may increase therapeutic effect (efficacy). In this case, the mitochondrion and the anticancer agent are as described above.

The cancer may be any one selected from the group consisting of breast cancer, lung cancer, pancreatic cancer, glioblastoma, gastric cancer, liver cancer, colorectal cancer, prostate cancer, ovarian cancer, cervical cancer, thyroid cancer, laryngeal cancer, acute myeloid leukemia, brain tumor, neuroblastoma, retinoblastoma, head and neck cancer, salivary gland cancer and lymphoma, but is not limited thereto.

As used herein, the term "prevention" refers to any action that inhibits or delays the onset of cancer by administration of the pharmaceutical composition. In addition, "treatment" refers to any action that ameliorates or beneficially changes cancer symptoms by administration of the pharmaceutical composition.

As used herein, the term "efficacy" may be determined by one or more parameters, such as survival or disease-free survival over a period of time, such as 1 year, 5 years, or 10 years. In addition, the parameter may include the inhibition of the size of at least one tumor in a subject.

Pharmacokinetic parameters such as bioavailability and underlying parameters such as clearance rate may also have an influence on efficacy. Therefore, "enhanced efficacy" (for example, improvement in efficacy) may be attributed to enhanced pharmacokinetic parameters and enhanced efficacy, and may be measured by comparing clearance rate and tumor growth in a test animal or human subject, or by comparing parameters such as survival, recurrence rate or disease-free survival.

The preferred dosage of the pharmaceutical composition varies depending on the condition and body weight of the patient, the severity of disease, the form of drug, the route and duration of administration, but may be appropriately selected by those of ordinary skill in the art. In the pharmaceutical composition for the prevention or treatment of cancer of the present invention, the active ingredient may be included in any amount (effective amount) depending on the use, formulation, purpose of combining, and the like as long as it may exhibit anticancer activity. Here, "effective amount" refers to an amount of an active ingredient capable of inducing an anticancer effect. Such effective amount may be determined experimentally within the ordinary ability of those of ordinary skill in the art.

The pharmaceutical composition of the present invention may further comprise a pharmaceutically acceptable carrier. The pharmaceutically acceptable carrier may be any carrier as long as it is non-toxic material suitable for delivery to a patient. Distilled water, alcohol, fats, waxes and inert solids may be included as a carrier. In addition, a pharmaceutically acceptable adjuvant (buffering agent, dispersing agent) may be included in the pharmaceutical composition.

Specifically, the pharmaceutical composition may be prepared as a parenteral formulation according to the route of administration by a conventional method known in the art, including a pharmaceutically acceptable carrier in addition to the active ingredient. Here, "pharmaceutically acceptable" means that it does not inhibit the activity of the active ingredient and does not have toxicity beyond what the application (prescription) target may adapt.

When the pharmaceutical composition of the present invention is prepared as a parenteral formulation, it may be formulated in the form of an injection, a transdermal preparation and a nasal inhalant together with suitable carriers according to methods known in the art. When it is formulated as an injection, as a suitable carrier, sterile water, ethanol, polyol such as glycerol or propylene glycol, or a mixture thereof may be used, and Ringer's solution, PBS (Phosphate Buffered Saline) containing triethanolamine, or sterile water for injection, an isotonic solution such as 5% dextrose, and the like may be preferably used.

The pharmaceutical composition of the present invention may be an injectable preparation. Therefore, the pharmaceutical composition according to the present invention may be manufactured as an injection that is very stable physically or chemically by adjusting the pH using a buffer solution such as an acidic aqueous solution or phosphate, which may be used as an injection, in order to secure product stability according to the distribution of an injection that is prescribed.

Specifically, the pharmaceutical composition of the present invention may comprise water for injection. The water for injection refers to distilled water prepared for dissolving a solid injection or for diluting a water-soluble injection. It may be glucose injection, xylitol injection, D-mannitol injection, fructose injection, physiological saline, dextran 40 injection, dextran 70 injection, amino acid injection, Ringer's solution, lactic acid-Ringer's solution, or a phosphate buffer solution or sodium dihydrogen phosphate-citrate buffer solution having a pH of about 3.5 to 7.5.

On the other hand, the pharmaceutical composition of the present invention is administered in a pharmaceutically effective amount. The term "therapeutically effective amount" or "pharmaceutically effective amount" refers to an amount of a compound or composition effective for preventing or treating a target disease, which is an amount that is sufficient to treat the disease with a reasonable benefit/risk ratio applicable to medical treatment and that does not cause side effects. The level of the effective amount may be determined according to factors including the health condition of the patient, the type and severity of the disease, the activity of the drug, sensitivity to the drug, administration method, administration time, the route of administration and excretion rate, treatment period, the drug to be combined or concurrently used, and other factors well known in the medical field. In one embodiment, a therapeutically effective amount refers to an amount of a drug effective to treat cancer.

As used herein, the term "administration" refers to introducing a predetermined substance to a subject by an appropriate method, and the route of administration of the composition may be through any general route as long as it may reach a target tissue. It may be intraperitoneal administration, intravenous administration, intramuscular administration, subcutaneous administration, intradermal administration, topical administration, intranasal administration, intrarectal administration, but is not limited thereto.

A preferred dosage of the pharmaceutical composition of the present invention may be administered once in an amount of 0.01 mg/kg to 5 mg/kg, 0.1 mg/kg to 4 mg/kg or 0.25 mg/kg to 2.5 mg/kg of mitochondria based on the body weight of the subject to be administered, but is not limited thereto. That is, it is most preferable in terms of cell activity that the pharmaceutical composition is administered with the modified mitochondrion containing the anticancer agent in an amount in the above range based on the body weight of the subject having cancer tissue. In addition, the pharmaceutical composition may be administered 1 to 10 times, 3 to 8 times, or 5 to 6 times, preferably 5 times. In this case, the administration interval may be an interval of 1 to 7 days or 2 to 5 days, preferably an interval of 3 days. Such dosage should not be construed as limiting the scope of the present invention in any aspect.

The term "subject" refers to a subject to which the composition of the present invention may be applied (prescribed), and may be a subject suffering from cancer. In addition, the subject may be a mammal, such as a rat, a mouse, or a livestock, including a human, and preferably a human. The composition of the present invention may further include any compound or natural extract known to have anticancer activity and safety, which has already been verified, for the enhancement and reinforcement of anticancer activity, in addition to the mitochondrion containing the anticancer agent. In this case, the pharmaceutical composition and the compound or natural extract having anticancer activity may be administered simultaneously or sequentially.

In another aspect of the present invention, there is provided a method for preparing mitochondrion containing an anticancer agent, comprising mixing the anticancer agent and the isolated mitochondrion. In this case, the anticancer agent and the mitochondrion are as described above.

The anticancer agent and the mitochondrion may be mixed in an appropriate ratio so the anticancer agent may be contained in the mitochondrion. For example, the mixing ratio of the anticancer agent and the mitochondrion based on a weight ratio may be a weight ratio of about 1:10 to about 10:1, about 1:9 to about 9:1, about 1:8 to about 8:1, about 1:7 to about 7:1, about 1:6 to about 6:1, about 1:5 to about 5:1, about 1:4 to about 4:1, about 1:3 to about 3:1, about 1:2 to about 2:1 or about 1:1. Preferably, it may be about 1:5.

In another aspect of the present invention, there is provided a modified anticancer agent to which TPP is chemically bound. In this case, TPP and the anticancer agent are as described above.

In another aspect of the present invention, there is provided use of a pharmaceutical composition comprising mitochondrion containing an anticancer agent for the prevention or the enhancement of therapeutic effect of cancer. In this case, the anticancer agent and the mitochondrion are as described above.

In another aspect of the present invention, there is provided a method for treating cancer and/or a method for enhancing therapeutic effect, comprising administering a pharmaceutical composition comprising mitochondrion containing an anticancer agent to a subject. In this case, the anticancer agent, the mitochondrion and the administration are as described above. The subject may be a subject suffering from cancer. In addition, the subject may be a mammal, preferably a human.

### Mode for Carrying out the Invention

Hereinafter, the present invention will be described in more detail by way of the following examples. However, the following examples are only for illustrating the present invention, and the scope of the present invention is not limited thereto.

### I. Confirmation of targeting ability of mitochondria to which targeting protein is bound

### Preparation Example 1. Preparation of HEK293 cell line comprising mitochondrion fused with anti-HER2scFv antibody

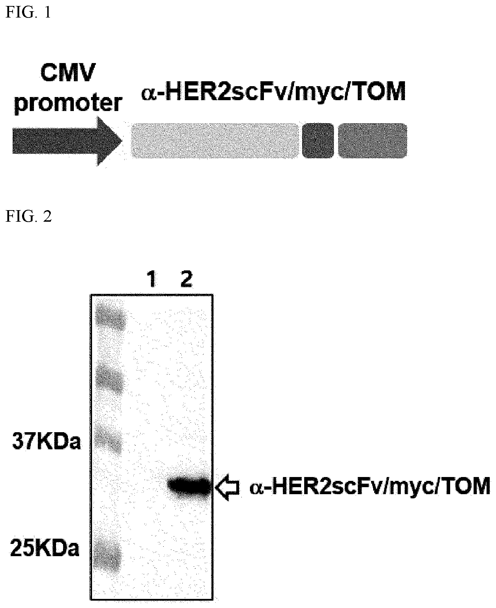

In order to specifically introduce mitochondria into tumor cells in which HER2 is expressed, mitochondria in which an anti-HER2scFv antibody (SEQ ID NO: 98) that specifically binds to HER2 is fused to the mitochondrial outer membrane were construct (FIGS. 1 and 2). A specific constructing method was performed according to the method described in Korean Patent Application Publication No. 10-2019-0124656.

### Example 1. Confirmation of property of mitochondria fused with anti-HER2scFv antibody

### Example 1.1. Analysis of anti-HER2scFv antibody expression

In order to confirm the expression level of anti-HER2scFv in HEK293 cells comprising the mitochondria (MT^{α-HER2scFv}) fused with an anti-HER2scFv antibody constructed in Preparation Example 1 above, the cells were stained using immunocytochemistry staining, and the images were observed using a confocal microscope.

Specifically, HEK293 cells were inoculated into a 24-well plate at 3×10⁴ cells/well and cultured for 24 hours. After culturing for 24 hours, the cells were stained with MitoTracker Red for about 30 minutes, and washed with PBS, and then fixed with 3.7% formaldehyde. Formaldehyde was removed, and a primary antibody, anti-myc antibody (Roche, 11667149001), was diluted 1:1,000 in PBS containing 1% goat serum, and the cells were treated with the dilution and reacted for 18 hours. After the reaction was completed, the primary antibody was removed by washing with PBS, and then a secondary antibody labeled with Alexa Fluor^{®} 488 (Invitrogen, 11001), an anti-mouse IgG antibody, was diluted 1:500 in PBS containing 0.1% BSA, and the cells were treated with the dilution and reacted for about 1 hour. After the reaction was completed, the cells were washed with PBS, mounted, and the cells were observed by a confocal microscope. At this time, the nuclei of the cells were observed by staining with DAPI.

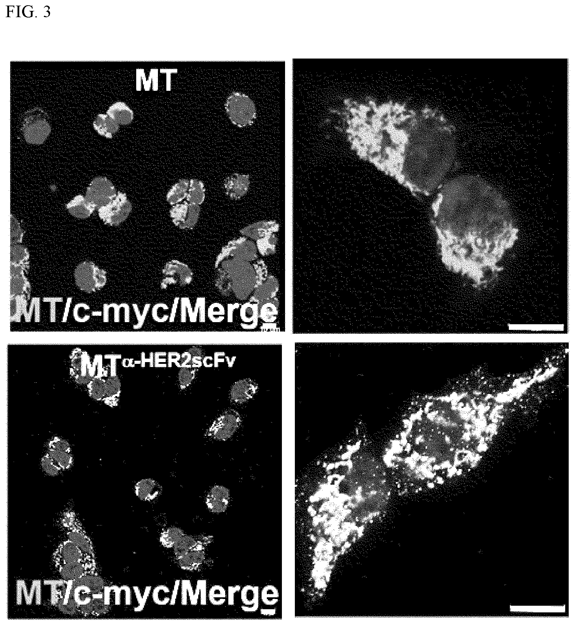

As a result, it was confirmed that the location of the red fluorescence labeling the mitochondria is coincident with the location of the green fluorescence labeling the anti-HER2scFv antibody (FIG. 3).

### Example 1.2. Confirmation of expression location of anti-HER2scFv antibody

Western blot was performed to confirm whether the anti-HER2scFv antibody constructed in the same manner as in Preparation Example 1 above was expressed in the mitochondria of HEK293 cells.

Specifically, after disrupting the cells in which anti-HER2scFv was expressed, the cell extract was separated into a mitochondria fraction and a cytoplasm fraction by centrifugation. Each of the fractions was electrophoresed and Western blot was performed. In order to identify anti-HER2scFv, a mitochondrial marker and a cytoplasmic marker protein, anti-myc antibody (Roche, 11667149001), anti-COX4 antibody (abcam, 33985) and anti-β-tubulin antibody (Thermo, MA5-16308) were used as primary antibodies, respectively. Anti-mouse IgG HRP or anti-rabbit IgG HRP was used as a secondary antibody.

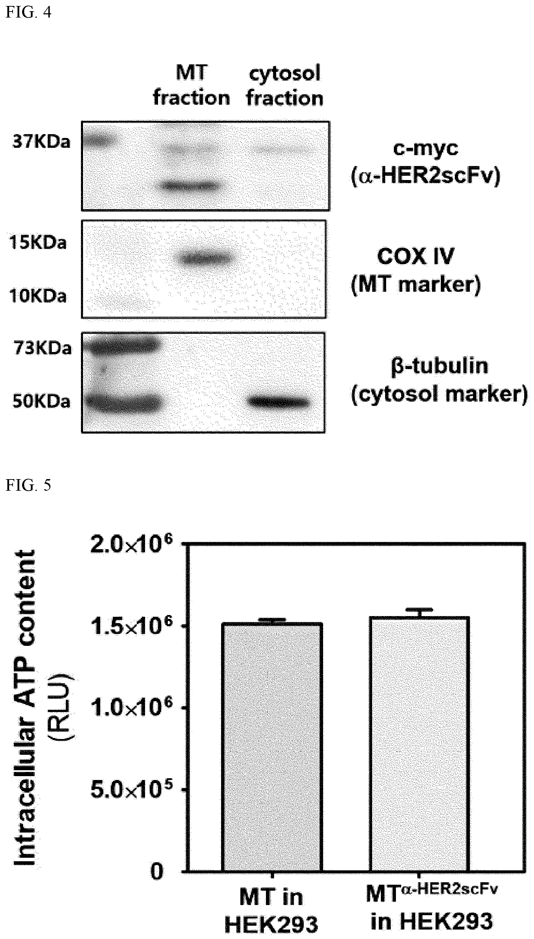

As a result, it was confirmed that the anti-HER2scFv antibody was present in the mitochondria fraction together with COX4 used as a mitochondrial positive marker, and was not present in the fraction in which the cytoplasmic positive marker β-tubulin was present (FIG. 4). It was confirmed that the anti-HER2scFv antibody was expressed in the mitochondria of HEK293 cells through the results of immunocytochemistry staining and Western blot analysis in the above Example.

### Example 1.3. Measurement of ability to produce ATP of mitochondria fused with anti-HER2scFv antibody

In order to compare the functional difference between the mitochondria fused with the anti-HER2scFv antibody constructed in the same manner as in Preparation Example 1 above and the normal mitochondria, the mitochondria were isolated from HEK293 cells (MT^{α-HER2scFv}), a cell line expressing the mitochondria fused with the anti-HER2scFv antibody, and HEK293 cells to compare their ability to produce ATP, which is one of the main functions of mitochondria.

Specifically, the ability to produce ATP was confirmed by treating 5 µg/100 µL of the isolated mitochondria with 25 µM ADP and 100 µL of luciferin, and measuring the luminescence value (RLU) generated by the reaction of ATP and luciferin produced in the mitochondria. As a result, there was no difference in the ability to produce ATP of MT and MT^{α-HER2scFv} (FIG. 5).

### Example 1.4. Measurement of membrane potential of mitochondria fused with anti-HER2scFv antibody

In order to compare the functional difference between the mitochondria fused with the anti-HER2scFv antibody constructed in the same manner as in Preparation Example 1 above and the normal mitochondria, the membrane potential, which is one of the main characteristics of mitochondria, of HEK293 cells comprising the mitochondria fused with the anti-HER2scFv antibody (MT^{α-HER2scFv}) and untreated HEK293 cells (MT) was compared.

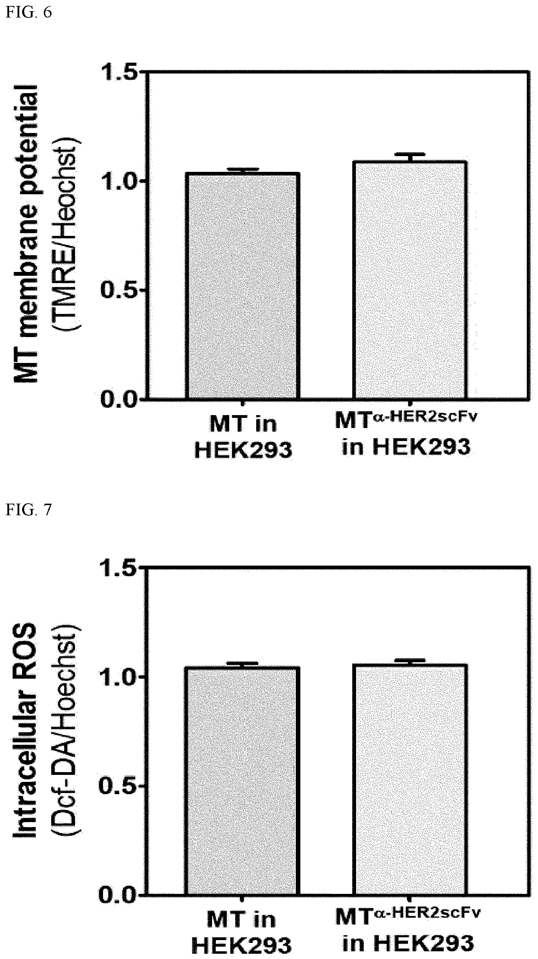

Specifically, the cells were inoculated into a 96-well plate at 3×10⁴ cells/well and cultured for 24 hours. After 24 hours, the cells were treated with DCFDA (Invitrogen, C6827) dye and reacted for 1 hour. Thereafter, the cells were washed with PBS and treated with Hoechst33242 to stain the cells for 10 minutes. The fluorescence values of DCFDA and Hoechst33242 were measured, and then the membrane potential was determined by calculating their ratio (the fluorescence value of DAFDA/the fluorescence value of Hoechst33242). As a result, there was no difference in the membrane potential of MT and MT^{α-HER2scFv} (FIG. 6).

### Example 1.5. Measurement of reactive oxygen species of mitochondria fused with anti-HER2scFv antibody

In order to compare the production level of the reactive oxygen species (ROS) of the mitochondria fused with the anti-HER2scFv antibody constructed in the same manner as in Preparation Example 1 above and the normal mitochondria, the reactive oxygen species of the mitochondria in HEK293 cells comprising the mitochondria fused with the anti-HER2scFv antibody (MT^{α-HER2scFv}) and untreated HEK293 cells (MT) was measured.

Specifically, the cells were inoculated into a 96-well plate at 3×10⁴ cells/well and cultured for 24 hours. After 24 hours, each cell was treated with TMRE (Invitrogen, T669) dye and reacted for 1 hour. The cells were washed with PBS and then stained with Hoechst33242 for 10 minutes. The fluorescence values of TMRE and Hoechst33242 were measured, and then the amount of the reactive oxygen species production was determined by calculating their ratio (the fluorescence value of TMRE/the fluorescence value of Hoechst33242). As a result, there was no difference in the amount of the reactive oxygen species production of MT and MT^{α-HER2scFv} isolated from cells (FIG. 7).

### Example 2. Analysis of HER2 expression in human cancer cell line and normal cell line

### Example 2.1. Confirmation of HER2 expression in human cancer cell line and normal cell line through Western blot analysis

Western blot was performed to confirm the expression level of HER2 in the human breast cancer, lung cancer, gastric cancer cell line and the lung fibroblast cell line.

Specifically, the human breast cancer cell line (SK-BR-3, BT-474 and MDA-MB-231), the human lung cancer cell line (NCI-H1975), the human gastric cancer cell line (MKN-74 and NCI-N87) and the human lung fibroblast cell line (WI-38) were inoculated into a 6-well plate at about 1×10⁶ cells/well and then cultured for 24 hours. At this time, SK-BR-3, BT-474 and NCI-N87 cells were prepared by culturing in RPMI-1640 medium containing 10% FBS, and MDA-MB-231, NCI-H1975, MKN-74 and WI-38 cells were prepared by culturing in DMEM medium containing 10% FBS.

After 24 hours, the culture solution was removed, and the cells were washed twice with PBS, and then 100 µL of RIPA buffer containing a protease inhibitor was added directly to the cells. After 10 minutes, the cells were recovered using a scraper, transferred to a microtube, and then centrifuged at about 12,000 xg for 10 minutes. The separated supernatant was obtained and transferred to a new microtube, and then protein was quantified using the BCA analysis. The same amount of protein for each sample was electrophoresed, and then Western blot was performed. Anti-HER2 antibody (Cell signaling, 29D8) and anti-β actin antibody (Sigma, A2208) were used as primary antibodies, and anti-mouse IgG HRP and anti-rabbit IgG HRP were used as secondary antibodies.

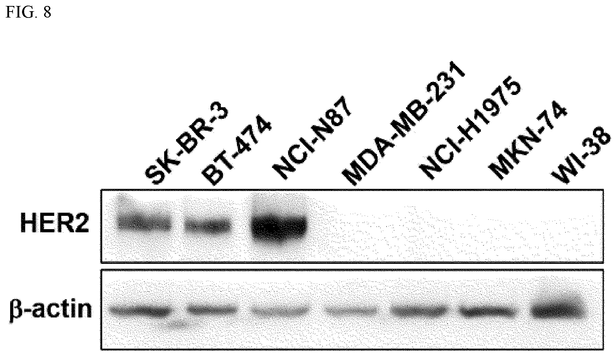

As a result, it was confirmed that SK-BR-3, BT-474, and NCI-N87 cells were HER2⁺ cells expressing HER2. On the other hand, it was found that MDA-MB-231, NCI-H1975, MKN-74 and WI-38 cells showed almost no HER2 expression, indicating that they were HER2⁻ cells (FIG. 8).

### Example 2.2. Confirmation of HER2 expression in human cancer cell line and normal cell line through immunocytochemistry staining

In order to confirm the expression level of HER2 in the human breast cancer, lung cancer, gastric cancer cell line and the human lung fibroblast cell line, immunocytochemistry staining was performed using an anti-HER2 antibody.

Specifically, the cells were inoculated into a 24-well plate at 3×10⁴ cells/well, respectively, and cultured for 24 hours. After 24 hours, the cells were fixed with 3.7% formaldehyde. Formaldehyde was removed, and a primary antibody, anti-HER2 antibody (Cell Signaling Technology, 2165), was diluted 1:1,000 in PBS containing 1% goat serum, and the cells were treated with the dilution and reacted for 18 hours. The primary antibody was removed by washing with PBS, and then a secondary antibody labeled with Alexa Fluor^{®} 488 (Invitrogen, 11008), an anti-rabbit IgG antibody, was diluted 1:500 in PBS containing 0.1% BSA, and the cells were treated with the dilution and reacted for 1 hour. After the reaction was completed, the cells were washed with PBS, mounted, and the cells were observed by a confocal microscope. At this time, the nuclei of the cells were observed by staining with DAPI.

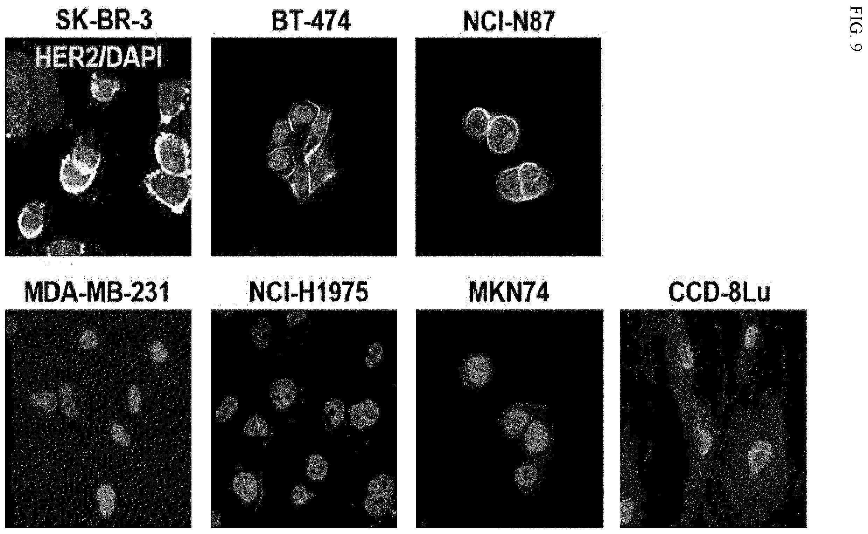

As a result, in the case of SK-BR-3, BT-474, and NCI-N87 cells, which are HER2⁺cells, HER2 was expressed on the cell surface and green fluorescence was observed, and in the case of MDA-MB-231, NCI-H1975, MKN74, and CCD-8Lu cells, which are HER2⁻ cells, green fluorescence was not observed (FIG. 9).

### Example 2.3. Confirmation of HER2 expression in human cancer cell line through flow cytometry

In order to confirm the expression level of HER2 in the human breast cancer, lung cancer and gastric cancer cell line, flow cytometry was performed using an anti-HER2 antibody.

Specifically, the human breast cancer cell line (SK-BR-3, BT-474 and MDA-MB-231), the human gastric cancer cell line (NCI-N87 and MKN-74) and the human lung cancer cell line (NCI-H1975) were inoculated into a 6-well plate at about 1×10⁶ cells/well, respectively, and cultured for 24 hours. After 24 hours, the culture solution was removed, and the cells were collected by treatment with 0.05% trypsin-EDTA and fixed with 3.7% formaldehyde. A primary antibody, an anti-HER2 antibody (Cell Signaling Technology, 2165), was diluted 1:1,000 in PBS containing 1% goat serum, and the cells were treated with the dilution and reacted for 18 hours. The primary antibody was removed by washing with PBS, and then a secondary antibody labeled with Alexa Fluor^{®} 555 (Invitrogen, 21428), an anti-rabbit IgG antibody, was diluted 1:500 in PBS containing 0.1% BSA, and the cells were treated with the dilution and reacted for 1 hour. After the reaction was completed, the cells were washed with PBS, and the expression of HER2 was confirmed through flow cytometer.

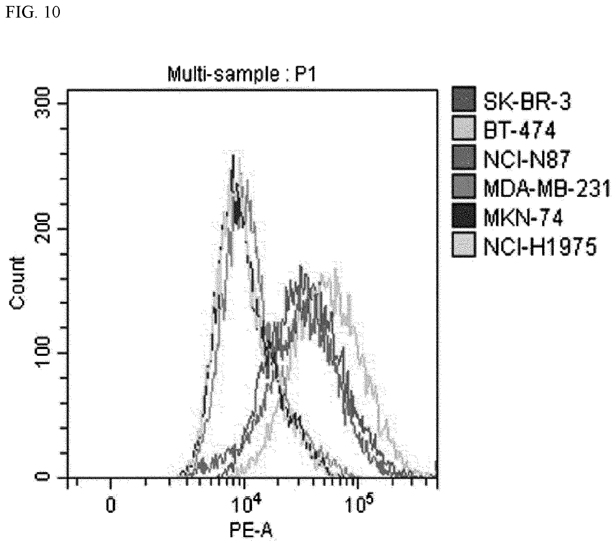

As a result, in the case of SK-BR-3, BT-474 and NCI-N87 cells, which are HER2⁺ cells, HER2 was expressed on the cell surface and the fluorescence value (PE-A) was observed to be high, whereas MDA-MB-231, NCI-H1975 and MKN-74 cells, which are HER2⁻ cells, showed lower fluorescence value than HER2⁺ cells (FIG. 10).

From the above results of the Western blot analysis, immunocytochemistry staining and flow cytometry, it was confirmed that SK-BR-3, BT-474 and NCI-N87 cells were HER2⁺ cells expressing HER2, and MDA-MB-231, NCI-H1975, MKN-74, WI-38 cells, and CCD-8Lu cells were HER2⁻cells.

### Example 3. Verification of HER2 targeting of mitochondria fused with anti-HER2scFv antibody

In order to confirm the HER2 targeting ability of the mitochondria fused with the anti-HER2scFv antibody constructed in the same manner as in Preparation Example 1 above, the mitochondria fused with the anti-HER2scFv antibody (MT^{α-HER2scFv}) were treated with the co-cultured HER2⁺ cells and HER2⁻ cells, and then immunocytochemistry staining was performed.

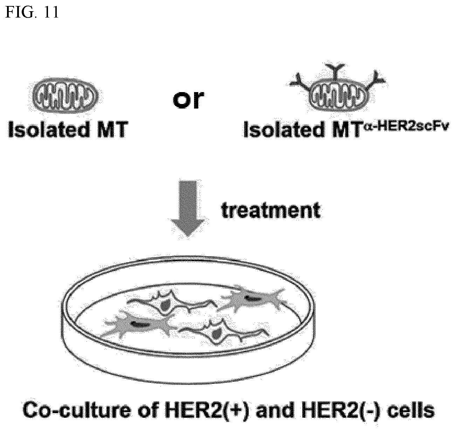

Specifically, HER2⁺cells and HER2⁻cells were inoculated together into a 24-well plate at 1.5×10⁴ cells/well and co-cultured in DMEM medium containing 10% FBS for 24 hours. At this time, HER2⁺ cells SK-BR-3, BT-474 and NCI-N87 were prepared by culturing in RPMI-1640 medium containing 10% FBS, and HER2⁻ cells MDA-MB-231, NCI-H1975 and MKN-74 were prepared by culturing in DMEM medium containing 10% FBS. The mitochondria were prepared by treating untreated HEK293 cells (MT) and HEK293 cells comprising mitochondria fused with an anti-HER2scFv antibody (MT^{α-HER2scFV}) with MitoTracker Red, respectively, and isolating the stained mitochondria. After co-culture for 24 hours, each isolated mitochondrion was treated with the co-cultured cells and reacted for 12 hours (FIG. 11).

### Example 3.1. Confirmation of location of mitochondria fused with anti-HER2scFv antibody in HER2 positive and negative cell lines

The cells treated in the same manner as in Example 3 above were washed once with PBS, and fixed by adding 3.7% formaldehyde at room temperature for 30 minutes, and then immunocytochemistry staining was performed. At this time, an anti-HER2 antibody (Cell signaling, 29D8) was used as a primary antibody, and an anti-rabbit IgG antibody labeled with Alexa Fluor^{®} 488 (Invitrogen, 11008) was used as a secondary antibody. The nuclei of the cells were observed by staining with DAPI.

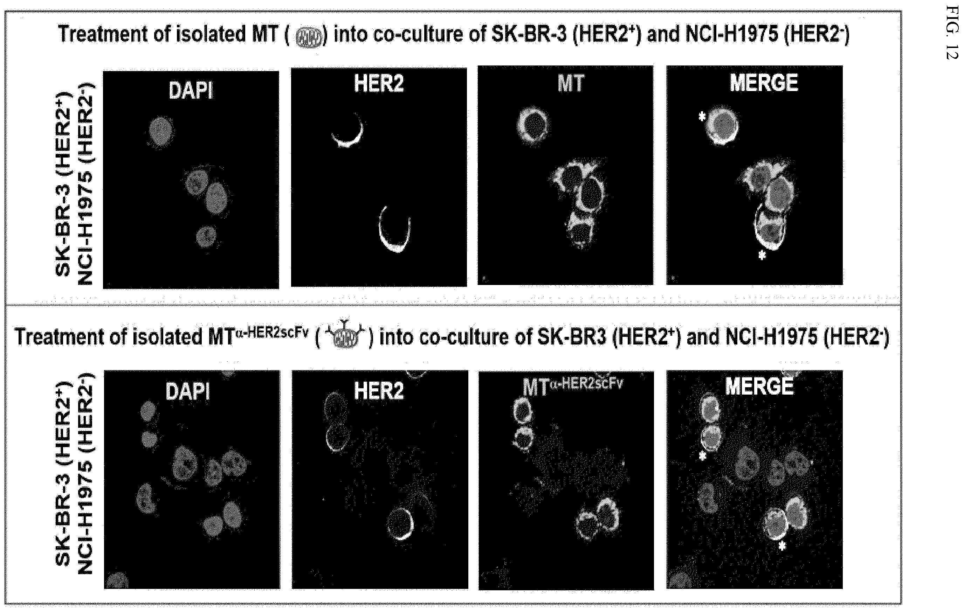

As a result, as shown in FIG. 12, when the co-cultured SK-BR-3 (HER2⁺) and NCI-H1975 (HER2⁻) cells were treated with untreated HEK293 cell-derived mitochondria (MT), the mitochondria stained with Mitotracker Red (red fluorescence) were observed equally in SK-BR-3 (HER2⁺) cells and NCI-H1975 (HER2) cells. On the other hand, when the cells were treated with the mitochondria (MT^{α-HER2scFV}) derived from HEK293 cells comprising the mitochondria fused with the anti-HER2scFv antibody, the mitochondria stained with Mitotracker Red were observed specifically in HER2⁺ cells, SK-BR-3 cells.

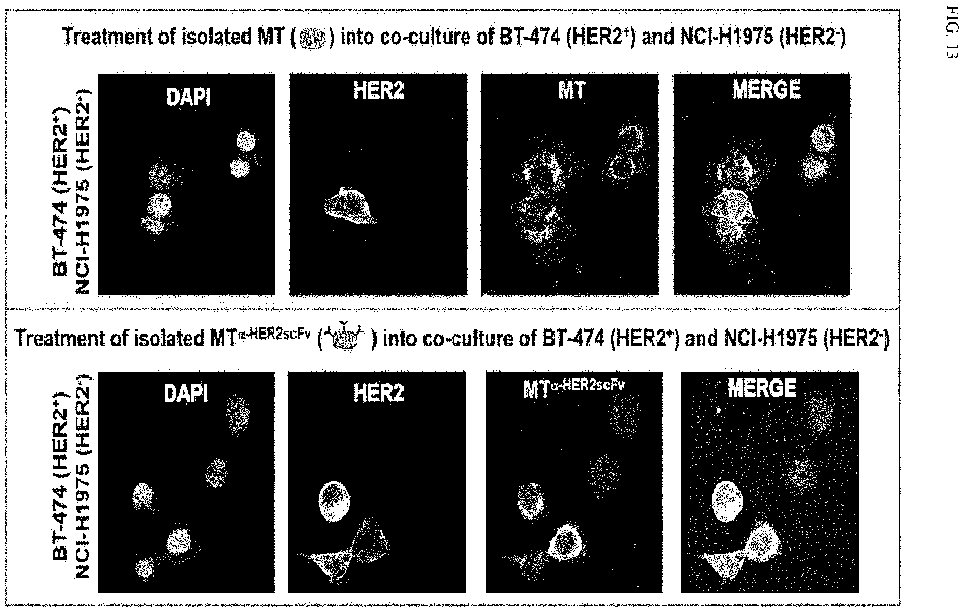

In the case of the co-cultured BT-474 (HER2⁺) and NCI-H1975 (HER2) cells, in the case of MT treated group, the mitochondria stained with Mitotracker Red were observed equally in BT-474 (HER2⁺) cells and NCI-H1975 (HER2⁻) cells, whereas in the case of MT^{α-HER2scFv} treated group, they were specifically observed only in HER2⁺cells, BT-474 cells (FIG. 13).

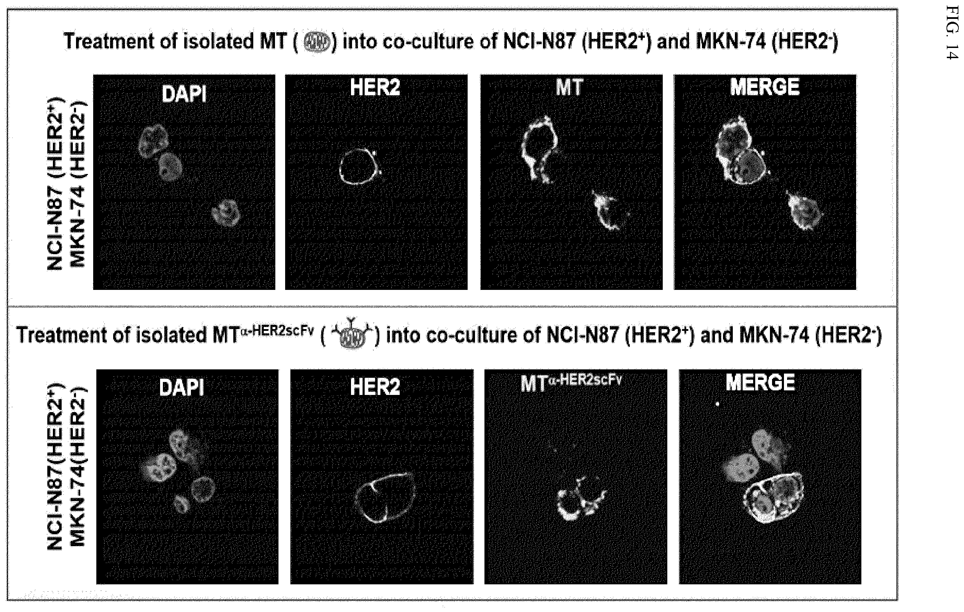

In the case of the co-cultured NCI-N87 (HER2⁺) and MKN-74 (HER2) cells, in the case of MT treated group, the mitochondria stained with Mitotracker Red were observed equally in NCI-N87 (HER2⁺) cells and MKN-74 (HER2) cells, whereas in the case of MT^{α-HER2scFv} treated group, they were specifically observed only in HER2⁺cells, NCI-N87 (FIG. 14).

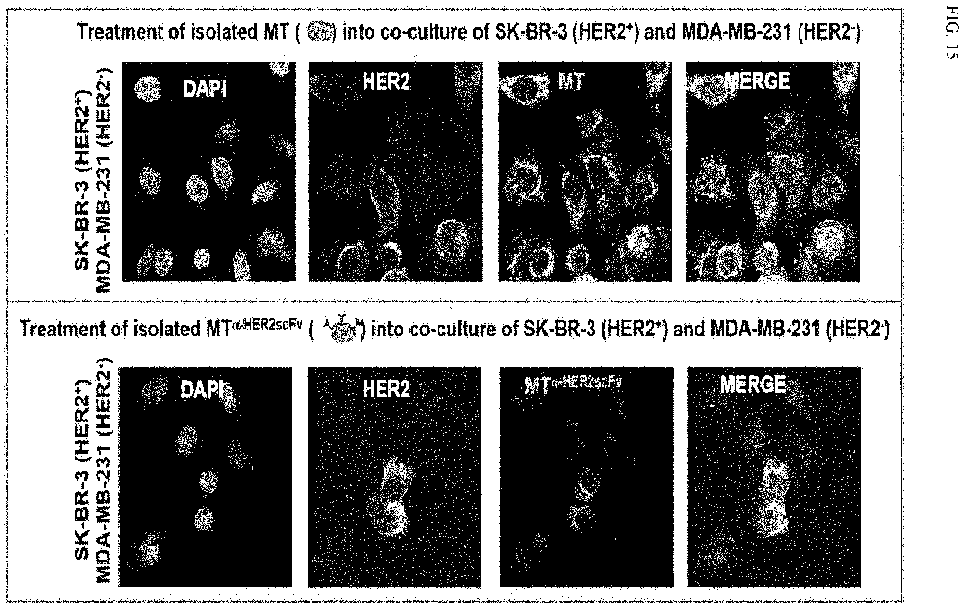

In the case of the co-cultured SK-BR-3 (HER2⁺) and MDA-MB-231 (HER2) cells, in the case of MT treated group, the mitochondria stained with Mitotracker Red were observed equally in SK-BR-3 (HER2⁺) cells and MDA-MB-231 (HER2⁻) cells, whereas in the case of MT^{α-HER2scFv} treated group, they were specifically observed only in HER2⁺cells, SK-BR-3 cell (FIG. 15).

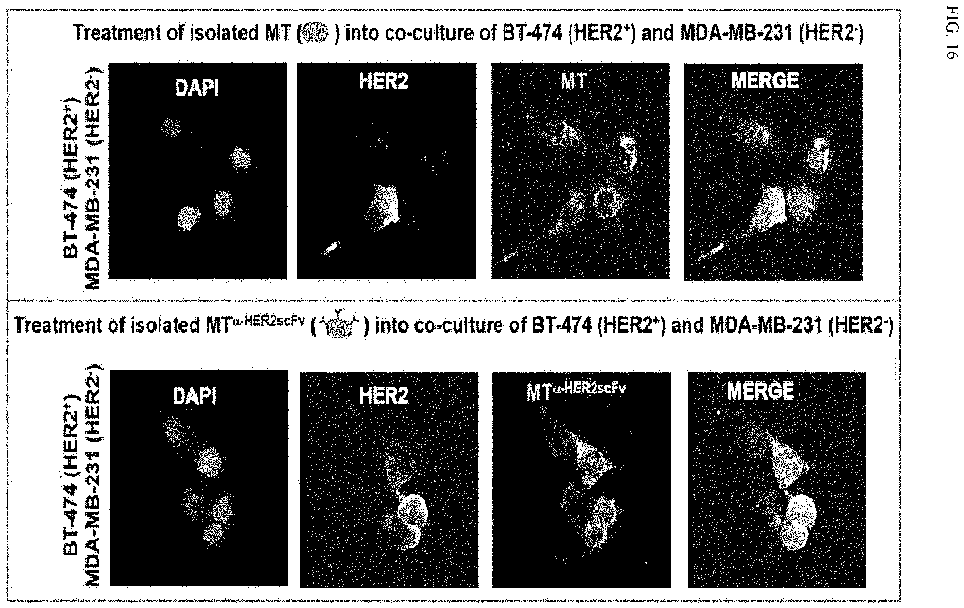

In the case of the co-cultured BT-474 (HER2⁺) and MDA-MB-231 (HER2) cells, in the case of MT treated group, the mitochondria stained with Mitotracker Red were observed equally in BT-474 (HER2⁺) cells and MDA-MB-231 (HER2⁻) cells, whereas in the case of MT^{α-HER2scFv} treated group, they were specifically observed only in HER2⁺cells, BT-474 cell (FIG. 16).

From the above results, it was confirmed that the mitochondria fused with the anti-HER2scFv antibody (MT^{α-HER2scFv}) could specifically target HER2⁺ cells expressing a HER2 antigen.

### II. Preparation of mitochondria in which an anticancer agent is contained and a targeting protein is bound, and confirmation of their activity

### Preparation Example 2. Preparation of complex of mitochondria fused with anti-HER2scFv antibody and doxorubicin

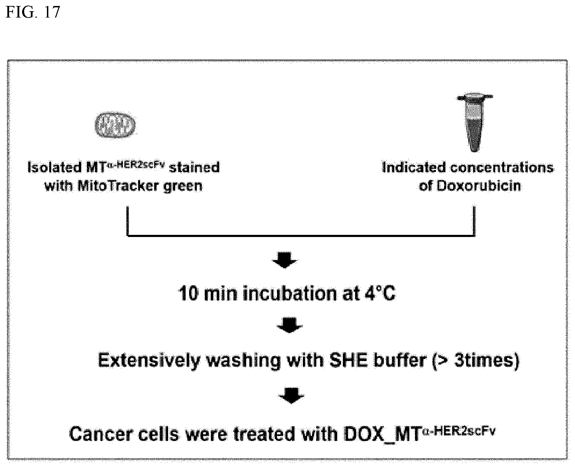

HEK293 cells comprising mitochondria fused with an anti-HER2scFv antibody were treated with MitoTracker green to stain the mitochondria (green fluorescence), and then the mitochondria (MT^{α-HER2scFv}) were isolated. 30 µg of the isolated mitochondria were reacted with 100 µM doxorubicin (DOX) at 4°C for 10 minutes, and then centrifuged at about 12,000 xg for 10 minutes to remove unreacted doxorubicin. Thereafter, they were washed twice with 500 µL of SHE (250 mM Sucrose, 20 mM HEPES (pH 7.4), 2 mM EGTA) buffer to finally obtain the mitochondria comprising doxorubicin (DOX_MT^{α-HER2scFv}) (FIG. 17).

### Example 4. Evaluation of anticancer activity of DOX_MT^{α-HER2scFv} against cancer cells

### Example 4.1. Confirmation of delivery property of DOX_MT^{α-HER2scFv} into cancer cells

The human breast cancer cell line (SK-BR-3, BT-474 and MDA-MB-231) and the human lung cancer cell line (NCI-H1975) were treated with DOX_MT^{α-HER2scFv} obtained in the same manner as in Preparation Example 2 above, respectively, and then the cells were observed by a confocal microscope. At this time, the nuclei of the cells were observed by staining with DAPI.

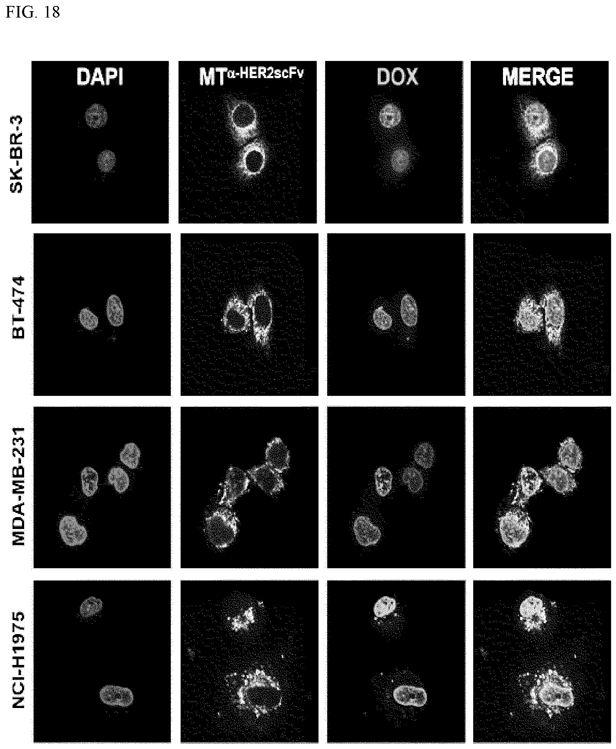

As a result, the mitochondria stained with MitoTracker green (MT^{α-HER2scFv}, green fluorescence) were observed in the cytoplasm, and doxorubicin (DOX) showing red fluorescence was observed in the nucleus (FIG. 18). From the above results, it was found that doxorubicin of DOX_MT^{α-HER2scFv} transported into the cell moved to the nucleus and was located in DNA in the nucleus.

### Example 4.2. Confirmation of morphological change in cancer cells by treatment with DOX MT^{α-HER2scFv}

The human breast cancer cell line (SK-BR-3 and BT-474) and the human gastric cancer cell line (NCI-N87) were treated with DOX_MT^{α-HER2scFv} obtained in the same manner as in Preparation Example 2 above, respectively, and then the morphological change in cancer cells over time was observed by a confocal microscope. At this time, the nuclei of the cells were observed by staining with DAPI.

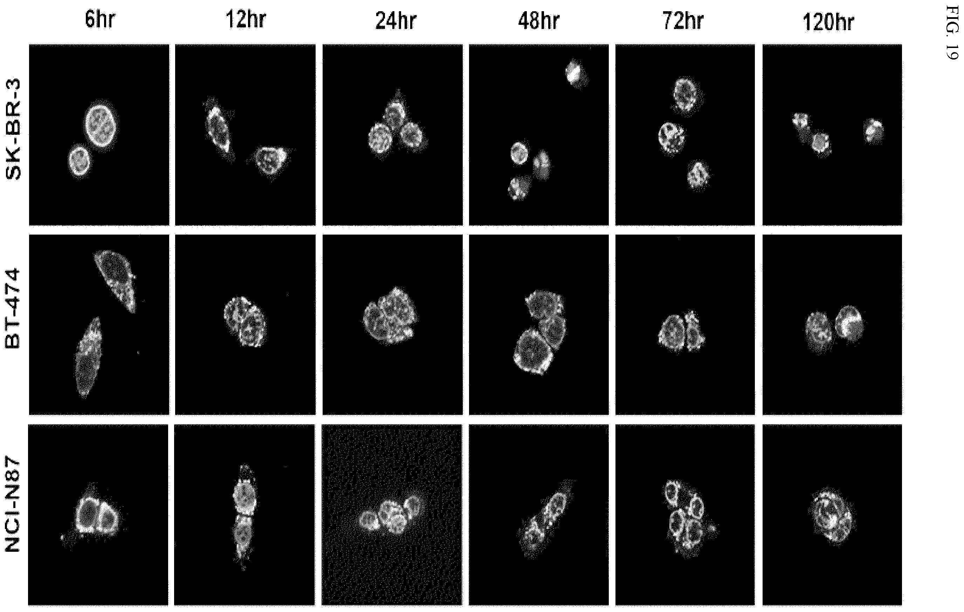

As a result, it was observed that most of doxorubicin (DOX, red fluorescence) exists together in the mitochondria (green fluorescence) after 6 hours of treatment with DOX_MT^{α-HER2scFv} (yellow fluorescence). However, doxorubicin was observed in the nucleus over time, and the morphological change in the cells was remarkably observed after 72 hours of treatment with DOX_MT^{α-HER2scFv} (FIG. 19).

### Example 4.3. Determination of concentration of doxorubicin bound to MT^{α-HER2scFv}

In order to quantify the concentration of doxorubicin bound to DOX_MT^{α-HER2scFv} obtained in the same manner as in Preparation Example 2 above, a standard curve was prepared by measuring the absorbance for each concentration of doxorubicin, and then the concentration of doxorubicin bound to DOX MT^{α-HER2scFv} was calculated.

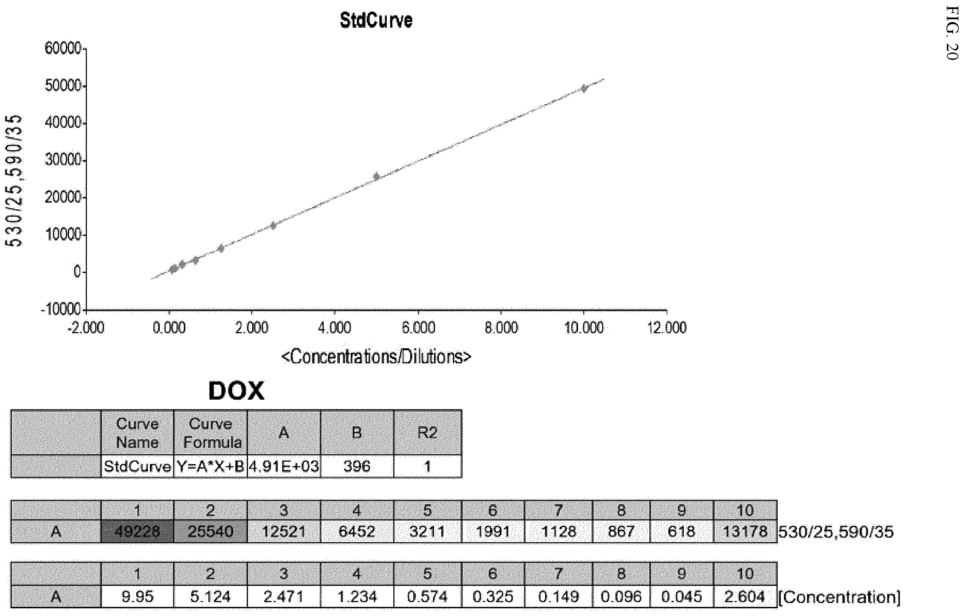

Specifically, 100 µL each of doxorubicin solution at a concentration of 20 µM, 10 µM, 5 µM, 2.5 µM, 1.25 µM, 0.625 µM, and 0 µM, and DOX_MT^{α-HER2scFv} diluted 1/20 in PBS was dispensed into a 96-well plate. Thereafter, the absorbance (emission wavelength of 590 nm) was measured with a multi-reader (BioTek, Synerge HTX). A standard curve was prepared using the absorbance values according to the concentration of doxorubicin, and then the concentration of doxorubicin bound to DOX MT^{α-HER2scFv} was determined by substituting the absorbance values of the DOX_MT^{α-HER2scFv} diluted solution (FIG. 20).

### Example 4.4. Confirmation of cancer cell proliferation inhibitory ability by treatment with DOX_MT^{α-HER2scFv} for each concentration

In order to confirm the anticancer activity of DOX_MT^{α-HER2scFv} obtained in the same manner as in Preparation Example 2 above using 200 µg of MT^{α-HER2scFv} and 100 µM doxorubicin (DOX), the cell proliferation inhibitory effect by treating the human breast cancer, lung cancer and gastric cancer cell lines with DOX_MT^{α-HER2scFv} was evaluated.

Specifically, the human breast cancer (SK-BR-3, BT-474 and MDA-MB-231), lung cancer (NCI-H1975) and gastric cancer (NCI-N87 and MKN-74) cell lines were inoculated into a 96-well plate at about 3×10³ cells/well, respectively, and cultured for 24 hours. At this time, SK-BR-3, BT-474 and NCI-N87 cells were cultured in RPMI-1640 medium containing 10% FBS, and MDA-MB-231, NCI-H1975 and MKN-74 cells were cultured in DMEM medium containing 10% FBS. After culturing for 24 hours, each cell was treated with doxorubicin (DOX) or DOX_MT^{α-HER2scFv} at a concentration of 2 µM, 1 µM, 0.5 µM, 0.25 µM, 0.1 µM, 0.05 µM, 0.025 µM, and 0.01 µM. After 144 hours of sample treatment, cell proliferation was measured by WST-1 assay.

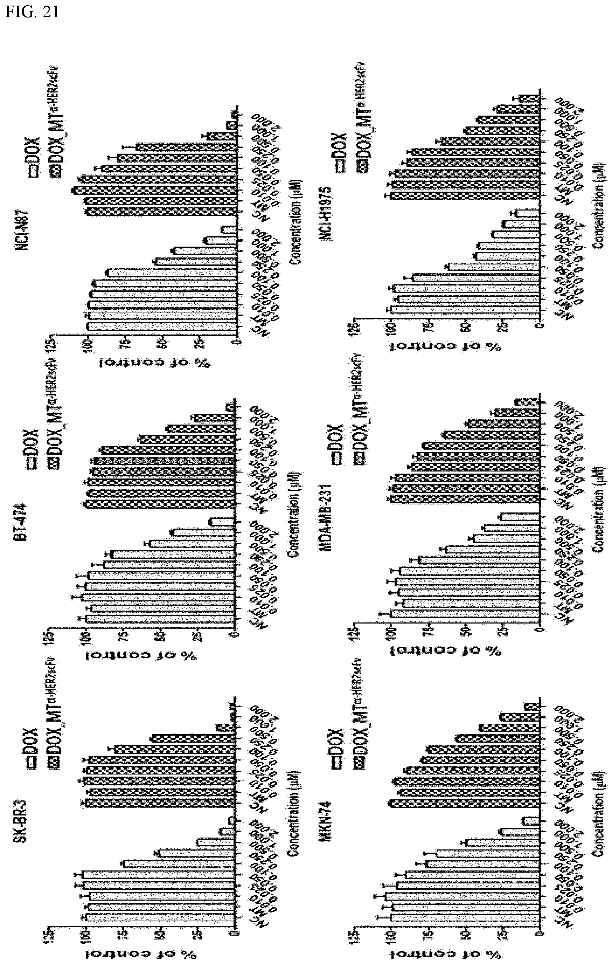

As a result, as shown in FIG. 21, for the untreated group (NC), cell proliferation inhibitory effect was not observed in the mitochondria-only treated group (MT). On the other hand, in DOX treated group and DOX_MT^{α-HER2scFv} treated group, cancer cell proliferation was inhibited in a concentration-dependent manner. In particular, it was confirmed that when treated with 1 µM DOX_MT^{α-HER2scFv}, cell growth was inhibited by about 60% to 90% compared to the untreated group depending on the cancer cell line (FIG. 21).

### Example 4.5. Confirmation of proliferation inhibitory ability by treatment with DOX_MT^{α-}^{HER2scFv} for each concentration in normal cells

200 µg of MT^{α-HER2scFv} and 100 µM doxorubicin (DOX) were reacted in the same manner as in Preparation Example 2 above. In order to confirm the influence of the obtained DOX_MT^{α-HER2scFv} on cell proliferation in normal cells, the cell proliferation inhibitory effect by treatment with DOX_MT^{α-HER2scFv} in human lung fibroblasts was evaluated.

Specifically, the human lung fibroblast cell lines WI-38 and CCD-8Lu were inoculated into a 96-well plate at about 3×10³ cells/well, respectively, and cultured for 24 hours. At this time, WI-38 and CCD-8Lu were cultured in DMEM medium containing 10% FBS. After culturing for 24 hours, each cell was treated with doxorubicin (DOX) or DOX_MT^{α-HER2scFv} at a concentration of 2 µM, 1 µM, 0.5 µM, 0.25 µM, 0.1 µM, 0.05 µM, 0.025 µM, and 0.01 µM. After 144 hours of sample treatment, cell proliferation was measured by WST-1 assay.

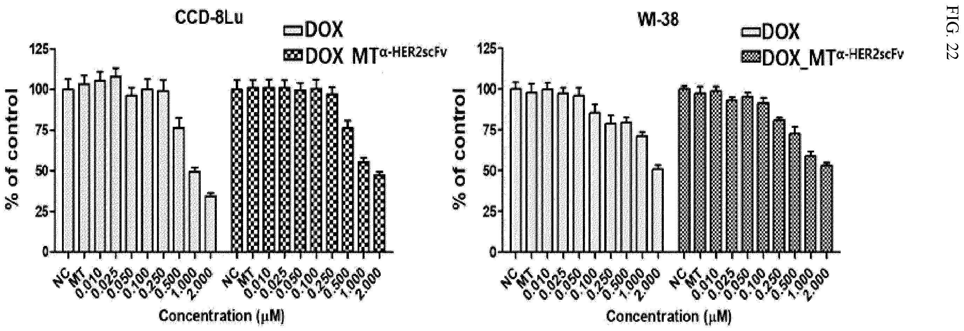

As a result, as shown in FIG. 22, for the untreated group (NC), cell proliferation inhibitory effect was not observed in the mitochondria-only treated group (MT). In addition, it was confirmed that in DOX treated group and DOX_MT^{α-HER2scFv} treated group, cell proliferation inhibitory ability was weaker than that of cancer cells.

### Example 4.6. Analysis of IC₅₀ value by treatment with DOX_MT^{α-HER2scFv} in cancer cells and normal cells

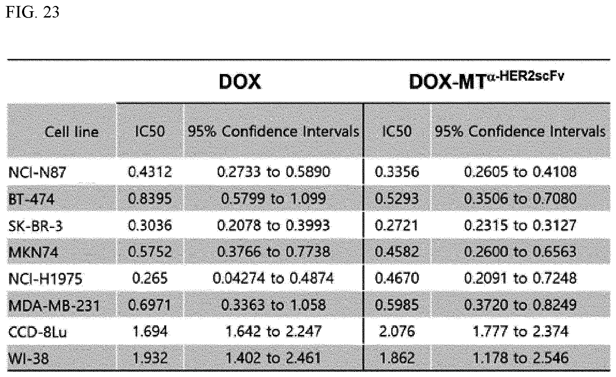

Based on the cell proliferation inhibition results of DOX_MT^{α-HER2scFv} for each concentration in Example 4.4 and Example 4.5, IC₅₀ values in each cell were analyzed and shown in FIG. 23.

### Example 4.7. Comparison of proliferation inhibitory ability by treatment with DOX_MT^{α-}^{HER2scFv} in cancer cells and normal cells

200 µg of MT^{α-HER2scFv} and 100 µM doxorubicin (DOX) were reacted in the same manner as in Preparation Example 2 above. In order to compare the influence of the obtained DOX_MT^{α-HER2scFv} on cell proliferation in cancer cells and normal cells, after treatment with DOX_MT^{α-HER2scFv} at the same concentration in the human breast cancer, lung cancer, gastric cancer cell lines and the lung fibroblast cell line, the cell proliferation inhibitory ability was measured.

Specifically, the human breast cancer (SK-BR-3, BT-474 and MDA-MB-231), lung cancer (NCI-H1975), gastric cancer (MKN-74 and NCI-N87) cell lines and the lung fibroblast cell line (CCD-8Lu and WI-38) were inoculated into a 96-well plate at about 3×10³ cells/well, respectively, and cultured for 24 hours. At this time, SK-BR-3, BT-474 and NCI-N87 cells were cultured in RPMI-1640 medium containing 10% FBS. MDA-MB-231, NCI-H1975, MKN-74, CCD-8Lu, and WI-38 cells were cultured in DMEM medium containing 10% FBS. After culturing for 24 hours, the cells were treated with 0.5 µM doxorubicin (DOX) or DOX_MT^{α-HER2scFv}, respectively, and after 144 hours, the cell proliferation inhibitory ability was evaluated by WST-1 assay. At this time, the mitochondria-only treated group (MT^{α-HER2scFv}) was used as a negative control for DOX_MT^{α-HER2scFv}. Statistical processing was performed using Graphpad 5.0 software, and p values were measured with one-way ANOVA (Turkey's) test (*p<0.05, **p<0.01, and ***p<0.001).

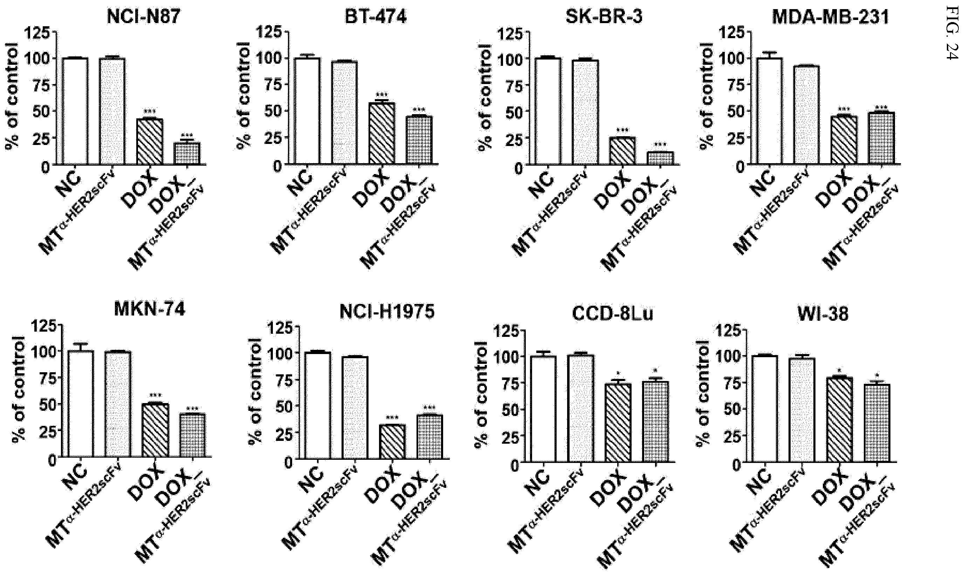

As a result, as shown in FIG. 24, for the untreated group (NC) in cancer cells and normal cells, cell proliferation inhibitory effect was not observed in the mitochondria-only treated group (MT^{α-HER2scFv}). In the case of DOX treated group and DOX_MT^{α-HER2scFv} treated group, cell proliferation inhibitory effect was shown by about 50% to 80% or higher compared to the untreated group in cancer cells, whereas cell proliferation inhibitory effect was shown by about 25% or lower in normal cells.

### Example 4.8. Confirmation of cancer cell apoptosis by treatment with DOX_MT^{α-HER2scFv} by TUNEL assay

In order to confirm the apoptosis inducing activity of DOX_MT^{α-HER2scFv} obtained in the same manner as in Preparation Example 2 above in cancer cells, after treatment with DOX_MT^{α-HER2scFv} in the human breast cancer and gastric cancer cell lines, TUNEL assay was performed.

Specifically, the human breast cancer cell line (SK-BR-3 and BT-474) and the human gastric cancer cell line (NCI-N87) were inoculated into a 24-well plate at about 3×10⁴ cells/well and cultured for 24 hours. After culturing for 24 hours, the cells were treated with MT^{α-HER2scFv} or 1 µM DOX_MT^{α-HER2scFv}, and after 48 hours, TUNEL assay was performed to confirm the apoptosis. At this time, the mitochondria-only treated group (MT^{α-HER2scFV}) was used as a control group for DOX_MT^{α-HER2scFv}. The nuclei of the cells were observed by staining with DAPI.

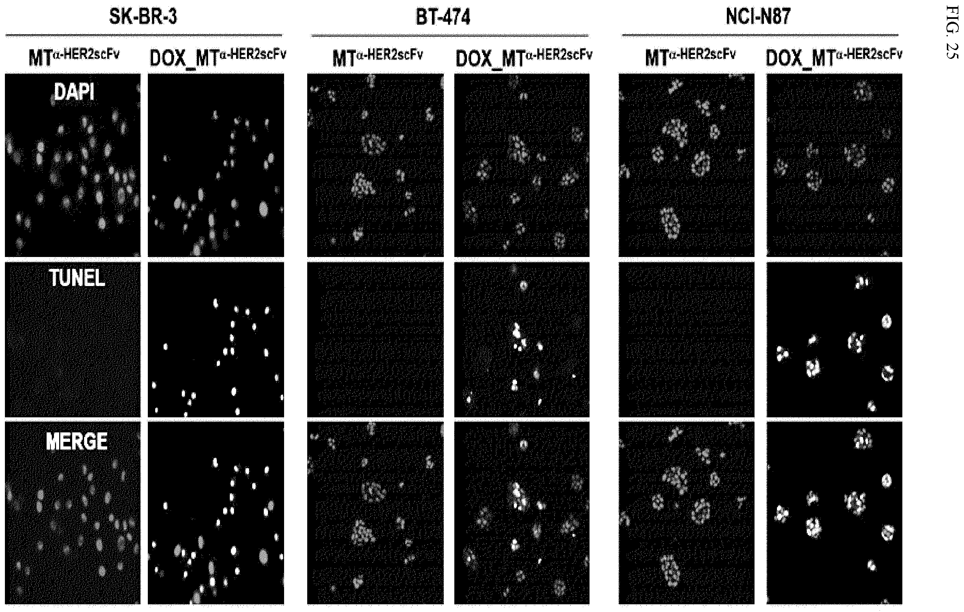

As a result, almost no apoptosis (green fluorescence) was observed in the mitochondria-only treated group (MT^{α-HER2scFv}). However, an increase in apoptosis was observed in the cell nucleus of DOX_MT^{α-HER2scFv} treated group. From the above results, it was confirmed that the apoptosis was induced by treatment with DOX_MT^{α-HER2scFv} (FIG. 25).

### Example 4.9. Confirmation of cancer cell apoptosis by treatment with DOX_MT^{α-HER2scFv} through Western blot analysis

In order to confirm the apoptosis effect of DOX_MT^{αHER2scFv} obtained in the same manner as in Preparation Example 2 above in cancer cells, after treatment with DOX_MT^{α-HER2scFv} in the human breast cancer cell line, Western blot was performed using an antibody against an apoptosis marker.

Specifically, the human breast cancer cell lines (SK-BR-3 and BT-474) were inoculated into a 6-well plate at about 5×10⁶ cells/well, respectively, and cultured for 24 hours. After culturing for 24 hours, the cells were treated with MT^{α-HER2scFv} or DOXMT^{α-HER2scFv}, respectively, and after 96 hours, the cells were lyzed, proteins were extracted, and Western blot was performed. Anti-cleaved PARP antibody (Cell signaling, 9541S), anti-cleaved caspase 3 antibody (Cell signaling, 9664S), anti-phospho-p53 antibody (ABclonal, AP0762) and anti-β actin antibody (Sigma, A2228) were used as primary antibodies. Anti-mouse IgG HRP or anti-rabbit IgG HRP was used as a secondary antibody. The amount of protein was corrected through the expression level of β actin protein. At this time, the mitochondria-only treated group (MT^{α-HER2scFv}) was used as a negative control for DOX_MT^{α-HER2scFv}.

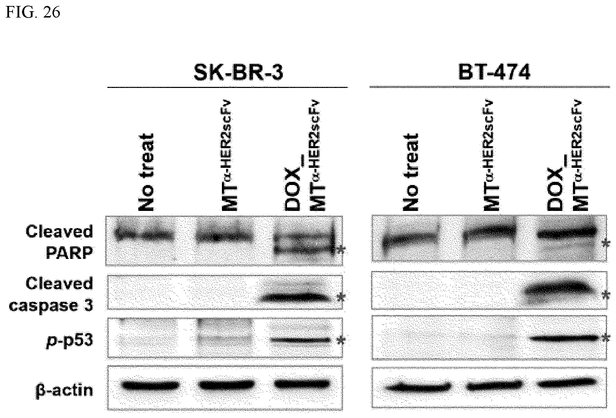

As a result, in the mitochondria-only treated group (MT^{α-HER2scFv}), similar to the untreated group (No treat), the cleaved forms of PARP and caspase 3 observed during the apoptosis were not observed, and the phosphorylated form of p53 was not also observed. On the other hand, in DOX_MT^{α-HER2scFv} treated group, the expression of the phosphorylated p53 and the cleaved forms of PARP and caspase 3 was observed (FIG. 26). From the above results, it was confirmed that the apoptosis was induced by treatment with DOX_MT^{α-HER2scFv}.

### Example 4.10. Confirmation of cancer cell apoptosis by DOX_MT^{α-HER2scFv} through flow cytometry

In order to confirm the apoptosis effect by DOX_MT^{α-HER2scFv} obtained in the same manner as in Preparation Example 2 above in cancer cells, after treatment with DOX_MT^{αHER2scFv} in the human breast cancer and gastric cancer cell lines, flow cytometry was performed by staining with Annexin V/PI.

Specifically, the human breast cancer cell line (SK-BR-3 and BT-474) and the human gastric cancer cell line (NCI-N87) were inoculated into a 6-well plate at about 5×10⁶ cells/well, respectively, and cultured for 24 hours. After culturing for 24 hours, the cells were treated with MT^{α-HER2scFv} or 1 µM DOX_MT^{α-HER2scFv}. After 96 hours of treatment, the cells were washed twice with PBS and obtained by treatment with 0.05% Trypsin-EDTA. The obtained cells were centrifuged and then washed with PBS to remove 0.05% Trypsin-EDTA. Thereafter, the cells were stained for 10 minutes using Annexin V/PI staining kit (Roche, 11988549001), and the stained cells were analyzed using FACS equipment (Beckman Coulter, CytoFLEX). At this time, the mitochondria-only treated group (MT^{α-HER2scFv}) was used as a negative control for DOX_MT^{α-}HER2scFv

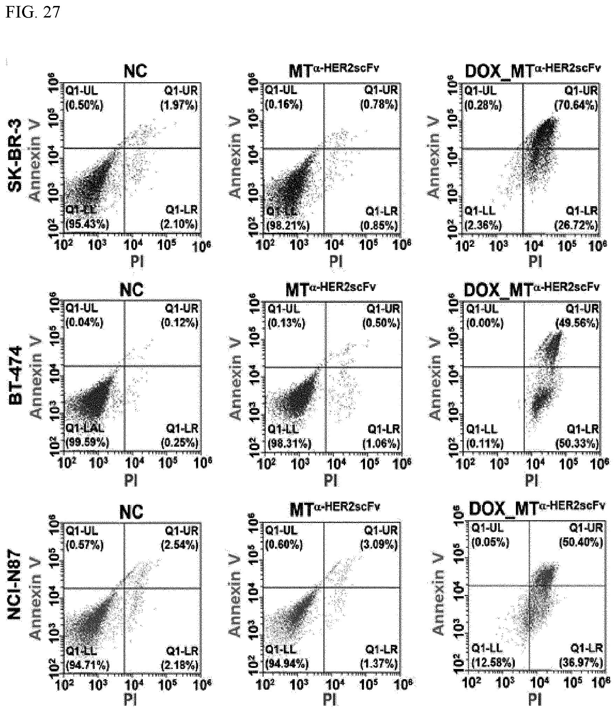

As a result, for the untreated group (NC), no change in Annexin V/PI values was observed in the mitochondria-only treated group (MT^{α-HER2scFv}). On the other hand, the Annexin V/PI value was increased in DOX_MT^{α-HER2scFv} treated group (FIG. 27). From the above results, it was confirmed that the apoptosis was induced by treatment with DOX_MT^{α-HER2scFv}.

### Example 4.11. Evaluation of HER2 expressing cell selective apoptosis inducing activity of DOX_MT^{α-HER2scFv}

In order to confirm the HER2 selective apoptosis inducing effect of DOX MT^{α-HER2scFv} obtained in the same manner as in Preparation Example 2 above, the co-cultured HER2⁺ cancer cells and HER2⁻ normal cells were treated with DOX_MT^{α-HER2scFv}, and then TUNEL assay and immunocytochemistry staining were performed.

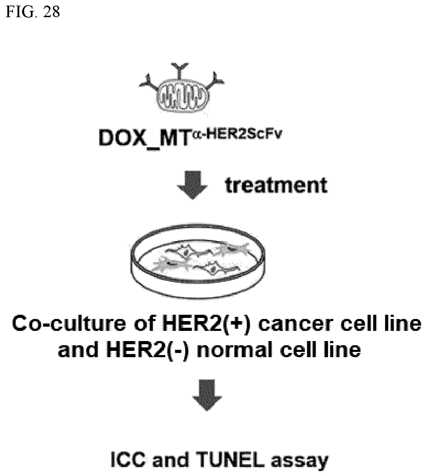

Specifically, the human breast cancer cell line BT-474 (HER2⁺) and the human lung fibroblast cell line WI-38 (HER2) were inoculated together into a 24-well plate at 3×10⁴ cells/well and co-cultured in DMEM medium containing 10% FBS for 24 hours. At this time, BT-474 cells were prepared by culturing in RPMI-1640 medium containing 10% FBS, and WI-38 cells were prepared by culturing in DMEM medium containing 10% FBS. After culturing for 24 hours, the co-cultured cells were treated with 1 µM DOX_MT^{α-HER2scFv} and cultured for additional 48 hours. The apoptosis was evaluated by TUNEL assay and immunocytochemistry staining (FIG. 28).

### Example 4.12. Confirmation of HER2 expressing cell selective apoptosis inducing activity of DOX_MT^{α-HER2scFv} by TUNEL assay

The cells treated in the same manner as in Example 4.11 above were washed twice with PBS and then were fixed by adding 3.7% formaldehyde at room temperature for 30 minutes. Thereafter, anti-HER2 antibody (Cell Signaling Technology, 2165) was used as a primary antibody, and anti-rabbit IgG antibody labeled with Alexa Fluor^{®}555 (Invitrogen, 21428) was used as a secondary antibody, and HER2⁺ cells were labeled with red fluorescence. Thereafter, the cells were stained with TUNEL solution for 30 minutes, and the cells in which the apoptosis occurs were labeled with green fluorescence and mounted, and the cells were observed by a confocal microscope. At this time, the nuclei of the cells were observed by staining with DAPI.

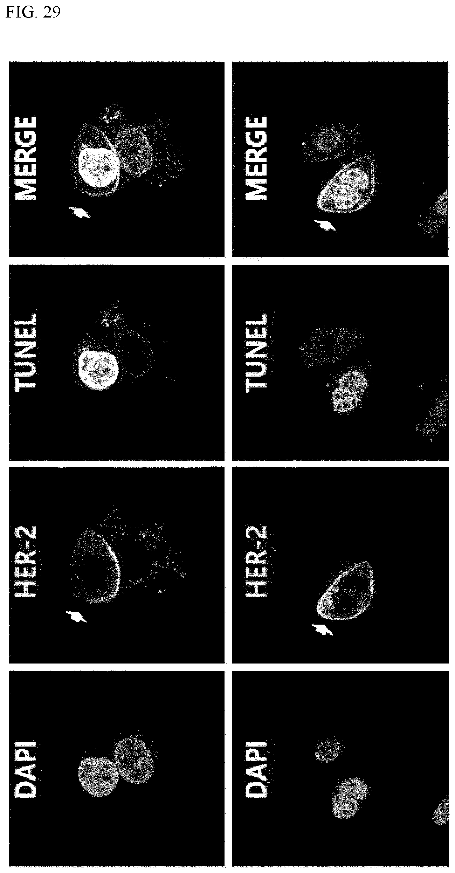

As a result, strong green fluorescence was selectively observed only in BT-474 cells (white arrow), which are HER2⁺ cancer cells (FIG. 29). From the above results, it was confirmed that the apoptosis by DOX_MT^{α-HER2scFv} could be selectively induced depending on the presence or absence of HER2 expression.

### Preparation Example 3. Preparation of compound to which triphenylphosphonium-doxorubicin is bound

In order to efficiently deliver the anticancer drug doxorubicin (DOX) into the isolated mitochondria, a compound capable of targeting mitochondria was fused with doxorubicin. The most representative mitochondria targeting compound is triphenylphosphonium (TPP), and the anticancer agent to which TPP is bound was reacted as shown in FIG. 30 using a method known in "Mitochondrial Delivery of Doxorubicin via Triphenylphosphine Modification for Overcoming Drug Resistance in MDA-MB-435/DOX Cells" (Molecular Pharmaceutics, 11 (8), 2640-2649) to prepare TPP-doxorubicin (TPP-DOX), and the compound was confirmed to be TPP-doxorubicin by MRI analysis of the compound (FIG. 31).

### Preparation Example 4. Preparation of complex of mitochondria-TPP and doxorubicin

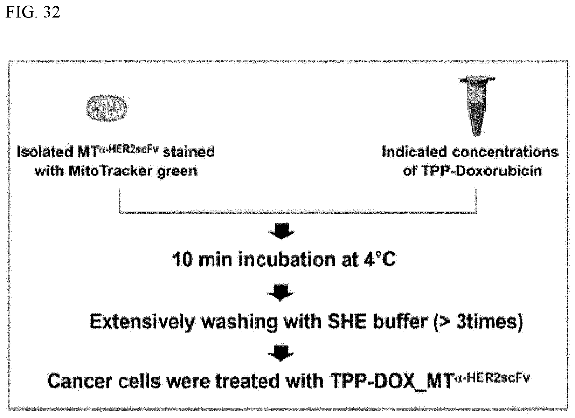

HEK293 cells in which an anti-HER2scFv antibody is expressed were treated with MitoTracker green to stain the mitochondria, and then the mitochondria MT^{α-HER2scFv}) were isolated. Thereafter, 30 µg of the mitochondria fused with an anti-HER2scFv antibody (MT^{α-HER2scFv}) were reacted with 100 µM TPP-doxorubicin at 4°C for 10 minutes, and then centrifuged at about 12,000 xg for 10 minutes to remove unreacted TPP-doxorubicin. The reaction products were washed twice with 500 µL of SHE (250 mM Sucrose, 20 mM HEPES (pH 7.4), 2 mM EGTA) buffer to finally obtain the mitochondria comprising TPP-doxorubicin (TPP-DOX_MT^{α-HER2scFv}) (FIG. 32).

### Example 5. Evaluation of anticancer activity of TPP-DOX_MT^{α-HER2scFv} against cancer cells

### Example 5.1. Confirmation of delivery property of TPP-DOX_MT^{α-HER2scFv} into cancer cells

The human breast cancer cell line (SK-BR-3, BT-474 and MDA-MB-231) and the human lung cancer cell line (NCI-H1975) were treated with TPP-DOX MT^{α-HER2scFv} obtained in the same manner as in Preparation Example 4 above, respectively, and then the cells were observed by a confocal microscope. At this time, the nuclei of the cells were observed by staining with DAPI.

As a result, the mitochondria stained with MitoTracker green (MT^{α-HER2scFv}) and doxorubicin showing red fluorescence (TPP-DOX) were observed at the same location in the cytoplasm (FIG. 33). From the above results, it was confirmed that both TPP-DOX and MT^{α-HER2scFv} transported by TPP-DOX MT^{α-HER2scFv} were located in the cytoplasm.

### Example 5.2. Confirmation of morphological change in cancer cells by treatment with TPP-DOX MT^{α-HER2scFv}

The human breast cancer cell line (SK-BR-3 and BT-474) and the human gastric cancer cell line (NCI-N87) were treated with TPP-DOX MT^{α-HER2scFv} obtained in the same manner as in Preparation Example 4 above, respectively, and then the morphological change in cancer cells over time was observed by a confocal microscope. At this time, the nuclei of the cells were observed by staining with DAPI.

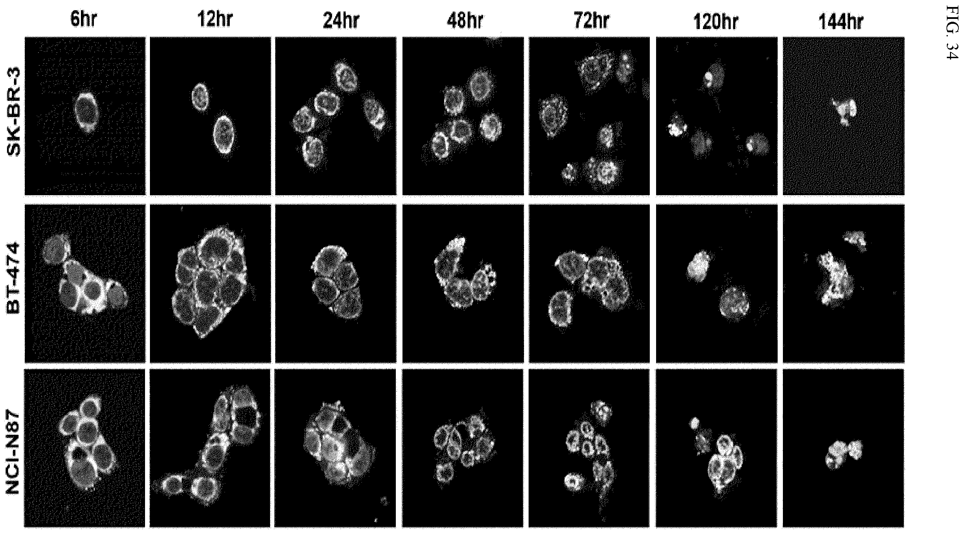

As a result, it was observed that most of TPP-DOX (red fluorescence) exists together in the mitochondria (green fluorescence) after 12 hours of treatment with TPP-DOX_MT^{α-HER2scFv} (yellow fluorescence). However, TPP-DOX was observed in the nucleus over time, and the morphological change in the cells was remarkably observed after 72 hours of treatment with TPP-DOX_MT^{α-HER2scFv} (FIG. 34).

### Example 5.3. Confirmation of cancer cell proliferation inhibitory ability by treatment with TPP-DOX_MT^{α-HER2scFv} for each concentration

In order to confirm the anticancer activity of TPP-DOX_MT^{αHER2scFv} obtained in the same manner as in Preparation Example 4 above, the cell proliferation inhibitory effect by treating the human breast cancer, lung cancer and gastric cancer cell lines with TPP-DOX_MT^{α-HER2scFv} was evaluated.

Specifically, the human breast cancer (SK-BR-3, BT-474 and MDA-MB-231), lung cancer (NCI-H1975) and gastric cancer (NCI-N87 and MKN-74) cell lines were inoculated into a 96-well plate at about 3×10³ cells/well, respectively, and cultured for 24 hours. At this time, the human breast cancer cell line (SK-BR-3 and BT-474) and the human gastric cancer cell line (NCI-N87) were cultured in RPMI-1640 medium containing 10% FBS, and the human breast cancer cell line MDA-MB-231, the human lung cancer cell line NCI-H1975 and the human gastric cancer cell line MKN-74 were cultured in DMEM medium containing 10% FBS. After culturing for 24 hours, each cell was treated with TPP-DOX or TPP-DOX MT^{α-HER2scFv} at a concentration of 2 µM, 1 µM, 0.5 µM, 0.25 µM, 0.1 µM, 0.05 µM, 0.025 µM and 0.01 µM. After 144 hours of sample treatment, cell proliferation was measured by WST-1 assay. At this time, the mitochondria-only treated group (MT, MT^{α-HER2scFv}) was used as a negative control for TPP-DOX_MT^{α-HER2scFv}.

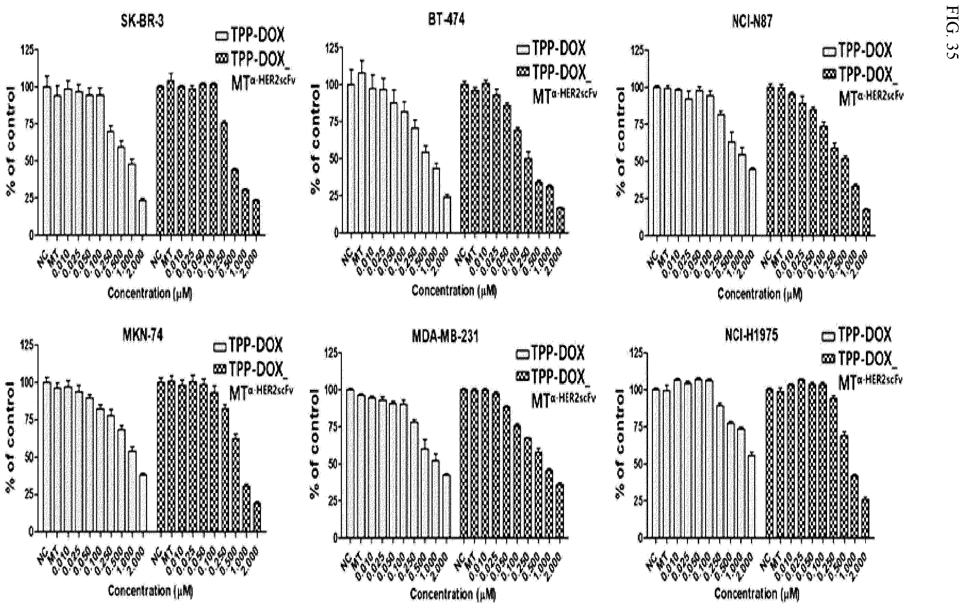

As a result, as shown in FIG. 35, for the untreated group (NC), cell proliferation inhibitory effect was not observed in the mitochondria-only treated group (MT). On the other hand, in TPP-DOX treated group and TPP-DOX MT^{α-HER2scFv} treated group, cancer cell proliferation was inhibited in a concentration-dependent manner. In particular, it was confirmed that when treated with 1 µM TPP-DOX MT^{α-HER2scFv}, cell growth was inhibited by about 60% to 70% compared to the untreated group depending on the cancer cell line.

### Example 5.4. Confirmation of proliferation inhibitory ability by treatment with TPP-DOX_MT^{α-HER2scFv} for each concentration in normal cells

In order to confirm the influence of TPP-DOX MT^{α-HER2scFv} obtained in the same manner as in Preparation Example 4 above on cell proliferation in normal cells, the cell proliferation inhibitory effect after treating the human lung fibroblasts with TPP-DOX MT^{α-HER2scFv} was evaluated.

Specifically, the human lung fibroblast cell lines WI-38 and CCD-8Lu were inoculated into a 96-well plate at about 3×10³ cells/well, respectively, and cultured for 24 hours. At this time, WI-38 and CCD-8Lu were cultured in DMEM medium containing 10% FBS. After culturing for 24 hours, each cell was treated with TPP-DOX or TPP-DOX MT^{α-HER2scFv} at a concentration of 2 µM, 1 µM, 0.5 µM, 0.25 µM, 0.1 µM, 0.05 µM, 0.025 µM and 0.01 µM. After 144 hours of sample treatment, cell proliferation was measured by WST-1 assay. At this time, the mitochondria-only treated group (MT, MT^{α-HER2scFv}) was used as a negative control for TPP-DOX_MT^{α-HER2scFv}.

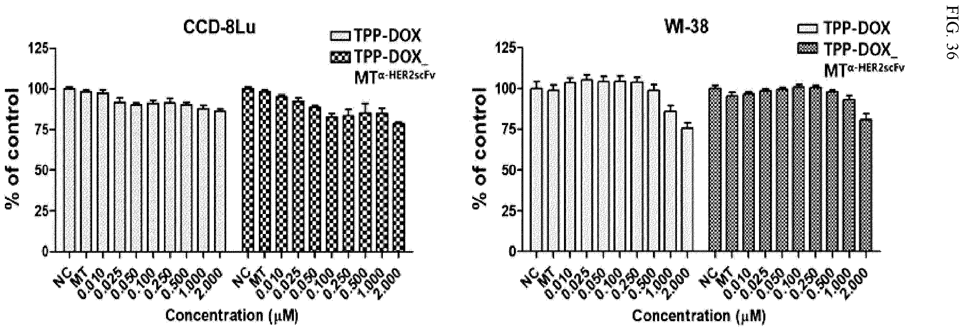

As a result, as shown in FIG. 36, for the untreated group (NC), cell proliferation inhibitory effect was not observed in the mitochondria-only treated group (MT). In addition, it was confirmed that in TPP-DOX treated group and TPP-DOX MT^{α-HER2scFv} treated group, cell proliferation inhibitory ability was weaker than that of cancer cells.

### Example 5.5. Analysis of IC₅₀ value by treatment with TPP-DOX_MT^{α-HER2scFv} in cancer cells and normal cells

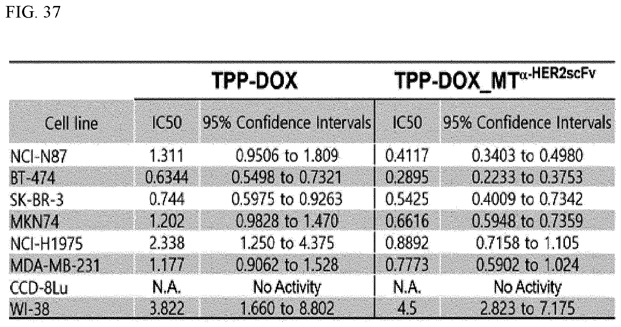

Based on the cell proliferation inhibition results of TPP-DOX_MT^{α-HER2scFv} for each concentration in Example 5.3 and Example 5.4, IC₅₀ values in each cell were analyzed and shown in FIG. 37.

### Example 5.6. Comparison of proliferation inhibitory ability by treatment with TPP-DOX_MT^{α-}^{HER2scFv} in cancer cells and normal cells

In order to compare the influence of TPP-DOX_MT^{αHER2scFv} obtained in the same manner as in Preparation Example 4 above on cell proliferation in cancer cells and normal cells, after treatment with TPP-DOX_MT^{α-HER2scFv} at the same concentration in the human breast cancer, lung cancer, gastric cancer cell lines and the lung fibroblast cell line, the cell proliferation inhibitory ability was measured.

Specifically, the human breast cancer (SK-BR-3, BT-474 and MDA-MB-231), lung cancer (NCI-H1975), gastric cancer (MKN-74 and NCI-N87) cell lines and the lung fibroblast cell line (CCD-8Lu and WI-38) were inoculated into a 96-well plate at about 3×10³ cells/well, respectively, and cultured for 24 hours. At this time, SK-BR-3, BT-474 and NCI-N87 cells were cultured in RPMI-1640 medium containing 10% FBS, and MDA-MB-231, NCI-H1975, MKN-74, CCD-8Lu and WI-38 cells were cultured in DMEM medium containing 10% FBS. After culturing for 24 hours, the cells were treated with 0.5 µM TPP-DOX or TPP-DOX_MT^{α-HER2scFv}, respectively, and after 144 hours, the cell proliferation inhibitory ability was evaluated by WST-1 assay. At this time, the mitochondria-only treated group (MT^{α-HER2scFv}) was used as a negative control for TPP-DOX_MT^{α-HER2scFv}. Statistical processing was performed using Graphpad 5.0 software, and p values were measured with one-way ANOVA (Turkey's) test (*p<0.05, **p<0.01 and ***p<0.001).

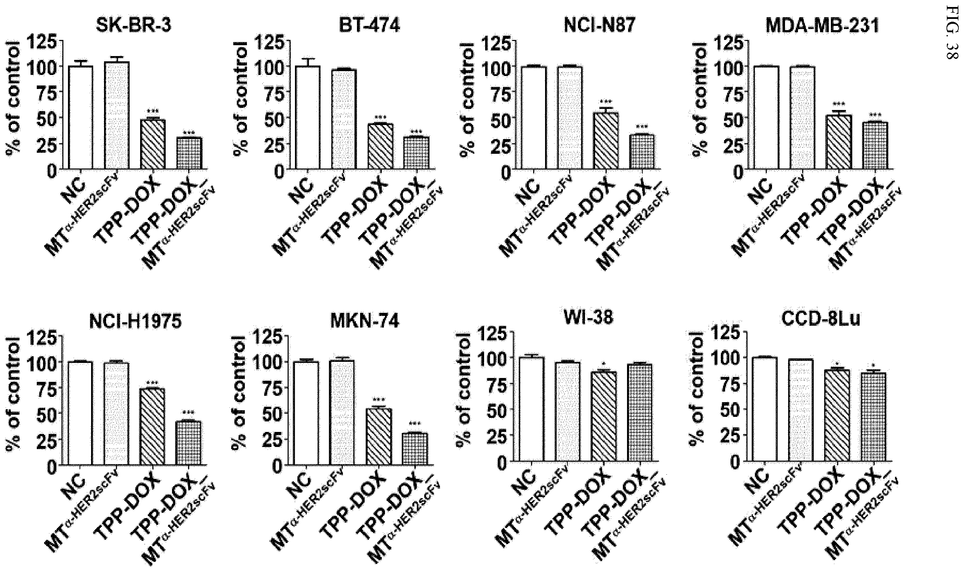

As a result, as shown in FIG. 38, for the untreated group (NC) in cancer cells and normal cells, cell proliferation inhibitory effect was not observed in the mitochondria-only treated group (MT^{α-HER2scFv}). In the case of TPP-DOX-only treated group and TPP-DOX_MT^{α-HER2scFv} treated group, cell proliferation inhibitory effect was shown by about 60% to 75% or higher compared to the untreated group in cancer cells. However, it was confirmed that proliferation inhibitory ability was weak in normal cells unlike in cancer cells.

### Example 5.7. Confirmation of cancer cell apoptosis by TPP-DOX_MT^{α-HER2scFv} by TUNEL assay

In order to confirm the apoptosis inducing activity of TPP-DOX_MT^{αHER2scFv} obtained in the same manner as in Preparation Example 4 above in cancer cells, after treatment with TPP-DOX_MT^{α-HER2scFv} in the human breast cancer and gastric cancer cell lines, TUNEL assay was performed.

Specifically, the human breast cancer cell line (SK-BR-3 and BT-474) and the human gastric cancer cell line (NCI-N87) were inoculated into a 24-well plate at about 3×10⁴ cells/well, respectively, and cultured for 24 hours. After culturing for 24 hours, the cells were treated with MT^{α-HER2scFv} or 1 µM TPP-DOX MT^{α-HER2scFv}, and after 48 hours, TUNEL assay was performed to confirm the apoptosis. At this time, the mitochondria-only treated group (MT^{α-HER2scFV}) was used as a control group for TPP-DOX_MT^{α-HER2scFv}. The nuclei of the cells were observed by staining with DAPI.

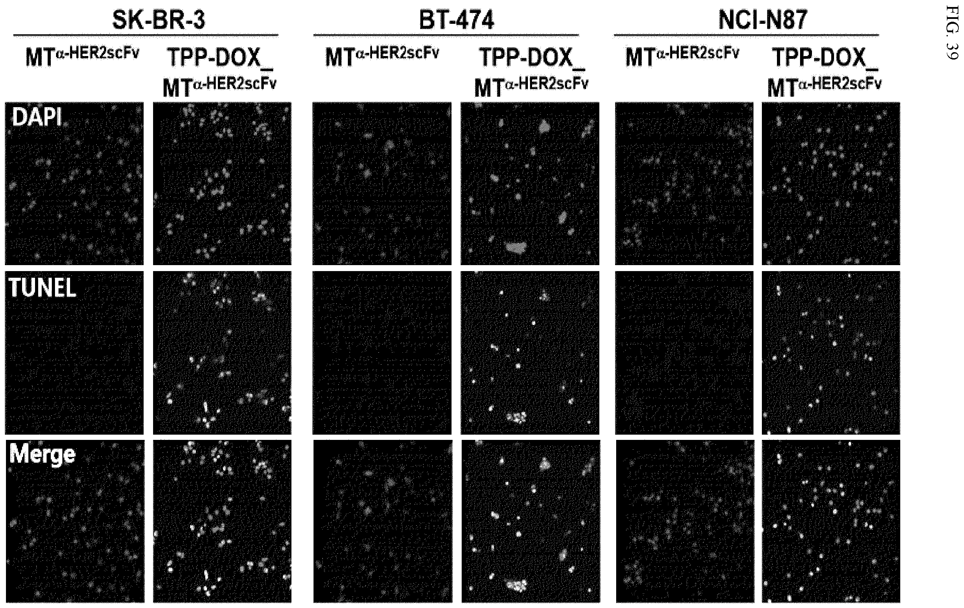

As a result, almost no apoptosis (green fluorescence) was observed in the mitochondria-only treated group (MT^{α-HER2scFv}). However, an increase in apoptosis was observed in the cell nucleus of TPP-DOX MT^{α-HER2scFv} treated group. From the above results, it was confirmed that the apoptosis was induced by treatment with TPP-DOX_MT^{α-HER2scFv} (FIG. 39).

### Example 5.8. Confirmation of cancer cell apoptosis by treatment with TPP-DOX_MT^{α-}^{HER2scFv} through Western blot analysis

In order to confirm the apoptosis effect by treatment with TPP-DOX MT^{α-HER2scFv} obtained in the same manner as in Preparation Example 4 above in cancer cells, after treatment with TPP-DOX_MT^{α-HER2scFv} in the human breast cancer and gastric cancer cell lines, Western blot was performed using an antibody against a apoptosis marker.

Specifically, the human breast cancer cell line (SK-BR-3 and BT-474) and the human gastric cancer cell line (NCI-N87) were inoculated into a 6-well plate at about 5×10⁶ cells/well, respectively, and cultured for 24 hours. After culturing for 24 hours, the cells were treated with MT^{α-HER2scFv} or 1 µM TPP-DOX_MT^{α-HER2scFv}, respectively, and after 96 hours, the cells were disrupted, proteins were extracted, and Western blot was performed. Anti-cleaved PARP antibody (Cell signaling, 9541S), anti-cleaved caspase 3 antibody (Cell signaling, 9664S), anti-phospho-p53 antibody (ABclonal, AP0762) and anti-β actin antibody (Sigma, A2228) were used as primary antibodies. Anti-mouse IgG HRP or anti-rabbit IgG HRP was used as a secondary antibody. The amount of protein was corrected through the expression level of β actin protein. At this time, the mitochondria-only treated group (MT^{α-HER2scFV}) was used as a control group for TPP-DOX_MT^{α-} HER2scFv

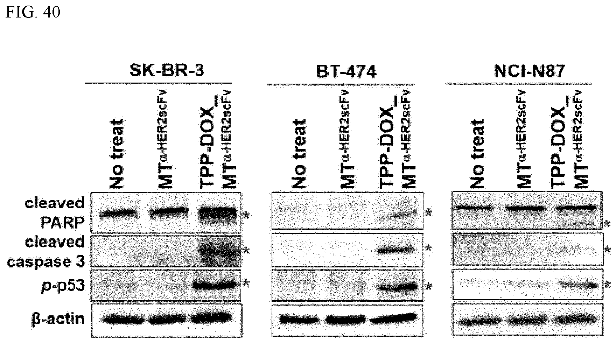

As a result, in the mitochondria-only treated group (MT^{α-HER2scFv}), similar to the untreated group (No treat), the cleaved forms of PARP and caspase 3 observed during the apoptosis were not observed, and the phosphorylated form of p53 was not also observed. On the other hand, in TPP-DOX MT^{α-HER2scFv} treated group, the expression of the phosphorylated p53 and the cleaved forms of PARP and caspase 3 was observed (FIG. 40). From the above results, it was confirmed that the apoptosis was induced by treatment with TPP-DOX MT^{α-HER2scFv}.

### Example 5.9. Confirmation of cancer cell apoptosis by treatment with TPP-DOX_MT^{α-HER2scFv} through flow cytometry

In order to confirm the apoptosis effect by treatment with TPP-DOX_MT^{α-HER2scFv} obtained in the same manner as in Preparation Example 4 above in cancer cells, after treatment with TPP-DOX_MT^{α-HER2scFv} in the human breast cancer and gastric cancer cell lines, flow cytometry was performed by staining with Annexin V/PI.

Specifically, the human breast cancer cell line (SK-BR-3 and BT-474) and the human gastric cancer cell line (NCI-N87) were inoculated into a 6-well plate at about 5×10⁶ cells/well, respectively, and cultured for 24 hours. After culturing for 24 hours, the cells were treated with MT^{α-HER2scFv} or 1 µM TPP-DOX_MT^{α-HER2scFv}. After 96 hours, the cells were washed twice with PBS and obtained by treatment with 0.05% Trypsin-EDTA. The obtained cells were centrifuged and then washed with PBS to remove 0.05% Trypsin-EDTA. Thereafter, the cells were stained for 10 minutes using Annexin V/PI staining kit (Roche, 11988549001), and the stained cells were analyzed using FACS equipment (Beckman Coulter, CytoFLEX). At this time, the mitochondria-only treated group (MT^{α-HER2scFv}) was used as a negative control for TPP-DOX_MT^{α-HER2scFv}.

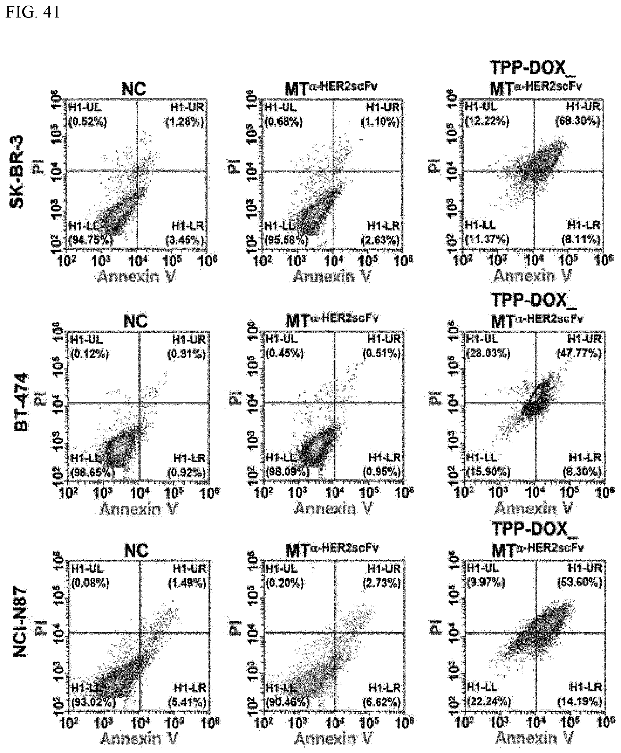

As a result, for the untreated group (NC), no change in Annexin V/PI values was observed in the mitochondria-only treated group (MT^{α-HER2scFv}). On the other hand, the Annexin V/PI value was increased in TPP-DOX_MT^{α-HER2scFv} treated group (FIG. 41). From the above results, it was confirmed that the apoptosis was induced by treatment with TPP-DOX_MT^{α-HER2scFv}.

### Example 5.10. Evaluation of HER2 expressing cell selective apoptosis inducing activity of TPP-DOX_MT^{α-HER2scFv}

In order to confirm the HER2 selective apoptosis inducing effect of TPP-DOX_MT^{α-HER2scFv} obtained in the same manner as in Preparation Example 4 above, the co-cultured HER2⁺ cancer cells and HER2⁻ normal cells were treated with TPP-DOX MT^{α-HER2scFv}, and then TUNEL assay and immunocytochemistry staining were performed.

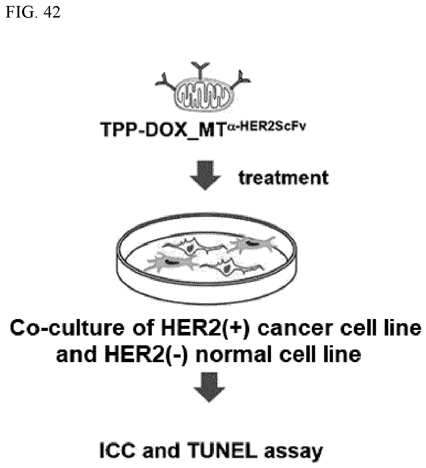

Specifically, the human breast cancer cell line SK-BR-3 (HER2⁺) and the human lung fibroblast cell line CCD-8Lu (HER2) were inoculated together into a 24-well plate at 3×10⁴ cells/well and co-cultured in DMEM medium containing 10% FBS for 24 hours. At this time, SK-BR-3 cells were prepared by culturing in RPMI-1640 medium containing 10% FBS, and WI-38 cells were prepared by culturing in DMEM medium containing 10% FBS. After culturing for 24 hours, the co-cultured cells were treated with 1 µM TPP-DOX MT^{α-HER2scFv} and cultured for additional 48 hours. The apoptosis was evaluated by TUNEL assay and immunocytochemistry staining (FIG. 42).

### Example 5.11. Confirmation of HER2 expressing cell selective apoptosis inducing activity of TPP-DOX_MT^{α-HER2scFv} by TUNEL assay

The cells treated in the same manner as in Example 5.10 above were washed twice with PBS and then fixed by adding 3.7% formaldehyde at room temperature for 30 minutes. Thereafter, anti-HER2 antibody (Cell signaling, 29D8) was used as a primary antibody, and Alexa Fluor^{®}555 (Invitrogen, 21428) was used as a secondary antibody, and HER2⁺ cells were labeled with red fluorescence. Thereafter, the cells were stained with TUNEL solution for 30 minutes, and the cells in which the apoptosis occurs were labeled with green fluorescence and mounted, and the cells were observed by a confocal microscope. At this time, the nuclei of the cells were observed by staining with DAPI.

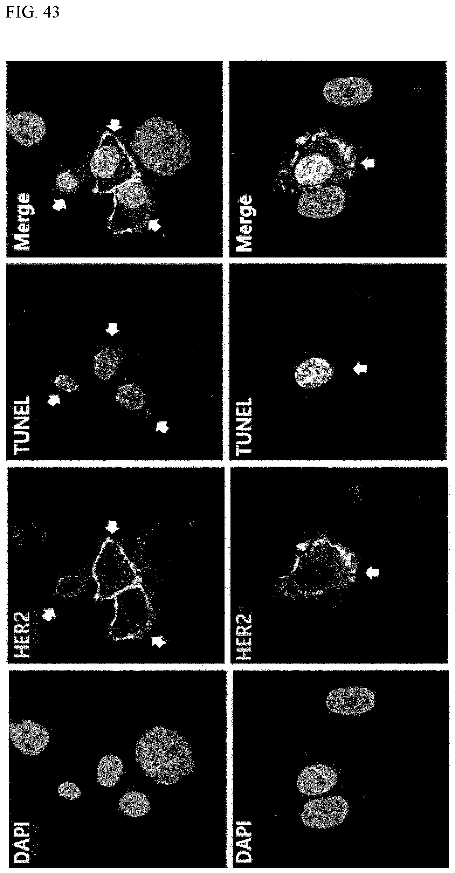

As a result, strong green fluorescence was selectively observed only in SK-BR-3 cells (white arrow), which are HER2⁺ cells (FIG. 43). From the above results, it was confirmed that the apoptosis by TPP-DOX MT^{α-HER2scFv} could be selectively induced depending on the presence or absence of HER2 expression.

### Preparation Example 5. Preparation of HEK293 cell line comprising mitochondria fused with anti-PDL1scFv antibody

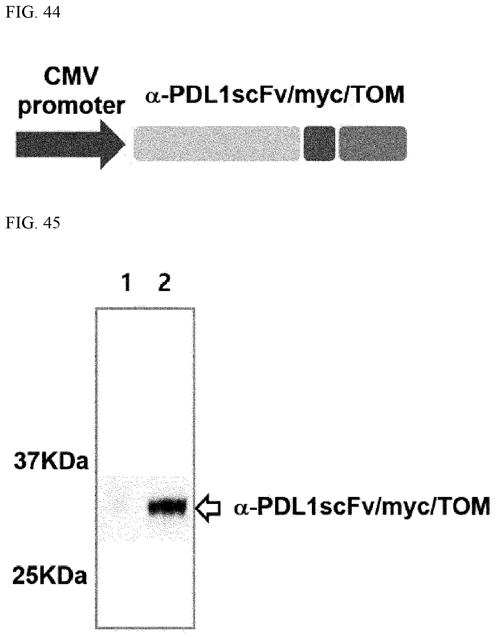

In order to specifically introduce mitochondria into tumor cells in which PDL1 is expressed, mitochondria in which an anti-PDL1scFv antibody (SEQ ID NO: 94) that specifically binds to PDL1 is fused to the mitochondrial outer membrane were construct (FIGS. 44 and 45). A specific constructing method was performed according to the method described in Korean Patent Application Publication No. 10-2019-0124656.

### Example 6. Analysis of PDL1 expression in human cancer cell line through Western blot analysis

Western blot was performed to confirm the expression level of PDL1 in the human pancreatic cancer cell line (BXPC-3 and CFPAC-1) and the human gastric cancer cell line (SNU-216 and SNU-484).

Specifically, the human pancreatic cancer cell line (BXPC-3 and CFPAC-1) and the human gastric cancer cell line (SNU-216 and SNU-484) were inoculated into a 6-well plate at about 1×10⁶ cells/well and then cultured in DMEM medium containing 10% FBS for 24 hours. After 24 hours, the culture solution was removed, and the cells were washed twice with PBS, and then 100 µL of RIPA buffer containing a protease inhibitor was added directly to the cells. After 10 minutes, the cells were recovered using a scraper, transferred to a microtube, and then centrifuged at about 12,000 xg for 10 minutes. The separated supernatant was obtained and transferred to a new microtube, and then protein was quantified using the BCA analysis. The same amount of protein for each sample was electrophoresed, and then Western blot was performed. Anti-PDL1 antibody (Cell signaling, 13684) and anti-β tubulin antibody (Thermo Fisher, MA5-16308) were used as primary antibodies, and anti-mouse IgG HRP antibody or anti-rabbit IgG HRP antibody were used as a secondary antibody.

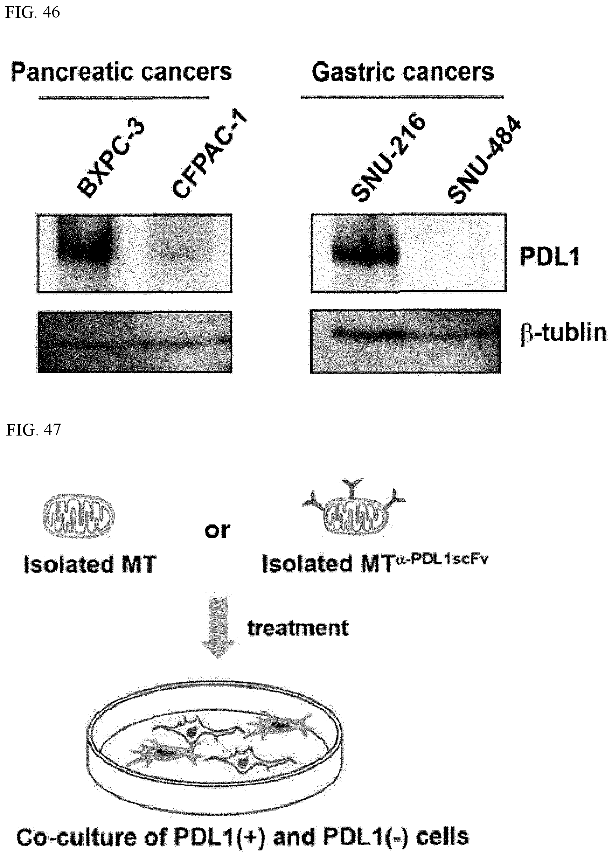

As a result, as shown in FIG. 46, it was confirmed that BXPC-3 and SNU-216 cells were PDL1⁺ cells expressing PDL1. On the other hand, it was confirmed that CFPAC-1 and SNU-484 cells showed almost no PDL1 expression, indicating that they were PDL1⁻ cells.

### Example 7. Verification of PDL1 targeting of mitochondria fused with anti-PDL1scFv antibody

In order to confirm the PDL1 targeting ability of the mitochondria fused with the anti-PDL1scFv antibody constructed in Preparation Example 5 above, the mitochondria fused with the anti-PDL1scFv antibody (MT^{α-PDL1scFv}) were treated with the co-cultured PDL1⁺ cells and PDL1⁻ cells, and then immunocytochemistry staining was performed.

Specifically, BXPC-3 (PDL1⁺) and CFPAC-1 (PDL1⁻), or SNU-216 (PDL1⁺) and SNU-484 (PDL1⁻) were inoculated together into a 24-well plate at 1.5×10⁴ cells/well and co-cultured in DMEM medium containing 10% FBS for 24 hours. Mitochondria to be used as samples were prepared by treating untreated HEK293 cells (MT) and HEK293 cells comprising mitochondria fused with an anti-PDL1scFv antibody (MT^{α-PDL1scFv}) with MitoTracker Red to stain the mitochondria, and then isolating the mitochondria. After co-culture for 24 hours, each isolated mitochondrion was treated with the co-cultured cells and reacted for 24 hours (FIG. 47).

### Example 7.1. Confirmation of location of mitochondria fused with anti-PDL1scFv antibody in PDL1 positive and negative cell lines

The cells treated in the same manner as in Example 4 above were washed once with PBS, and fixed by adding 3.7% formaldehyde at room temperature for 30 minutes, and then immunocytochemistry staining was performed. At this time, an anti-PDL1 antibody (Cell signaling, 86744) was used as a primary antibody, and Alexa Fluor 488 goat anti-rabbit IgG (H+L) was used as a secondary antibody. The nuclei of the cells were observed by staining with DAPI.

As a result, as shown in FIG. 48, when the co-cultured BXPC-3 (PDL1⁺) and CFPAC-1 (PDL1⁻) cells were treated with untreated HEK293 cell-derived mitochondria (MT), the mitochondria stained with Mitotracker Red (red fluorescence) were observed equally in BXPC-3 (PDL1⁺) and CFPAC-1 (PDL1⁻) cells. On the other hand, when the cells were treated with the mitochondria (MT^{α-PDL1scFv}) derived from HEK293 cells comprising the mitochondria fused with the anti-PDL1scFv antibody, the mitochondria stained with Mitotracker Red were observed specifically in PDL1⁺ cells, BXPC-3 cells.

In addition, equally, in the case of the co-cultured SNU-216 (PDL1⁺) and SNU-484 (PDL1⁻) cells, in the case of MT treated group, the mitochondria stained with Mitotracker Red were observed equally in PDL1⁺ (SNU-216) and SNU-484 (PDL1⁻) cells, whereas in the case of MT^{α-PDL1scFv} treated group, they were specifically observed only in PDL1⁺ cells, SNU-216 cells. From the above results, it was found that the mitochondria fused with the anti-PDL1scFv antibody specifically targeted the cells expressing a PDL1 antigen.

### Preparation Example 6. Preparation of complex of mitochondria fused with anti-PDL1scFv antibody and doxorubicin

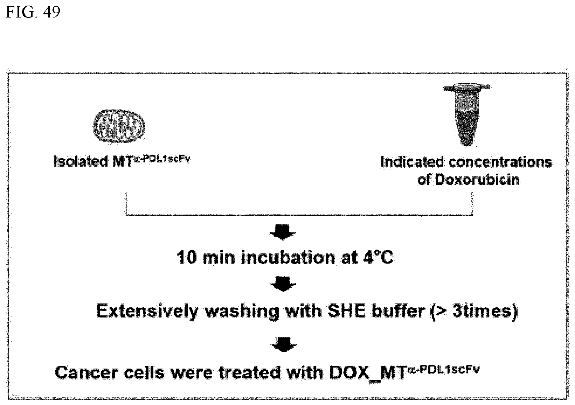

30 µg of mitochondria (MT^{α-PDL1scFv}) isolated from the HEK293 cells comprising mitochondria fused with an anti-PDL1scFv antibody were reacted with 100 µM doxorubicin (DOX) at 4°C for 10 minutes, and then centrifuged at about 12,000 xg for 10 minutes to remove unreacted doxorubicin. Thereafter, they were washed twice with 500 µL of SHE (250 mM Sucrose, 20 mM HEPES (pH 7.4), 2 mM EGTA) buffer to finally obtain the mitochondria comprising doxorubicin (DOX MT^{α-PDL1scFv}) (FIG. 49).

### Example 8. Evaluation of anticancer activity of DOX_MT^{α-PDL1scFv} against cancer cells

### Example 8.1. Confirmation of cell proliferation inhibitory ability by treatment with DOX_MT^{α-}PDL1scFv for each concentration

In order to confirm the anticancer activity of DOX_MT^{α-PDL1scFv} obtained in the same manner as in Preparation Example 6 above, after treating the human pancreatic cancer and gastric cancer cell lines with DOX_MT^{α-PDL1scFv}, the cell proliferation inhibitory effect was evaluated.

Specifically, the human pancreatic cancer cell line (BXPC-3 and CFPAC-1) and the human gastric cancer cell line (SNU-216 and SNU-484) were inoculated into a 96-well plate at about 5×10³ cells/well and cultured for 24 hours. After culturing for 24 hours, each cell was treated with DOX_MT^{α-PDL1scFv} at a concentration of 0.01 µM, 0.025 µM, 0.05 µM, 0.1 µM, 0.25 µM, 0.5 µM and 1 µM. Cell proliferation was measured by WST-1 assay every 24 hours until 168 hours after sample treatment. At this time, the mitochondria-only treated group (MT^{α-PDL1scFv}) was used as a negative control for DOX_MT^{α-PDL1scFv}.

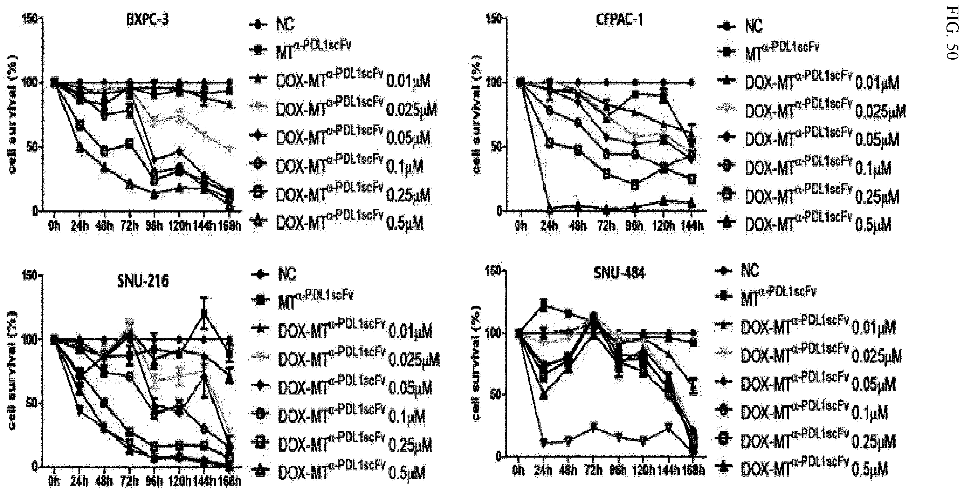

As a result, for the untreated group (NC), cell proliferation inhibitory effect was not observed in the mitochondria-only treated group (MT^{α-PDL1scFv}). On the other hand, in DOX_MT^{α-PDL1scFv} treated group, cancer cell proliferation was inhibited over time in a concentration-dependent manner (FIG. 50).

### Example 8.2. Confirmation of cancer cell apoptosis by DOX_MT^{α-}^{PDL1scFv} through Western blot analysis

In order to confirm the apoptosis effect by DOX_MT^{α-PDL1scFv} in cancer cells, after treatment with DOX_MT^{α-PDL1scFv} in the human pancreatic cancer and gastric cancer cell lines, Western blot was performed using an antibody against an apoptosis marker.

Specifically, the human pancreatic cancer cell line (BXPC-3 and CFPAC-1) and the human gastric cancer cell line (SNU-216 and SNU-484) were inoculated at about 5×10⁶ cells/well, respectively, and cultured for 24 hours.

After culturing for 24 hours, the cells were treated with MT^{α-PDL1scFv} or DOX_MT^{α-PDL1scFv}, respectively, and after 96 hours, the cells were disrupted, proteins were extracted, and Western blot was performed. Anti-cleaved PARP antibody (Cell signaling, 9541S), anti-cleaved caspase 3 antibody (Cell signaling, 9664), anti-phospho-p53 antibody (ABclonal, AP0762) and anti-β actin antibody (Sigma, A2228) were used as primary antibodies, and anti-mouse IgG HRP or anti-rabbit IgG HRP was used as a secondary antibody. The amount of protein was corrected through the expression level of β actin protein. At this time, the mitochondria-only treated group (MT^{α-PDL1scFv}) was used as a negative control for DOX_MT^{α-PDL1scFv.}

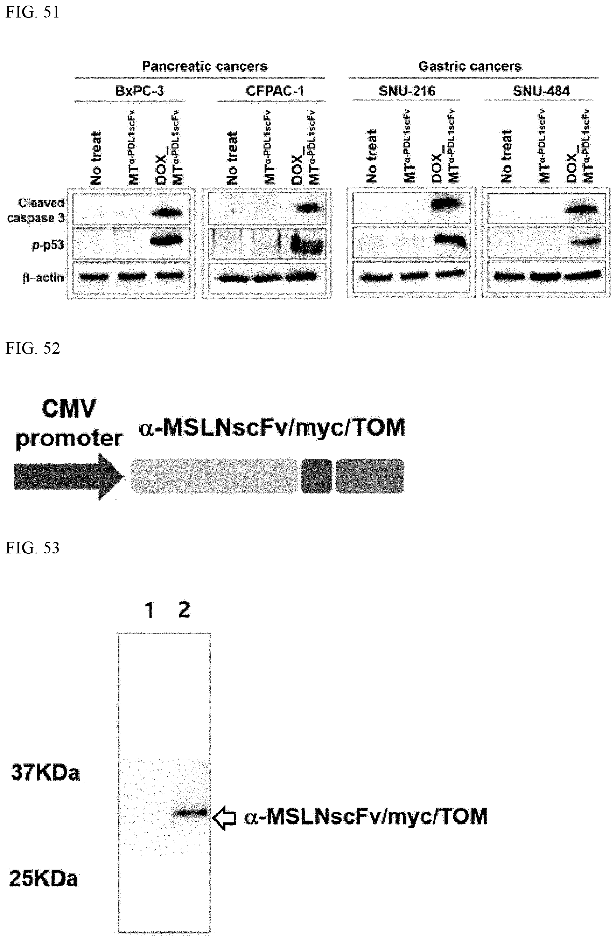

As a result, in the mitochondria-only treated group (MT^{α-PDL1scFv}), similar to the untreated group (No treat), the cleaved forms of PARP and caspase 3 observed during the apoptosis were not observed, and the phosphorylated form of p53 was not also observed. On the other hand, in DOX_MT^{α-PDL1scFv} treated group, the expression of the phosphorylated p53 and the cleaved forms of PARP and caspase 3 was observed (FIG. 51). From the above results, it was confirmed that the apoptosis was induced by treatment with DOX_MT^{α-PDL1scFv}.

### Preparation Example 7. Preparation of HEK293 cell line comprising mitochondria fused with anti-MSLN scFv antibody

In order to specifically introduce mitochondria into tumor cells in which mesothelin (MSLN) is expressed, mitochondria in which an anti-MSLNscFv antibody (SEQ ID NO: 102) that specifically binds to MSLN is fused to the mitochondrial outer membrane were construct (FIGS. 52 and 53). A specific method was performed according to the method described in Korean Patent Application Publication No. 10-2019-0124656.

### Preparation Example 8. Preparation of complex of mitochondria fused with anti-MSLNscFv antibody and pheophorbide A

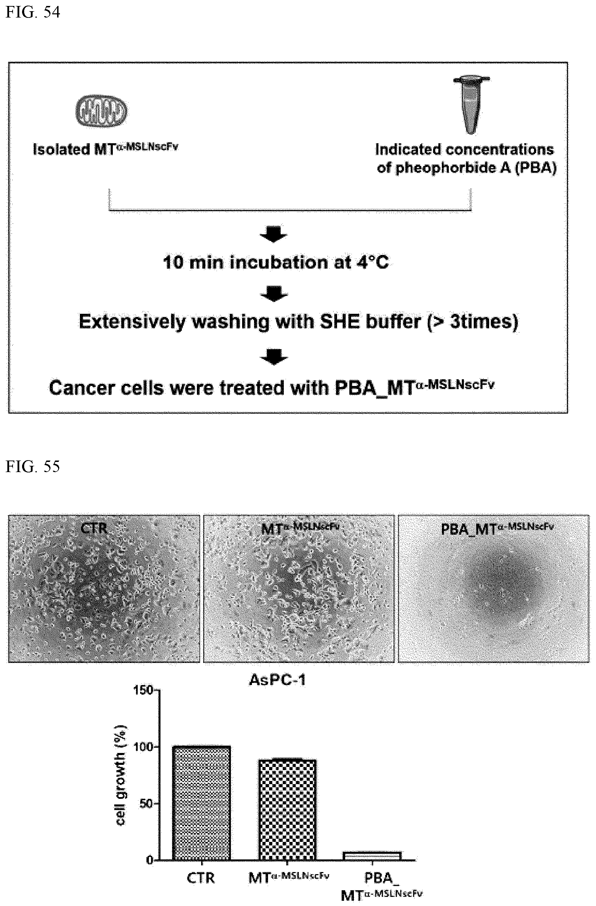

100 µg of mitochondria (MT^{α-MSLNscFv}) isolated from the HEK293 cells comprising mitochondria fused with an anti-MSLNscFv antibody were reacted with 100 µM pheophorbide A (PBA) at 4°C for 10 minutes, and then centrifuged at about 12,000 xg for 10 minutes to remove unreacted pheophorbide A. Thereafter, they were washed twice with 500 µL of SHE (250 mM Sucrose, 20 mM HEPES (pH 7.4), 2 mM EGTA) buffer to finally obtain the mitochondria comprising pheophorbide A (PBA_MT^{α-MSLNscFv}) (FIG. 54).

### Example 9. Confirmation of cell proliferation inhibitory ability by treatment with PBA_MT^{α-MSLNscFv}

In order to confirm the anticancer activity of PBA MT^{α-MSLNscFv} obtained in the same manner as in Preparation Example 8 above, after treating the human pancreatic cancer cell line with PBA_MT^{α-MSLNscFv}, the cell proliferation inhibitory effect was evaluated.

Specifically, the human pancreatic cancer cell lines AsPC-1, Capan-1, Capan-2 and Mia PaCa-2 were inoculated into a 96-well plate at 5×10³ cells/well and cultured for 24 hours. Thereafter, the cells were treated with MT^{α-MSLNscFv} or 1 µg of PBA-MT^{α-MSLNscFv} and cultured for additional 72 hours, and then cell proliferation was evaluated by WST-1 assay. At this time, the mitochondria-only treated group (MT^{α-MSLNscFv}) was used as a negative control for PBA_MT^{α-}MSLNscFv

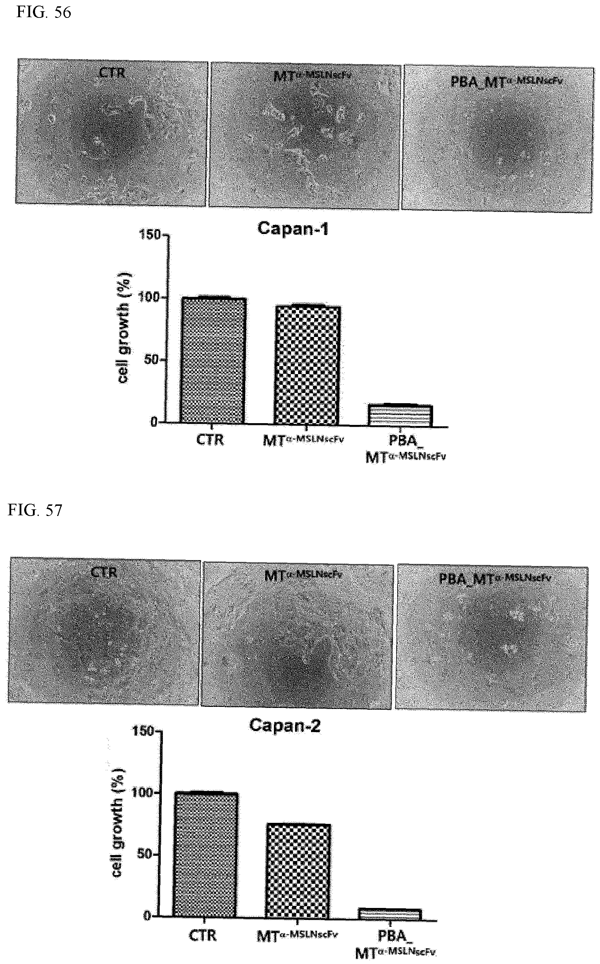

As a result, for the untreated group (CTR), cell proliferation inhibitory effect was not observed in the mitochondria-only treated group (MT^{α-MSLNscFv}). On the other hand, it was confirmed that in PBA_MT^{α-MSLNscFv} treated group, cell growth was inhibited by about 80% to 95% compared to the untreated group (bottom of FIGS. 55 to 58).

In addition, the morphological change according to treatment with MT^{α-MSLNscFv} and PBA_MT^{α-MSLNscFv} in each pancreatic cancer cell line was observed by an inverted microscope. In MT^{α-MSLNscFv} treated group, almost no morphological change was observed compared to the untreated group, whereas in PBA_MT^{α-MSLNscFv} treated group, it was confirmed that the cell morphology was altered in all cancer cell lines (top of FIGS. 55 to 58).

### Preparation Example 9. Preparation of complex of mitochondria fused with anti-MSLNscFv antibody and gemcitabine

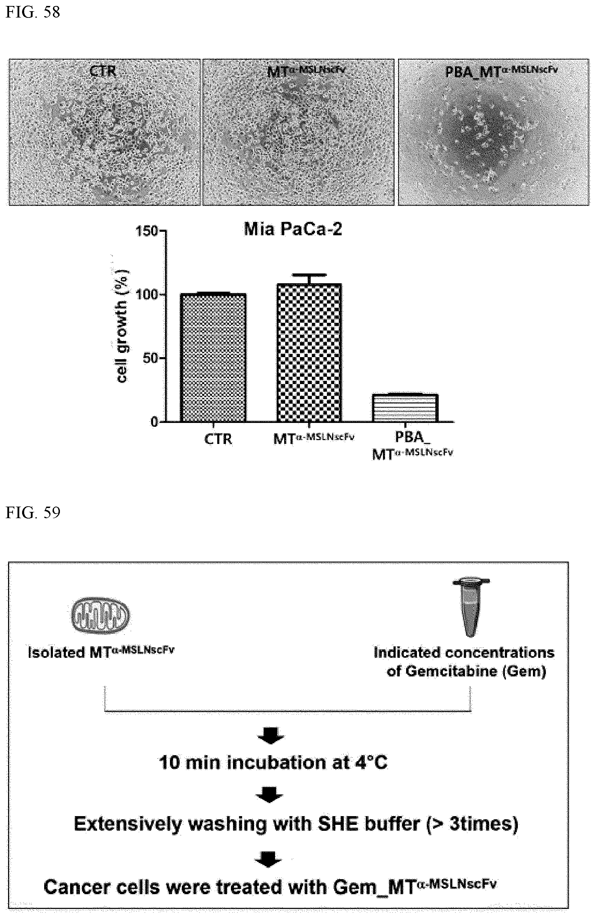

100 µg of mitochondria isolated from the HEK293 cells comprising mitochondria fused with an anti-MSLNscFv antibody were reacted with 100 µM gemcitabine (Gem) at 4°C for 10 minutes, and then centrifuged at about 12,000 xg for 10 minutes to remove unreacted gemcitabine. Thereafter, they were washed twice with 500 µL of SHE (250 mM Sucrose, 20 mM HEPES (pH 7.4), 2 mM EGTA) buffer to finally obtain the mitochondria comprising gemcitabine (Gem_MT^{α-MSLNscFv}) (FIG. 59).

### Example 10. Confirmation of cell proliferation inhibitory ability by treatment with Gem_MT^{α-MSLNscFv}

In order to confirm the anticancer activity of Gem_MT^{α-MSLNscFv} obtained in the same manner as in Preparation Example 9 above, after treating the human pancreatic cancer cell line with Gem_MT^{α-MSLNscFv}, the cell proliferation inhibitory effect was evaluated.

Specifically, the human pancreatic cancer cell lines AsPC-1, Capan-1, Capan-2 and Mia PaCa-2 were inoculated into a 96-well plate at about 5×10³ cells/well, respectively, and cultured for 24 hours. After culturing for 24 hours, each cell was treated with MT^{α-MSLNscFv} or 2 µg of Gem_MT^{α-MSLNscFv} and cultured for additional 72 hours, and then cell proliferation was measured by WST-1 assay. At this time, the mitochondria-only treated group (MT^{α-MSLNscFv}) was used as a negative control for Gem_MT^{α-MSLNscFv}.

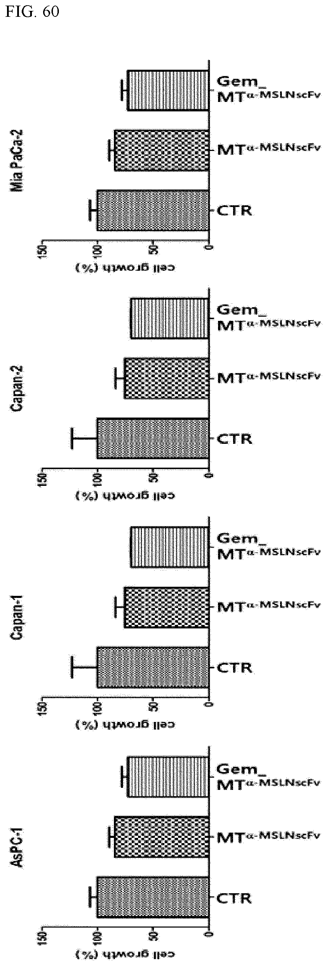

As a result, for the untreated group (CTR), cell proliferation inhibitory effect was not observed in the mitochondria-only treated group (MT^{α-MSLNscFv}). On the other hand, it was confirmed that Gem_MT^{α-MSLNscFv} treated group showed cell growth inhibitory effect by about 20% to 30% compared to the untreated group depending on the cancer cell line (FIG. 60).

### Preparation Example 10. Preparation of complex of mitochondria fused with anti-MSLNscFv antibody and vinorelbine

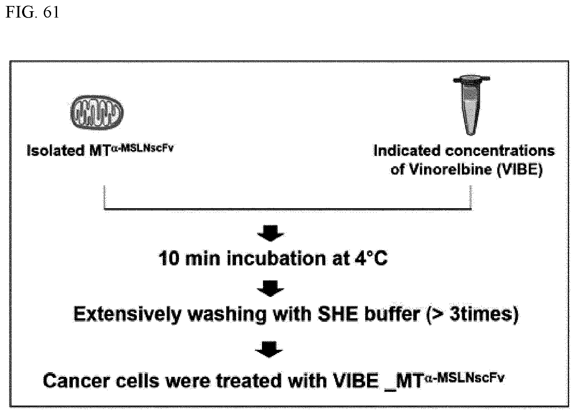

100 µg of mitochondria (MT^{α-MSLNscFv}) isolated from the HEK293 cells comprising mitochondria fused with an anti-MSLNscFv antibody were reacted with 100 µM vinorelbine (VIBE) at 4°C for 10 minutes, and then centrifuged at about 12,000 xg for 10 minutes to remove unreacted vinorelbine. Thereafter, they were washed twice with 500 µL of SHE (250 mM Sucrose, 20 mM HEPES (pH 7.4), 2 mM EGTA) buffer to finally obtain the mitochondria comprising vinorelbine (VIBE_MT^{α-MSLNscFv}) (FIG. 61).

### Example 11. Confirmation of cell proliferation inhibitory ability by treatment with VIBE_MT^{α-MSLNscFv}

In order to confirm the anticancer activity of VIBE_MT^{α-MSLNscFv} obtained in the same manner as in Preparation Example 10 above, after treating the human pancreatic cancer cell line with VIBE_MT^{α-MSLNscFv}, the cell proliferation inhibitory effect was evaluated.

Specifically, the human pancreatic cancer cell lines AsPC-1, Capan-1, Capan-2 and MIA PaCa-2 were inoculated into a 96-well plate at about 5×10³ cells/well, respectively, and cultured for 24 hours. After culturing for 24 hours, each cell was treated with MT^{α-MSLNscFv} or 2 µg of VIBE_MT^{α-MSLNscFv} and cultured for additional 72 hours, and then cell proliferation was measured by WST-1 assay. At this time, the mitochondria-only treated group (MT^{α-MSLNscFv}) was used as a negative control for VIBE MT^{α-MSLNscFv}.

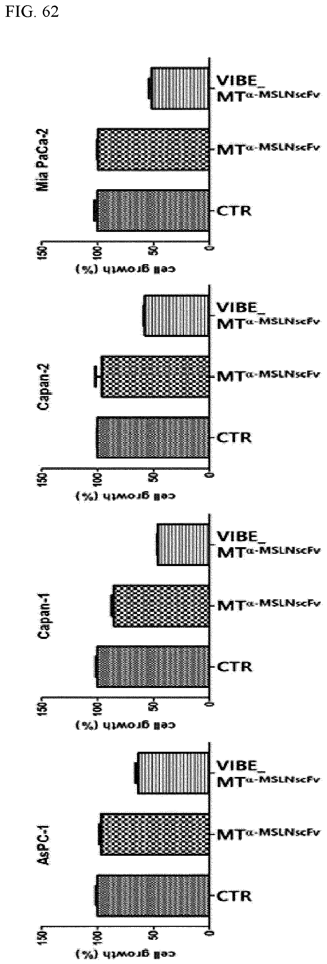

As a result, for the untreated group (CTR), cell proliferation inhibitory effect was not observed in the mitochondria-only treated group (MT^{α-MSLNscFv}). On the other hand, it was confirmed that VIBE_MT^{α-MSLNscFv} treated group showed cell growth inhibitory effect by about 50% compared to the untreated group depending on the cancer cell line (FIG. 62).

### Example 12. Verification of tumor cell targeting of MT^{α-}^{HER2scFv} in xenograft tumor mouse model

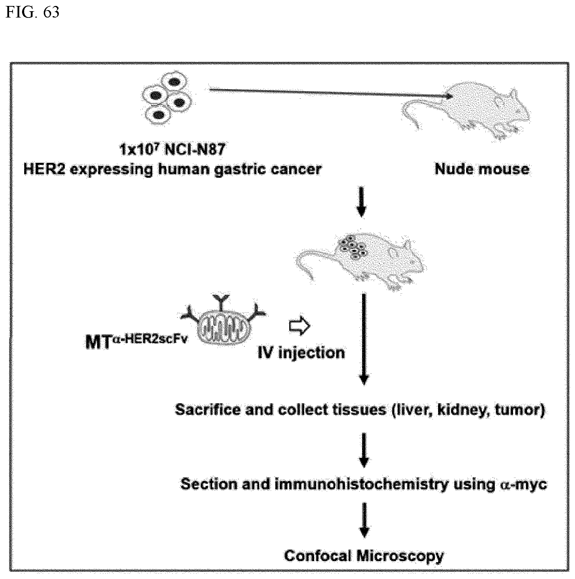

5-week-old BALB/c male nude mice (initial body weight of 18 - 20 g) (Nara Biotech) were acclimatized for 1 week, and then HER2⁺ human gastric cancer cell line NCI-N87 was subcutaneously transplanted into the lateral abdomen at 1×10⁷ cells/mouse to construct a xenograft tumor model mouse. When the average volume of the tumor reached 50 mm³ or more, 20 µg of MT^{α-HER2scFv} was intravenously administered. After 24 hours, the tissues of the mice were extracted. Each extracted tissue was analyzed by immunohistochemistry staining using an anti-myc antibody to confirm the distribution of MT^{α-HER2scFv} in mouse tissues (FIG. 63).

### Example 12.1. Confirmation of tumor cell targeting of MT^{α-}^{HER2scFv} in xenograft tumor mouse model

Immunohistochemistry staining was performed on the tissues of the mice treated in the same manner as in Example 12 above. An anti-myc antibody (Roche, 11667149001) labeled with mitochondria MT^{α-HER2scFv} was used as a primary antibody, and an anti-mouse IgG antibody labeled with Alexa Fluor^{®} 488 (Invitrogen, 11001) was used as secondary antibody. At this time, the nuclei of the cells were observed by staining with DAPI.

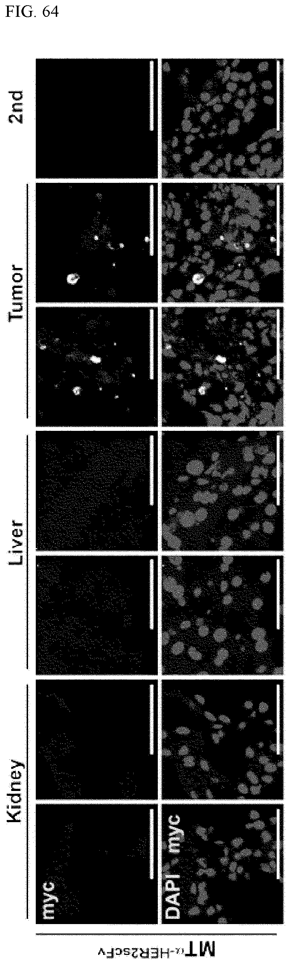

As a result, MT^{α-HER2scFv} was not observed in kidney and liver tissues, but MT^{α-HER2scFv} was observed in tumor tissues (FIG. 64). From the above results, it was confirmed that MT^{α-HER2scFv} injected into the mouse vein specifically targeted NCI-N87 (HER2⁺) tumor cells subcutaneously transplanted into the lateral abdomen of the mouse.

### Example 13. Evaluation of anticancer activity of TPP-DOX_MT^{α-HER2scFv} in xenograft tumor mouse model

5-week-old BALB/c male nude mice (initial body weight of 18 - 20 g) (Nara Biotech) were acclimatized for 1 week, and then HER2⁺ human gastric cancer cell line NCI-N87 was subcutaneously transplanted into the lateral abdomen at 1×10⁷ cells/mouse to construct a xenograft tumor model mouse. When the average volume of the tumor reached 50 - 100 mm³, the mice were divided into 3 groups of 7 mice per test group, and 4 µg of TPP-DOX or TPP-DOX MT^{α-HER2scFv} was intravenously administered once a week for 4 weeks for a total of 7 times. The tumor size was measured at an interval of 2 - 4 days, and after 25 days from the start of administration, the tumor tissues were extracted, and the size and weight were measured (FIG. 65).

### Example 13.1. Confirmation of tumor cell growth inhibitory ability of TPP-DOX_MT^{α-}^{HER2scFv} in xenograft tumor mouse model

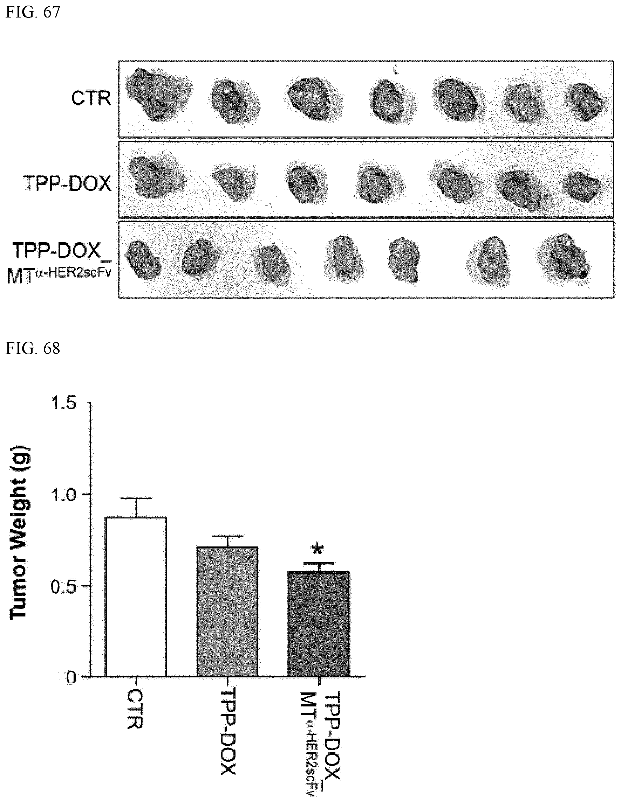

As a result of the experiment through the method of Example 13 above, in the case of TPP-DOX administration group, the tumor size was similar until the 30^{th} day compared to the control group (CTR). On the other hand, the tumor size in TPP-DOX_MT^{α-HER2scFv} administration group was significantly reduced compared to the control group (FIG. 66). In addition, the size (FIG. 67) and weight (FIG. 68) of the tumor extracted after the end of the experiment were significantly reduced in TPP-DOX_MT^{α-HER2scFv} treated group. Statistical processing was performed using Graphpad 5.0 software, and p values were measured with one-way ANOVA (Turkey's) test (*p<0.05).

### Example 14. Evaluation of anticancer activity of TPP-DOX_MT^{α-HER2scFv} in allograft tumor mouse model

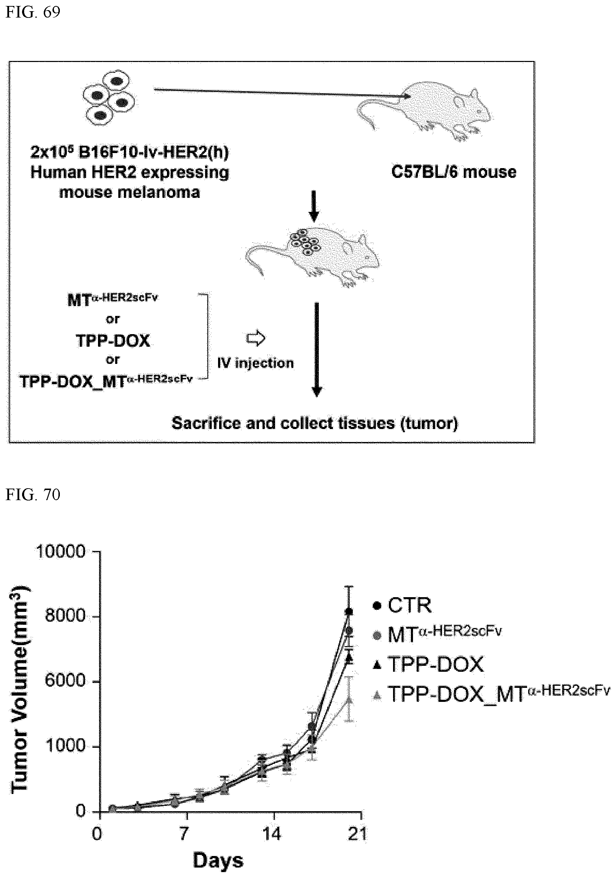

7-week-old C57BL/6 female mice (Samtako Bio Korea) were acclimatized for 1 week, and then the mouse melanoma cell line B16F10 was subcutaneously transplanted into the lateral abdomen of the mice at about 5×10⁵ cells/mouse to construct an allograft tumor mouse. At this time, B 16F 10 cells in which the human HER2 gene was overexpressed using lentivirus were used as B 16F 10 cells. When the average volume of the tumor reached 100 - 200 mm³, the mice were divided into 4 groups of 3 mice per test group, and MT^{α-HER2scFv} (20 µg), TPP-DOX (5 µg) or TPP-DOX_MT^{α-HER2scFv} (TPP-DOX/MT: 5 µg/20 µg) was intravenously administered 3 times a week for 3 weeks. The tumor size was measured 3 times a week, and after the end of the experiment (after 3 weeks from the start of administration), the tumor tissues of the mice were extracted, and the size and weight were measured (FIG. 69).

### Example 14.1. Confirmation of tumor cell growth inhibitory ability of TPP-DOX_MT^{α-HER2scFv} in allogenic cancer cell transplantation animal model

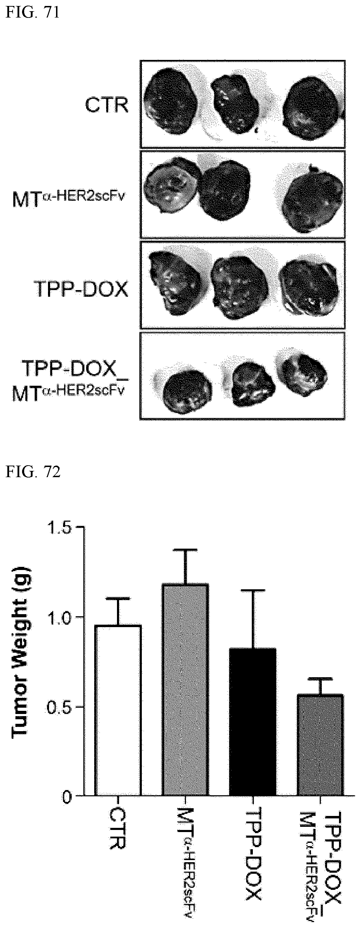

As a result of the experiment through the method of Example 14 above, in the case of MT^{α-HER2scFv} and TPP-DOX administration group, the tumor size was similar to that of the control group (CTR). On the other hand, the tumor size in TPP-DOX_MT^{α-HER2scFv} administration group was significantly reduced compared to the control group (FIG. 70). In addition, the size (FIG. 71) and weight (FIG. 72) of the tumor extracted after the end of the experiment were reduced in TPP-DOX_MT^{α-HER2scFv} treated group.

### Example 15. Evaluation of anticancer activity of DOX_MT^{α-HER2scFv} in allograft tumor animal model

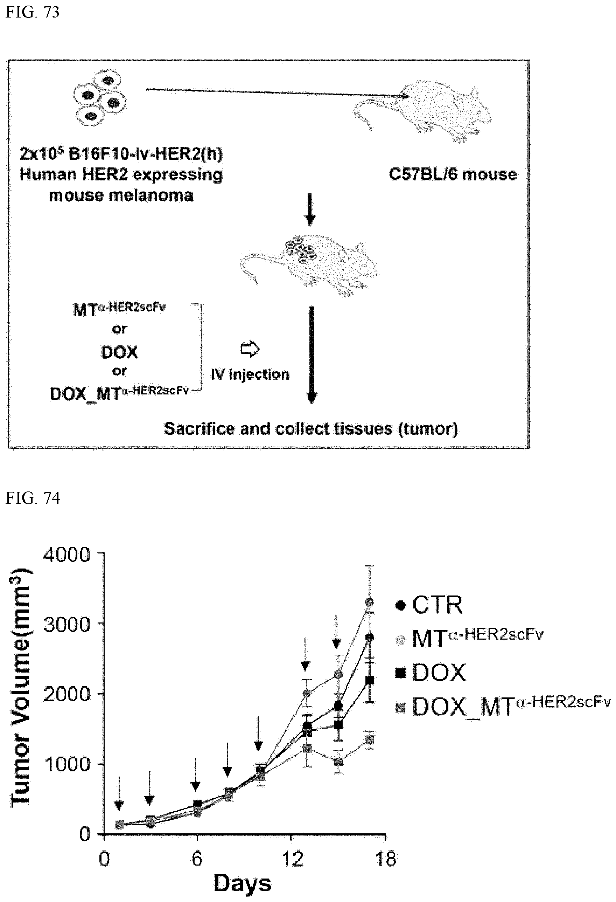

7-week-old C57BL/6 female mice (Samtako Bio Korea) were acclimatized for 1 week, and then the mouse melanoma cell line B 16F 10 was subcutaneously transplanted into the lateral abdomen of the mice at about 5×10⁵ cells/mouse to construct an allograft tumor mouse. At this time, B 16F 10 cells in which the human HER2 gene was overexpressed using lentivirus were used as B 16F 10 cells. When the average volume of the tumor reached 100 - 200 mm³, the mice were divided into 4 groups of 3 mice per test group, and MT^{α-HER2scFv} (20 µg), DOX (4 µg) or DOX_MT^{α-HER2scFv} (TPP-DOX/MT: 4 µg/20 µg) was intravenously administered 3 times a week for 3 weeks. The tumor size was measured 3 times a week, and after the end of the experiment (after 3 weeks from the start of administration), the tumor tissues of the mice were extracted, and the size and weight were measured (FIG. 73).

### Example 15.1. Confirmation of tumor cell growth inhibitory ability of DOX_MT^{α-HER2scFv} in allograft tumor animal model

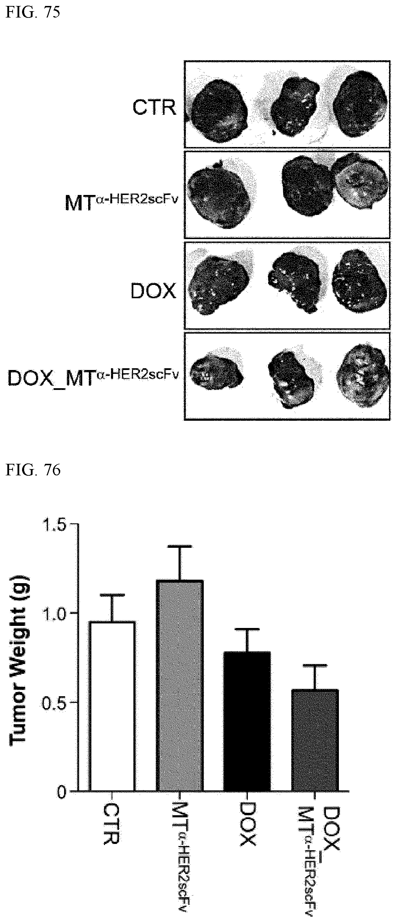

As a result of the experiment through the method of Example 14 above, in the case of MT^{α-HER2scFv} administration group, the tumor size was similar to that of the control group (CTR). On the other hand, the tumor size in DOX administration group and DOX_MT^{α-HER2scFv} administration group was significantly reduced compared to the control group. In addition, it was confirmed that the tumor size in DOX_MT^{α-HER2scFv} administration group was further reduced compared to DOX treated group (FIG. 74). In addition, the size (FIG. 75) and weight (FIG. 76) of the tumor extracted after the end of the experiment was reduced in DOX_MT^{α-HER2scFv} administration group compared to the control group.

### [Free Text of Sequence Listing]

SEQ ID NO: 1: T2p53 primer
SEQ ID NO: 2: Xp53 primer
SEQ ID NO: 3: nucleotides sequence coding p53
SEQ ID NO: 4: NdeUB primer
SEQ ID NO: 5: T2UB primer
SEQ ID NO: 6: nucleotides sequence coding UB-p53
SEQ ID NO: 7: NdeTOM70 primer
SEQ ID NO: 8: TOM70-AS primer
SEQ ID NO: 9: TOM70UB-S primer
SEQ ID NO: 10: T2UB-AS primer
SEQ ID NO: 11: nucleotides sequence coding TOM70-UB-p53
SEQ ID NO: 12: TOM70 (G)3-AS primer
SEQ ID NO: 13: (G)3UB-S primer
SEQ ID NO: 14: Xp53 (noT) primer
SEQ ID NO: 15: nucleotides sequence coding TOM70-(GGGGS)3-UB-p53
SEQ ID NO: 16: B (G)3p53
SEQ ID NO: 17: nucleotides sequence coding TOM70-(GGGGS)3-p53
SEQ ID NO: 18: Xp53 (noT) primer
SEQ ID NO: 19: XTOM7 primer
SEQ ID NO: 20: LTOM7 primer
SEQ ID NO: 21: nucleotides sequence coding UB-p53-TOM7
SEQ ID NO: 22: Rp53 primer
SEQ ID NO: 23: nucleotides sequence coding p53-myc/His
SEQ ID NO: 24: T2GZMB primer
SEQ ID NO: 25: XGZMB (noT) primer
SEQ ID NO: 26: nucleotides sequence coding GranzymeB
SEQ ID NO: 27: nucleotides sequence coding TOM70-(GGGGS)3-UB-Granzyme B
SEQ ID NO: 28: nucleotides sequence coding UB-Granzyme B-TOM7
SEQ ID NO: 29: T2RKIP primer
SEQ ID NO: 30: XRKIP (noT) primer
SEQ ID NO: 31: nucleotides sequence coding RKIP
SEQ ID NO: 32: nucleotides sequence coding TOM70-(GGGGS)3-UB-RKIP
SEQ ID NO: 33: T2PTEN primer
SEQ ID NO: 34: XPTEN (noT) primer
SEQ ID NO: 35: nucleotides sequence coding PTEN
SEQ ID NO: 36: nucleotides sequence coding TOM70-(GGGGS)3-UB-PTEN
SEQ ID NO: 37: nucleotides sequence coding scFvHER2
SEQ ID NO: 38: nucleotides sequence coding UB-scFvHER2-TOM7
SEQ ID NO: 39: RscFvHER2 primer
SEQ ID NO: 40: XTOM7 (noT) primer
SEQ ID NO: 41: nucleotides sequence coding scFvHER2-TOM7-myc/His
SEQ ID NO: 42: nucleotides sequence coding scFvMEL
SEQ ID NO: 43: nucleotides sequence coding UB-scFvMEL-TOM7
SEQ ID NO: 44: RscFvMEL primer
SEQ ID NO: 45: nucleotides sequence coding scFvMEL-TOM7-myc/His
SEQ ID NO: 46: nucleotides sequence coding scFvPD-L1
SEQ ID NO: 47: nucleotides sequence coding scFvPD-L1-TOM7-myc/His
SEQ ID NO: 48: amino acid sequence of p53
SEQ ID NO: 49: amino acid sequence of UB-p53
SEQ ID NO: 50: amino acid sequence of TOM70-UB-p53
SEQ ID NO: 51: amino acid sequence of TOM70-(GGGGS)3-UB-p53
SEQ ID NO: 52: amino acid sequence of TOM70-(GGGGS)3-p53
SEQ ID NO: 53: amino acid sequence of UB-p53-TOM7
SEQ ID NO: 54: amino acid sequence of p53-myc/His
SEQ ID NO: 55: amino acid sequence of GranzymeB
SEQ ID NO: 56: amino acid sequence of TOM70-(GGGGS)3-UB-Granzyme B
SEQ ID NO: 57: amino acid sequence of UB-Granzyme B-TOM7
SEQ ID NO: 58: amino acid sequence of RKIP
SEQ ID NO: 59: amino acid sequence of TOM70-(GGGGS)3-UB-RKIP
SEQ ID NO: 60: amino acid sequence of PTEN
SEQ ID NO: 61: amino acid sequence of TOM70-(GGGGS)3-UB-PTEN
SEQ ID NO: 62: amino acid sequence of scFvHER2
SEQ ID NO: 63: amino acid sequence of UB-scFvHER2-TOM7
SEQ ID NO: 64: amino acid sequence of scFvHER2-TOM7-myc/His
SEQ ID NO: 65: amino acid sequence of scFvMEL
SEQ ID NO: 66: amino acid sequence of UB-scFvMEL-TOM7
SEQ ID NO: 67: amino acid sequence of scFvMEL-TOM7-myc/His
SEQ ID NO: 68: amino acid sequence of scFvPD-L1
SEQ ID NO: 69: amino acid sequence of scFvPD-L1-TOM7-myc/His
SEQ ID NO: 70: linker
SEQ ID NO: 71: amino acid sequence of ubiquitin
SEQ ID NO: 72: nucleotides sequence coding ubiquitin
SEQ ID NO: 73: amino acid sequence of p53
SEQ ID NO: 74: nucleotides sequence coding p53
SEQ ID NO: 75: TOM70 of S. cerevisiae
SEQ ID NO: 76: TOM70 of Homo sapiens
SEQ ID NO: 77: TOM20 of S. cerevisiae
SEQ ID NO: 78: TOM20 of Homo sapiens
SEQ ID NO: 79: OM45 of S. cerevisiae
SEQ ID NO: 80: TOM5 of S. cerevisiae
SEQ ID NO: 81: TOM5 of Homo sapiens
SEQ ID NO: 82: TOM7 of S. cerevisiae
SEQ ID NO: 83: TOM7 of Homo sapiens
SEQ ID NO: 84: TOM22 of S. cerevisiae
SEQ ID NO: 85: TOM22 of Homo sapiens
SEQ ID NO: 86: Fis1 of S. cerevisiae
SEQ ID NO: 87: Fis1 of Homo sapiens
SEQ ID NO: 88: Bcl-2 alpha of Homo sapiens
SEQ ID NO: 89: VAMP1 of S. cerevisiae
SEQ ID NO: 90: VAMP1 of Homo sapiens
SEQ ID NO: 91: P53-promter-S
SEQ ID NO: 92: P53-promter-AS
SEQ ID NO: 93: nucleotides sequence coding scFvPDL1-myc-TOM7
SEQ ID NO: 94: amino acid sequence of scFvPDL1-myc-TOM7
SEQ ID NO: 95: nucleotides sequence coding scFvHER2
SEQ ID NO: 96: amino acid sequence of scFvHER2
SEQ ID NO: 97: nucleotides sequence coding scFvHER2-myc-TOM7
SEQ ID NO: 98: amino acid sequence of scFvHER2-myc-TOM7
SEQ ID NO: 99: nucleotides sequence coding scFvMSLN
SEQ ID NO: 100: amino acid sequence of scFvMSLN
SEQ ID NO: 101: nucleotides sequence coding scFvMSLN-myc-TOM7
SEQ ID NO: 102: amino acid sequence of scFvMSLN-myc-TOM7

## Claims

1. A modified mitochondrion comprising an anticancer agent.

2. The modified mitochondrion according to claim 1, wherein the anticancer agent is one to which triphenylphosphonium (TPP) or a derivative thereof is bound.

3. The modified mitochondrion according to claim 1, wherein the anticancer agent is selected from the group consisting of a chemical anticancer agent, a target anticancer agent and an anthelmintic agent.

4. The modified mitochondrion according to claim 3, wherein the chemical anticancer agent is any one selected from the group consisting of an alkylating agent, a microtuble inhibitor, an antimetabolite and a topoisomerase inhibitor.

5. The modified mitochondrion according to claim 3, wherein the target anticancer agent is any one selected from the group consisting of compounds targeting BTK, Bcr-abl, EGFR, PDGFR/VEGFR/FGFR family, MEK/RAF, HER2/Neu, CDK, ubiquitin, JAK, MAP2K, ALK, PARP, TGFβRI, Proteasome, Bcl-2 Braf, C-Met, VR1, VR2, VR3, c-kit, AXL and RET.

6. The modified mitochondrion according to claim 3, wherein the anthelmintic agent is any one selected from the group consisting of an OXPHOS (oxidative phosphorylation) inhibitor, a glycolysis inhibitor, a glycolysis related indirect inhibitor, a PPP (pentose phosphate pathway) inhibitor and an autophagy inhibitor.

7. The modified mitochondrion according to claim 1, wherein the mitochondrion is the modified mitochondrion in which an antibody specifically binding to a tumor-associated antigen is present in a cell.

8. The modified mitochondrion according to claim 1, wherein the tumor-associated antigen is any one selected from the group consisting of CD19, CD20, melanoma antigen E (MAGE), NY-ESO-1, carcinoembryonic antigen (CEA), mucin 1 cell surface associated (MUC-1), prostatic acid phosphatase (PAP), prostate specific antigen (PSA), survivin, tyrosine related protein 1 (tyrp1), tyrosine related protein 2 (tyrp2), Brachyury, Mesothelin, Epidermal growth factor receptor (EGFR), human epidermal growth factor receptor 2 (HER-2), ERBB2, Wilms tumor protein (WT1), FAP, EpCAM, PD-L1, ACPP, CPT1A, IFNG, CD274, FOLR1, EPCAM, ICAM2, NCAM1, LRRC4, UNCSH2 LILRB2, CEACAM, Nectin-3 and a combination thereof.

9. The modified mitochondrion according to claim 1, wherein the cancer is any one selected from the group consisting of breast cancer, lung cancer, pancreatic cancer, glioblastoma, gastric cancer, liver cancer, colorectal cancer, prostate cancer, ovarian cancer, cervical cancer, thyroid cancer, laryngeal cancer, acute myeloid leukemia, brain tumor, neuroblastoma, retinoblastoma, head and neck cancer, salivary gland cancer and lymphoma.

10. A pharmaceutical composition for the prevention or treatment of cancer, comprising the modified mitochondrion according to claim 1 as an active ingredient.

11. Use of the modified mitochondrion according to claim 1 for the manufacture of a pharmaceutical composition for the treatment of cancer.

12. A method for treating cancer, comprising administering the modified mitochondrion according to claim 1 to a subject.

13. A method for preparing mitochondrion comprising an anticancer agent, comprising:
mixing the anticancer agent and the isolated mitochondrion.

14. A modified anticancer agent in which TPP or a derivative thereof is bound to an anticancer agent.
